# EUROPEAN PATENT SPECIFICATION

(11) **EP 3 013 271 B1**
(45) Date of publication and mention of the grant of the patent: **02.10.2019**
(21) Application number: 14816575.6
(22) Date of filing: 20.06.2014
(51) Int. Cl.: A61B 18/12, A61B 17/3209, A61B 18/14

(54) **APPARATUS FOR DISSECTION AND MODIFICATION OF TISSUES**
VORRICHTUNG ZUM SEZIEREN UND MODIFIZIEREN VON GEWEBE
APPAREIL DE DISSECTION ET DE MODIFICATION DE TISSUS

(30) Priority: 27.06.2013 US 201361840406 P
(43) Date of publication of application: 04.05.2016
(73) Proprietor: Weber, Paul, Palm Harbor, Florida 34683 (US)
(72) Inventor: Weber, Paul, Palm Harbor, Florida 34683 (US)
(74) Representative: Lock, Howard John
(86) International application number: PCT/IB2014/062495
(87) International publication number: WO 2014/207640

(56) References cited:
- WO-A1-99/16371
- US-A- 5 766 153
- US-A- 5 776 092
- US-A- 5 776 092
- US-A- 5 993 445
- US-A1- 2002 072 741
- US-A1- 2003 040 744
- US-A1- 2005 113 827
- US-A1- 2007 049 922
- US-A1- 2011 144 729
- US-A1- 2011 144 729
- US-B1- 6 203 540
- US-B2- 7 223 267
- US-B2- 7 717 913

## Description

### DISCUSSION OF THE PRIOR ART

WO 99/16371 A1 discloses apparatus for electrosurgical tissue removal. US 2002/072741 A1 discloses devices for epicardial mapping and ablation for the treatment of atrial fibrillation.

US 2011/144729 A1 discloses a face lifting device, wherein forward motion of the device divides and energizes various tissue planes. US 6203540 B1 discloses a device for separating subcutaneous tissue and for tightening and contracting internal dermal tissue.

### GENERAL DESCRIPTION OF THE INVENTION

The invention is as defined in independent claim 1. Dependent claims 2-14 disclose exemplary embodiments.

According to one aspect of this invention, there is provided an apparatus for tissue separation and modification, comprising:
a surgical device comprising:
a main tip comprising a plurality of protrusions;
at least one lysing element positioned between at least two adjacent protrusions in the plurality of protrusions;
an elongate main shaft coupled to the main tip, the main shaft having a longitudinally extending axis;
wherein the at least two adjacent protrusions are oriented in a non-axial direction relative to the aforesaid axis of the main shaft, characterised in that the at least two adjacent protrusions are spaced from each other along the aforesaid axis of the main shaft;
and further characterised in that the plurality of protrusions (151b) comprise two sets of non-axial protrusions (151b) extending in directions that are substantially opposite from one another, and wherein the apparatus includes a respective lysing element (153b), between adjacent protrusions (151b) of each set.

### BRIEF DESCRIPTION OF THE DRAWINGS

The written disclosure herein describes illustrative embodiments that are non-limiting and non-exhaustive. Reference is made to certain of such illustrative embodiments that are depicted in the figures, in which:
FIG. 1a is a perspective view of an embodiment of a modular tissue dissector illustrating the protrusions and lysing elements, wherein some of the protrusions and lysing elements are oriented in a non-axial direction.
FIG. 1b is a side view of the embodiment previously depicted in FIG. 1a.
FIG. 1c is an upper plan view of the embodiment previously depicted in FIG. 1a also depicting a spot coagulating lysing element.
FIG. 1d is an upper plan view of the embodiment previously depicted in FIG. 1a reversibly attached to a rigid shaft of an electrosurgical 'pencil'.
FIG. 1e is a cross sectional view of the rigid shaft portion of the embodiment depicted in FIG. 1d.
FIG. 2a is an upper plan view of an embodiment of a modular tissue dissector and modifier with an electrosurgical energy window on the upper side of the device.
FIG. 2b is a perspective view of the embodiment previously depicted in FIG. 2a reversibly attached to a rigid electrosurgical 'pencil' shaft.
FIG 3 is an upper plan view of an embodiment of a modular tissue dissector illustrating 30 the protrusions and lysing elements, wherein the protrusions and lysing elements are oriented only in non-axial directions.
FIG 4 is an upper plan view of an embodiment of a modular tissue dissector illustrating the protrusions and lysing elements, wherein the protrusions and lysing elements are oriented in axial and non-axial directions.
FIG. 5a is an upper plan view illustrating the protrusions and lysing elements of an embodiment of a modular tissue dissector lacking a spot coagulating lysing element, wherein the protrusions and lysing elements are oriented in axial and non-axial directions.
FIG. 5b is an upper plan view of the embodiment previously depicted in FIG. 5a reversibly attached to a rigid electrosurgical shaft.
FIG. 6a is an upper plan view illustrating an embodiment of a modular tissue dissector, wherein some of the protrusions and lysing elements are oriented in a non-axial direction with an attached axially-slidable spot coagulator.
FIG. 6b is a side view of the embodiment previously depicted in FIG. 6a.
FIG. 7a is an upper plan view illustrating an embodiment of a tissue dissector, wherein some of the protrusions and lysing elements are oriented in a non-axial direction with a rotationally deployable spot coagulation boom.
FIG. 7b is a side view of the embodiment previously depicted in FIG. 7a.
FIG. 8a is an upper plan view illustrating an example not according to the invention of a tissue dissector wherein the protrusions and lysing elements are oriented in an axial direction with an attached axially-slidable spot coagulator.
FIG. 8b is a side view of the example previously depicted in FIG. 8a.
FIG. 9a is an upper plan view illustrating an example not according to the invention of a tissue dissector, wherein the protrusions and lysing elements are oriented in an axial direction with an attached rotationally deployable spot coagulation boom.
FIG. 9b is a side view of the example previously depicted in FIG. 9a.
FIG. 10a is an upper plan view illustrating an embodiment of a tissue dissector wherein some of the protrusions and lysing elements are oriented in a non-axial direction with an attached axially-slidable spot coagulator, spot coagulator sheath, and spot coagulator connecting conductor cable.
FIG. 10b is a side view of the embodiment previously depicted in FIG. 10a
FIG. 11a is an upper plan view illustrating an example not according to the invention of a tissue dissector wherein the protrusions and lysing elements are oriented in an axial direction with an attached axially-slidable spot coagulator, spot coagulator shield, and spot coagulator connecting conductor cable.
FIG. 11b is a side view of the example previously depicted in FIG. 11a
FIG. 12a is an upper plan view illustrating an embodiment of a tissue dissector wherein some of the protrusions and lysing elements are oriented in a non-axial direction with an attached axially-slidable spot coagulator and spot coagulator toggle.
FIG. 12b is a side view of the embodiment previously depicted in FIG. 12a
FIG. 13a is an upper plan view illustrating an example not according to the invention of a tissue dissector wherein the protrusions and lysing elements are oriented in an axial direction with an attached axially-slidable spot coagulator and spot coagulator toggle.
FIG. 13b is a side view of the example previously depicted in FIG. 13a
FIG. 14a is a perspective view of an example not according to the invention of a tissue dissector and modifier with an energy window on the upper side of the device and a handle.
FIG. 14b is a side elevation view of the example previously depicted in FIG. 14a.
FIG 15a is a wiring diagram of one example of the TDM employing two switches.
FIG 15b is a wiring diagram of another example of the TDM employing one switch.
FIG 15c is a wiring diagram of another example of the TDM employing one switch.
FIG 15d is a wiring diagram of a circuit in another example of the TDM designed to modulate the energy delivered.
FIG 16 is a wiring diagram of a bipolar example of the TDM
FIG. 17a is a side view of a robotic surgery system comprising a TD.
FIG. 17b depicts an alternative robotic arm that may be used with the system of FIG. 17a.
FIG. 18 is a flow chart illustrating one implementation of a method not according to the invention of an energy emission - sensor feedback loop.
FIG. 19 is a flow chart illustrating one implementation of a method not according to the invention for accessing an organ using the TDM.
FIG. 20 depicts an embodiment comprising a shaft having a flexible segment.
FIG. 21 is a flow chart illustrating one implementation of a method not according to the invention for separating and/or modifying tissue using a TD.
FIG. 22a is an upper plan view illustrating an example not according to the invention of a bipolar tissue dissector wherein the protrusions and lysing elements are oriented an axial direction and return electrode(s) comprise lysing elements.
FIG. 22b is an upper plan view that depicts an example of wiring to the lysing elements of the example previously depicted in FIG. 23a.
FIG. 22c is a side view of a bipolar circuit activating foot-pedal bypass comprising a sticker.
FIG. 22d is a side view of the example previously depicted in FIG. 22a with the bipolar circuit activating foot-pedal bypass sticker applied.
FIG. 23 is a perspective view of a bipolar circuit activating foot-pedal bypass comprising an adhesive backed pad.
FIG. 24a is a side view of a bipolar circuit activating foot-pedal bypass comprising a sheath.
FIG. 24b is a side view of a bipolar tissue dissector ensheathed in bipolar circuit activating foot-pedal bypass comprising a sheath.
FIG. 25a is an upper plan view illustrating an example not according to the invention of a modular common intermediate configuration tissue dissector wherein the protrusions and lysing elements are oriented an axial direction and return electrode is not located on a lysing segment.
FIG. 25b is a side view of the example previously depicted in FIG. 25a.
FIG. 26a is an upper plan view illustrating an embodiment of a tissue dissector wherein some of the protrusions and lysing elements are oriented in a non-axial direction with an attached axially-slidable spot coagulator, spot coagulator toggle and spot coagulator moving means which is rigidly affixed to an electrosurgical 'pencil' shaft.
FIG. 26b is a side view of the embodiment previously depicted in FIG. 26a.
FIG. 27 is an upper plan view illustrating an embodiment of a tissue dissector wherein some of the protrusions and lysing elements are oriented in a non-axial direction with an attached axially-slidable spot coagulator, spot coagulator toggle and spot coagulator moving means which is rigidly affixed to a modular tissue dissector shaft.
FIG. 28a is an upper plan view illustrating an example not according to the invention of a tissue dissector tip wherein the protrusions and lysing elements are oriented in a axial direction with an attached slidable spot coagulator passing through a TD tip passageway.
FIG. 28b is a side view of the example previously depicted in FIG. 28a.
FIG. 28c is an upper plan view illustrating an example not according to the invention of a tissue dissector wherein some of the protrusions are oriented in an axial direction with a TD tip passageway (containing an axially-slidable spot coagulator in a storage position within the TD tip), spot coagulator toggle and spot coagulator moving means which is rigidly affixed to an electrosurgical 'pencil' shaft.
FIG. 28d is a side view of the example previously depicted in FIG. 28c with the spot coagulator deployed in the operational position.
FIG. 28e is an upper plan view illustrating an example not according to the invention of a spot coagulator shaft & SC tip wherein the SC tip may resemble a lysing element.
FIG. 29 is a flow chart of an implementation of a method not according to the invention for making an electrosurgical incision using the lysing elements of a tissue dissector.
FIG. 30a is a perspective view of another example not according to the invention of a modular TDM comprising a spot coagulator coupled with a shaft of an electrosurgical instrument.
FIG. 30b is a perspective view of a conductive shaft of the modular TDM of FIG. 30a.
FIG. 30c is a close-up perspective view of the tip of the modular TDM of FIG. 30a.

### DETAILED DESCRIPTION

The term dissection may indicate the separation of tissues or of one tissue plane from another (ref: Free Online Medical Dictionary). Some also consider dissection to comprise separation of a single tissue into portions. Much of the bodies of animals and humans are formed from embryonic fusion planes. Many of the organs of the human body are categorized from the embryonic fusion planes from whence they came. The interfaces between organs may often be referred to as 'tissue planes.' Such planes may be considered substantially planar depending upon the size of a comparative planar living or inanimate object (such as a surgical instrument). The TD and/or TDM may perform the functions of sharp dissection, blunt dissection and electrosurgical cutting and/or coagulation simultaneously without a surgeon having to switch instruments. Sharp dissection has been referred to by some as separation of tissues by means of the sharp edge of a knife or scalpel or with the inner sharp edge of scissors. Blunt dissection has been defined by Webster as surgical separation of tissue layers by means of an instrument without a cutting edge or by the fingers.

The term 'minimally invasive surgery' has been used to describe a procedure (surgical or otherwise) that is less invasive than open surgery used for the same purpose. Some minimally invasive procedures typically involve use of laparoscopic devices and remote-control manipulation of instruments with indirect observation of the surgical field through an endoscope or similar device, and are carried out through the skin or through a body cavity or anatomical opening. This may result in shorter hospital stays, or allow outpatient treatment (reference: Wikipedia).

The term 'open surgery' is used to indicate cutting skin and tissues to 'open the body' so that the surgeon has direct access to the structures or organs involved. Often an incision is made of a size that permits the surgeon's hands entry into a patient's body. The structures and tissues involved can be seen and touched, and they are directly exposed to the air of the operating room.

The term Tissue Dissector (TD) is intended to encompass any of the devices for dissecting tissue disclosed herein including but not limited to Tissue Dissecting and Modifying Wands (TDM) comprising lysing elements, tissue dissecting and modifying wands lacking lysing elements, and tissue dissecting wands either comprising or lacking energy windows. In some embodiments, the lysing elements may comprise lysing segments.

The term 'modifying' in this context may refer to or may encompass application of energy to living tissue using one or more lysing elements as discussed herein. The term 'modifying' in this context may also refer to application of energy to tissue by way of an energy window as also described herein. Such methods may be performed using a Tissue Dissecting and Modifying Wand ("TDM"). Examples of various embodiments of such wands may be found in U.S. Patent No. 6,203,540 titled "Ultrasound and Laser Face-Lift and Bulbous Lysing Device," U.S. Patent No. 6,391,023 titled "Thermal Radiation Facelift Device," U.S. Patent No. 6,432,101 titled "Surgical Device for Performing Face-Lifting Using Electromagnetic Radiation," U.S. Patent No. 6,440,121 titled "Surgical Device For Performing Face-Lifting Surgery Using Radiofrequency Energy," U.S. Patent No. 6,974,450 titled "Face-Lifting Device," and U.S. Patent No. 7,494,488 titled "Facial Tissue Strengthening and Tightening Device and Methods." Example of a lysing device is found in U.S. Patent No. 6,203,540 titled "Ultrasound and Laser Face-Lift and Bulbous Lysing Device," the section titled "Description of the Preferred Embodiments".

Some tissues and/or organs and/or tumors treated with the TDM may be of varying sensitivity to electrosurgical and/or other forms of energy. Modulation and feedback may be helpful for such tissues. Controlling the amount of energy delivered by a moving device within the body that is continuously delivering energy and/or delivering energy in a pulsed format may rely upon methods to locate the device in real time, and/or the change in position of the device relative to a tissue or portion of the tissue over time. Real time calculations may be made by hardware and/or software to record and/or control energy delivery. For example, an antenna system in relation to Tissue Dissecting Wands and/or Tissue Dissection and Modifying Wands has been previously described by Weber in US Patent application number 13/802,731, published as US 2014/0188095 A1, titled: Apparatus and Systems for Tissue Dissection and Modification & US Patent application number 13/767,876, published as US 2014/0187870 A1, titled: System, Apparatus and Methods for Tissue Dissection. Although a common laser operated computer mouse may be used to track distances moved over time on surface skin with limited degree of accuracy, such a system may be unsuitable to track distanced moved when placed on a primarily fatty undersurface of the skin (i.e. applied from within the body). This may be related to the inability of such a device to detect a speckle pattern in tissue that may share similarities to butter such as reflectance and homogeneity. Herein to be discussed will be solutions to 'tracking' movement against internal organs and/or tissues and/or the undersurface of the body skin.

The TD and/or TDM may deposit energy in tissues via for example energized lysing elements, one or more energy windows or other such components on the TD or TDM and thus heat tissue to a measurable level. The speed of passage of the TD and/or TDM may influence the amount of energy deposited by said lysing elements, energy window or other such energy delivering element. Thus, methods of reasonable accuracy to determine the speed of motion of the area(s) emitting the energy may with other determined parameters (such as power output per unit area) permit real time or delayed calculation of the power delivered per unit area for a given exposure time. For real time calculations, a feedback loop may be created to control energy emissions (amount, frequency, intensity, etc).

The TDM may be "energized" by various forms of energy in its top side energy window, as described in greater detail below. Such energy absorptions may result in the formation of heat which may, in turn, denature tumor and/or other tissue cells themselves, and/or their surrounding environment in order to achieve a desired effect of a surgical method or procedure.

In some embodiments, energy may be delivered from one or more energy windows so as to heat tissue to a given temperature range. Various methods may therefore be implemented in which the amount of energy and/or the delivery time may be adjusted so as to heat the tissue to within a desired temperature range. Temperature sensors may therefore be incorporated on or near the energy windows to allow a surgeon to heat the tissue to a desired temperature or within a desired temperature range. In some embodiments, the sensor may be configured to provide an average temperature over a particular period of time and or over a particular range of distances within the tissue. Systems consistent with the disclosure provided herein may be configured to prevent or to shut down or otherwise limit energy transfer if a particular tissue temperature were beyond a threshold or alternatively if an average temperature threshold is reached.

Temperature sensors that may be useful in connection with embodiments disclosed herein include, but are not limited to, resistance temperature sensors, such as carbon resistors, film thermometers, wire-wound thermometers, or coil elements. Some embodiments may comprise thermocouples, pyrometers, or non-contact temperature sensors, such as total radiation or photoelectric sensors. In some embodiments, one or more temperature sensors may be coupled with a processor and/or a monitor to allow a surgeon to better visualize or otherwise control the delivery of energy to selected areas of target tissue. For example, some embodiments may be configured such that a surgeon can visualize the temperature of tissue positioned adjacent to one or more locations along the TD and/or TDM to ensure that such temperatures are within a desired temperature range. Some embodiments may alternatively, or additionally, be configured such that one or more temperature sensors are coupled with a processor in a feedback loop such that energy delivery may be automatically adjusted by the system in response to temperature data. For example, when temperatures exceed a particular threshold, such as somewhere between about 65° C and about 90° C, the system may be configured to shut down or otherwise limit further energy delivery. In some such embodiments, the threshold may be between about 68° C and about 75° C. Because the TDM is capable of delivering energy in a fractionated pattern, temperature thresholds and or ranges may vary depending upon the size of the spot being measured and/or the proximity and/or recency (time) of the spot being measured to the treatment area. Thus, some embodiments and implementations may comprise a threshold lesser or greater than those referenced above.

Some embodiments may comprise a feedback means, such as a visual, audible, or tactile feedback means, to provide information to a user to avoid excess energy delivery to tissues. In some embodiments, the feedback means may be configured to notify the surgeon when the temperature has reached a particular threshold. In some embodiments, the feedback means may be configured to notify the surgeon when the TD and/or TDM have been positioned in a particular location within the target region for a particular time period. Examples of visual feedback means include LED lights, LASERS, visual light source, display screen, etc. Examples of audible feedback means include speakers, alarms, audible vibration, etc. Examples of tactile feedback means include vibration, minimal electrical shock, heat, etc. The feedback means may be configured with multiple thresholds with different feedback at each threshold. For example, at a first threshold, the TD and/or TDM may be configured to deliver a first noise and at a second threshold the TD and/or TDM may be configured to deliver a second noise. The second noise may be louder than the first noise to indicate a greater urgency for changing the energy delivery and/or moving the TD and/or TDM from its current location within a patient's body. In some embodiments, an antenna(s) may be present on the shaft or tip of the TD and/or TDM. In some embodiments, a camera or fiberoptic may gather optical data to allow the surgeon knowledge of the placement of the TD and/or TDM. During some open surgeries, surgeons using the TD and/or TDM may lyse a vessel that exceeds the hemostatic capabilities of a given TD and/or TDM. Even though an isolated electrode from the 'upper' plane energy window may be inverted to coagulate said vessel, it may be more advantageous to have a spot coagulator extend from an embodiment of the TD and/or TDM at such a distance and/or location that allows complete viewing and/or contact of the bleeding area with a portion of the spot coagulator (for example the distal end point of a tip of the coagulator). Such a probe may be deployable and obtain electrical energy off of a conductive element located between the lysing elements of the tip and the plug.

Further details regarding various embodiments will now be provided with reference to the drawings.

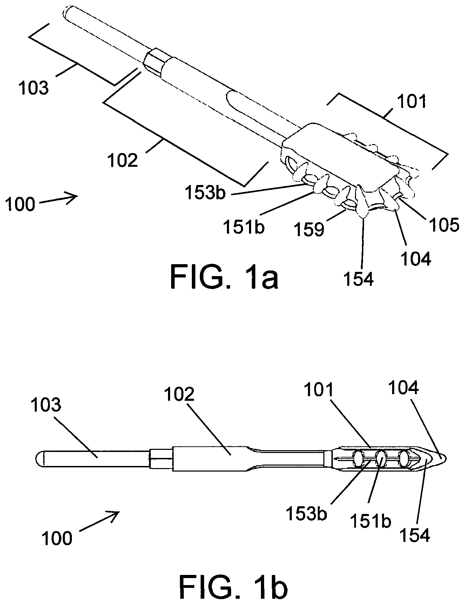

FIGs. 1a,b depict an embodiment of a modular Tissue Dissector (modular TD) **100** comprising a modular TD tip **101**, a modular TD shaft **102** and a modular TD plug **103**, wherein some of the protrusions and lysing elements are oriented in non-axial directions. In other words, protrusions **151b** are not oriented in a direction of the axis of modular TD shaft **102.** In the depicted embodiment, modular TD shaft **102** may comprise an insulating and/or supportive coating and/or cover overlying a conductive material and/or metal axial core. The embodiment depicted in FIGs. 1a&b also comprises transitional protrusions **154**. Transitional protrusions **154** extend from opposing corners (apices) of modular TD tip **101** and extend in directions at least approximately at midpoint between an axial direction and a direction perpendicular to the axial direction as indicated by protrusion **151b**. Transitional protrusions may be useful in preventing the device from becoming entangled in dense tissue. This embodiment also comprises a corner (or transitional) protrusion **154** that extends at a transitional angle relative to axial protrusions **104** and non-axial side protrusion **151b.** In some embodiments, one or more transitional angles may be acute. In some embodiments, one or more transitional angles may be obtuse.

In this embodiment, non-axial protrusion **151b** extends in a direction that is at least substantially perpendicular to the direction in which axial protrusions **104** extend. More particularly, there are two sets of non-axial protrusions (one set is depicted on the right side and one set is depicted on the left side of the embodiment of FIG. 1a). Both sets of non-axial protrusions extend in directions that are at least substantially perpendicular to the direction in which axial protrusions **104** extend (namely, along a longitudinal axis of the TDM shaft). In addition, it can be seen in FIG. 1a that the two sets of non-axial protrusions extend in directions that are at least substantially opposite from one another.

In some embodiments, axial protrusions **104** may extend at least substantially along a longitudinal axis of the shaft, as described above. In some embodiments non-axial protrusion such as **151b** may extend at an angle of between zero degrees and 30 degrees of a normal to the direction in which the axial protrusions **104** extend. It is contemplated that it may be desirable for some implementations and embodiments to provide non-axial tips extending in a direction or directions falling within this range in order to, for example, allow a surgeon to effectively perform both a to and fro, and a side-to-side ("windshield wiper") motion using the TDM. As described above transitional protrusions may be useful to enable a surgeon to avoid entangling the dissector in tissue during one or both such motions.

In some embodiments, the modular TD tip can be a separate piece that is secured to the modular TD shaft by a variety of methods such as a snap mechanism, mating grooves, plastic sonic welding, etc. Alternatively, in some other embodiments, the modular TD tip can be integral or a continuation of a modular TD shaft made of similar metal or materials. In some embodiments, the modular TD tip may also be constructed of materials that are both electrically non-conductive and of low thermal conductivity; such materials might comprise, for example, porcelain, ceramics, glass-ceramics, plastics, varieties of polytetrafluoroethylene, carbon, graphite, and/or graphite-fiberglass composites, etc. In some embodiments, the modular TD tip may be constructed of a support matrix of an insulating material (e.g., ceramic or glass material such as alumina, zirconia). In some embodiments, the modular TD tip may comprise cermets.

In other contemplated embodiments, the modular TD tip may comprise polyether etherketone &/or polytetrafluoroethylene with hollow glass microspheres (3M, St. Paul, MN).

External power control bundles as previously described in other embodiments may connect to electrically conductive elements to bring RF electrosurgical energy from an electrosurgical generator via, for example a handle and/or electrosurgical 'pencil,' down to the modular TD plug **103** (which is electrically connected) to modular TD shaft **102** to electrically conductive lysing elements, such as lysing elements **153b,** mounted in the recessions in between the protrusions, such as protrusions **151b**. In some embodiments, the protrusions may comprise bulbous protrusions. The tip shown in this embodiment has two relative protrusions **104** and one relative recession **105** pointing along the main axis of the TDM and provides for a monopolar tip conductive element; the tip shown also has eight protrusions pointing in non-axial directions as well as relative recessions pointing in non-axial directions. In other embodiments the modular TD tip may have one or more non-axial protrusions and one or more non-axial relative recessions. In some embodiments the tip may have between 3 and 100 non-axial protrusions and relative recessions. It should be understood that the number of protrusions need not match the number of lysing elements or recessions. In the depicted embodiment, the modular TD tip **101** may alternatively be made partially or completely of concentrically laminated or annealed-in wafer layers of materials that may include plastics, silicon, glass, glass/ceramics, cermets or ceramics. Lysing elements may also be made partially or completely of a cermet material. Alternatively, in a further embodiment the tip may be constructed of insulation covered metals or electroconductive materials. The lysing elements may be located at the termini of conductive elements. In some embodiments, lysing elements may be continuous and continue to extend all the way around a tip. In some embodiments lysing elements may be discrete and do not extend all the way around a tip. For example, in some embodiments each of the lysing elements may comprise a separate piece of material. In other embodiments, each of the lysing elements may comprise a portion of a single plate, wire or other piece of material.

In the depicted embodiment, modular TD tip **101** which terminates in protrusions, such as **104** and **151b**, may comprise materials that are both electrically non-conductive and of low thermal conductivity such as porcelain, epoxies, ceramics, glass-ceramics, plastics, or varieties of polytetrafluoroethylene. Alternatively, the tip may be made from metals or electroconductive materials that are completely or partially insulated. In some embodiments, the electrically conductive tissue lysing elements(s), such as **153b,** may have any geometric shape including a thin cylindrical wire, and may be positioned within the relative recessions of the tip. The electrically conductive lysing elements can be in the shape of a plate or plane or wire and made of any metal or alloy that does not melt under operating conditions or give off toxic residua. The electrically conductive lysing elements may comprise steel, nickel, alloys, palladium, gold, tungsten, silver, copper, and platinum.

In the depicted embodiment, modular TD tip **101,** which terminates in protrusions such as **154,** may have one or more convex lysing elements (some embodiments of which may be of a greater size than any or most other contained (contained between 2 protrusions) lysing elements) such as spot coagulation lysing elements **159.** In some embodiments, the electrically conductive tissue lysing elements (s) **159** may have any geometric shape including a thin cylindrical wire, and may be positioned within any of the relative recessions of the tip. Again, during some open surgeries, surgeons using the TD and/or TDM may lyse a vessel that exceeds the hemostatic capabilities of a given TD and/or TDM. Even though an isolated electrode from the 'upper' plane energy window may be inverted to coagulate said vessel, it may be more advantageous to have spot coagulator coagulation capabilities within the same instrument. So as not to have the surgeon set down the TD or TDM, to pick up other instrumentation from the surgical tray to stem the bleeding blood vessel, it may be beneficial for the surgeon to have the option of 'spot' coagulation using the same dissecting TD or TDM. Thus, with a protruding lysing element, the surgeon may simply request operating personnel to increase the electrosurgical generator coagulation power (often in 'coagulation' current mode or 'blend' mode) while the surgeon angles the TD or TDM tip toward the bleeding vessel with the spot coagulation lysing element leading the way. The surgeon may then activate the electrosurgical generator while touching the prominent lysing element to the bleeding blood vessel. Carbonized debris remaining on the spot coagulation lysing element **159** may be removed using a nylon brush and/or surgical 'scratch' pad. Once the bleeding vessel has been stopped, the surgeon can request power to the electrosurgical generator be modified appropriately and may continue any remaining dissection with the TD or TDM. The spot coagulation lysing element may be 50% larger in surface area than similarly curved lysing elements and may vary from 20% larger to 70% larger.

In some embodiments, the shaft may be flat, rectangular or geometric in cross-section and/or substantially flattened. In some embodiments, smoothing of the edges of the shaft may reduce friction on the tissues surrounding the entrance wound. In some further embodiments, the shaft may be made of metal or plastic or other material with a completely occupied or hollow interior that can contain insulated wires, electrical conductors, fluid/gas pumping or suctioning conduits, fiber-optics, or insulation.

In some embodiments, shaft plastics, such as polytetrafluoroethylene, may act as insulation about wire or electrically conductive elements. In some embodiments, the shaft may alternatively be made partially or completely of concentrically laminated or annealed-in wafer layers of materials that may include plastics, silicon, glass, glass/ceramics, ceramics carbon, graphite, and/or graphite-fiberglass composites.

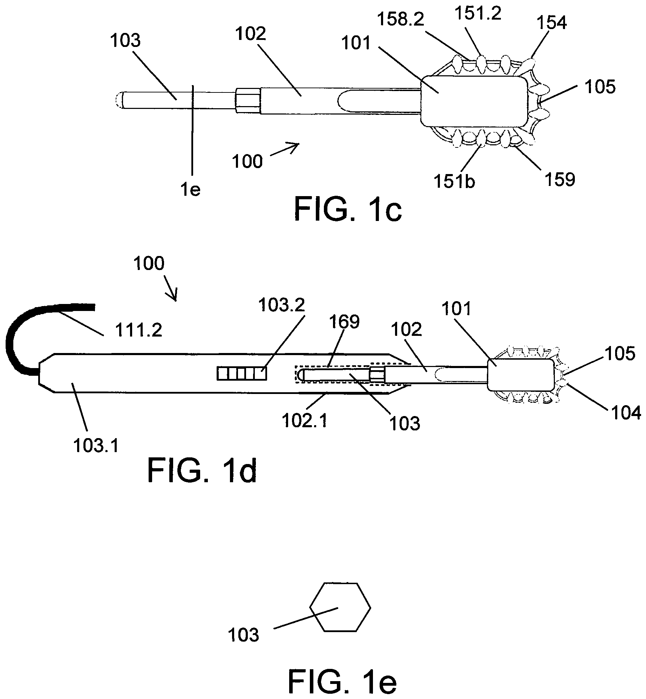

FIG. 1c is an upper plan view of the embodiment depicted in FIG. 1a of a modular TD **100** comprising a modular TD tip **101** with 3 different shaped lysing elements. Lysing element **105** is concave, lysing element **158.2** is straight as are the other lysing elements on the same side, lysing element **159** is convex, as are the other lysing elements on its same side. Non-axial protrusions **151b** and **151.2** point in substantially opposite directions. In the depicted embodiment, the double lines on the lysing elements indicate a bevel that is sharpened. In other embodiments, the bevel is not required to be sharp. In other embodiments the lysing element may be a wire. In some embodiments, all the lysing elements are of the same shape. In some contemplated embodiments, one or more of the lysing elements may differ from the rest in shape and/or other characteristics. Lysing elements shapes may be selected depending upon the operating surgeon's concern of the for a variety of factors, including, but not limited to: level of cutting and/or aggressiveness of dissection and/or tissue type dissected and/or proximity to vital structures and/or intended speed of dissection and/or viability/condition of tissue being dissected (semi-dead burn tissue or living interface) and/or density and/or vascularity. For example, a convex lysing element may protrude beyond what a concave lysing element might protrude and may cut into or dissect tissue, such as fat, faster but may higher chance of cutting blood vessels (fortunately, nerves may be fewer in fat so the surgeon may be able to dissect faster). Another example may be that a straight blade may pass more readily though devitalized tissue, such as burn victim skin, as the tissue may become gelatinous and/or homogenous in consistency. Concave lysing elements may be beneficial for forward motion as the forces applied to bulbs on forward/axial tip motion may exceed those usually encountered on side/perpendicular-to-axial/'normal' tip motion; thus, compaction of tissues at the relative recessions and/or lysing elements may beneficially exclude certain larger diameter blood vessels and/or nerves from being lysed (possibly depending upon the anatomic site and/or angle of attack).

FIG. 1d depicts an embodiment of a modular TD connected to an electrosurgical 'pencil' shaft **102.1,** and an electrosurgical 'pencil' handle **103.1.** Rocker switch **103.2** located on or about handle **103.1** and/or shaft **102.1** may control the electrosurgical energy/energies derived from an electrosurgical generator (not seen in this view) brought into the handle via conduit **111.2.** The modular TD **100** is modular in that it is removable from shaft **102.1.** More particularly, the modular TD comprises a means for removably coupling the modular TD with an electrosurgical pencil shaft **102.1** at modular TD plug **103.** In the depicted embodiment, this coupling means comprises a modular TD plug **103.** In some embodiments, modular TD plug **103** may be threaded to facilitate a secure coupling between modular TD **100** and shaft **102.1.** However, in other embodiments, the coupling means may comprise a recess configured to receive a plug formed on the shaft. In still other embodiments, the coupling means may comprise a snap-fit coupling, a friction fit coupling, a bayonet clip, etc.

In the depicted embodiment, modular TD plug **103** is configured to be received within a corresponding recess **169** formed within shaft **102.1.** In some embodiments, elements within modular TD plug **103** and/or recess **169** may be electrically connected with electrical elements within shaft **102.1** and/or handle **103.1** which are in turn connected with switch **103.2** and/or conduit **111.2.** In some embodiments, modular TD plug **103** may be configured to electrically couple the modular TD with electrosurgical pencil shaft **102.1.** In this manner, in embodiments comprising, for example, electricity from a power source may be transmitted through the coupling between modular TD plug **103** and recess **169** to allow for energizing the lysing elements.

In some embodiments, modular TD **100** may be disposable as well, such that a surgeon can place an appropriate modular TD on the shaft and remove and dispose of the modular TD after surgery. Alternatively or additionally, a plurality of different modular TDs may be provided, each of which may be disposable, or may be configured for sterilization and re-use, and an appropriate modular TD may be selected as needed for a particular surgery.

In the depicted embodiment, modular TD tip **101** comprises a plurality of protrusions **104,** some of which are non-axial, and a plurality of recessions **105** positioned therebetween, as described above. In some embodiments a modular TD comprising a tip comprising only axial protrusions may be swapped for modular TD **100** as desired to suit a particular surgical procedure.

In an embodiment, the modular TD tip **101** may measure about 12mm in width, about 15mm in length and about 5mm in maximal thickness; the modular TD shaft **102** may measure about 4mm in diameter and/or about 20mm in length; the modular TD plug **103** may measure about 2.5mm in diameter and about 20mm in length; the overall modular TD **100** may measure about 12mm in width, 55mm in length and about 5mm in maximal thickness. Embodiments are contemplated wherein sizes of about one-fifth to about five times these dimensions may have possible uses. It is also contemplated, for example in some veterinary embodiments, tip sizes of about one-tenth to 20 times the aforementioned dimensions may also have possible uses. In some embodiments wherein electrical insulation and/or polymeric insulating coating is present on such parts, for example modular TD shaft **102,** such insulation may measure about 0.5mm in thickness; in some contemplated embodiments, the insulation thickness may range from 0.1mm to 3mm. In some embodiments, insulations may comprise materials that are both electrically non-conductive and of low thermal conductivity; such materials might comprise, for example, porcelain, ceramics, glass-ceramics, plastics, various halogenated carbon molecules, polytetrafluoroethylene, carbon, graphite, and graphite-fiberglass composites and the like.

In some embodiments, wherein the modular TD plug **103** may be manufactured with a circular cross section to plug into standard electrosurgical pencils with a corresponding circular cross section, it may be possible that torque forces resulting from moving the modular TD tip **101** through tissue may result in loosening of the fit and/or modular TD 100 rotations. In some embodiments, the TD plug **103** connection may be made tighter by things such as: screw threads and/or gnarling and/or cross-hatchings and/or a conductive textured surface and/or a jacket and/or a form-fit and/or snap-fit and/or an adhesive and/or a spear prong and/or macro or microscopic surface texture change and/or cross-sectional geometry and/or a 'jacket' and/or a hole (through and through, for a wire to be passed and spiral twisted to mimic a screw thread), etcetera in one or more portions of the receiving areas or the giving portions of the plug; such things may prevent rotation of the tip in/on certain devices. In contemplated embodiments, material may be welded onto one or more portions of the receiving areas and or giving portions of the plug to reduce and/or prevent rotation.

In some embodiments, loosening may be minimized by a brief polygonal cross section (sometimes comprised of a plastic and/or insulated surface zone) in the outer, more proximal surface layer of the shaft that fits that matches a manufactured corresponding receiving area in models of current 'pencils' (for example those made by ValleyLab).

FIG. 1e is a cross sectional view of line marking '**1e**' of the modular TD plug **103** found in FIG. 1c. As shown in FIG. 1e, in some embodiments, modular TD plug **103** may have a noncircular cross sectional shape, for example, in the embodiment shown in FIG. 1e comprises a hexagonal cross sectional shape however other polygonal cross sectional shapes comprising any number of flat or curved surfaces as desired. Some embodiments may also comprise a jacket configured to be received over a plug so as to, for example, transform a plug having a circular cross section into a plug having an alternative shape. Some embodiments comprising a hexagonal or other polygonal cross sectional shape may be useful for allowing a surgeon to orient the TD during a surgical procedure.

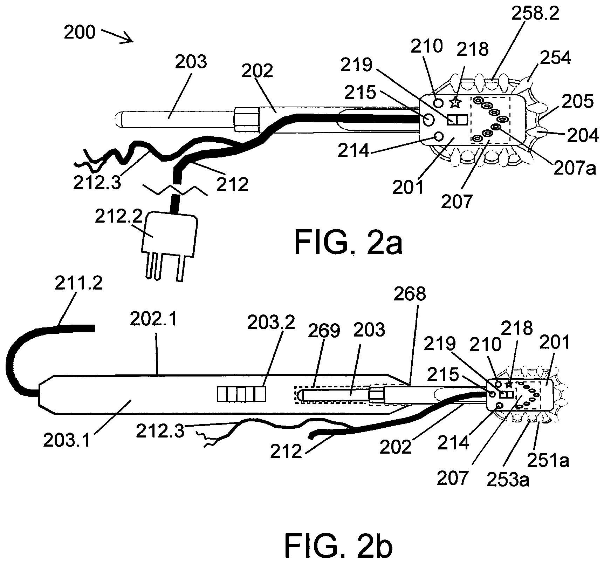

FIGs. 2a,b depict an embodiment of a modular Tissue Dissector & Modifier (modular TDM) **200** comprising a modular TDM tip **201,** a modular TDM shaft **202** and a modular TDM plug **203,** wherein some of the protrusions and lysing elements are oriented in non-axial directions. In other words protrusions, such as **251a,** are not oriented in a direction of the axis of modular TDM shaft **202.** In the depicted embodiment, modular TDM **200** comprises energy window **207** which may comprises electromagnetic energy emitting elements. In the depicted embodiment, elements **207a** may be configured to emit radiofrequency/electrosurgical energy such as RF energy. Energy window **207** may be configured with a plurality of energy emitting elements **207a.** In some such embodiments energy delivering elements **207a** may be spaced apart so as to provide for areas of energy delivery and interspersed areas of tissue sparing. It should be noted that the term "energy window" is intended to encompass what is referred to as a planar-tissue-altering-window/zone in U.S. Patent No. 7,494,488 and, as described herein, need not contain radiofrequency/electrosurgical energy emitting elements in all embodiments Additionally, the "energy window" may comprise a variety of other energy emitting devices, including but not limited to radiofrequency, microwave, light, intense pulsed light, LASER, thermal, thermochromic film and ultrasonic. Certain components of the energy window, such as the electro-conductive components of the energy window, could comprise a cermet. A second energy window may also be included in some embodiments, and may comprise yet another radiofrequency emitting device or another variety of energy emitting device. In some embodiments, energy windows **207** may only be substantially planar, or may take on other cross-sectional shapes that may correspond with a portion of the shape of the shaft, such as arced, stair-step, or other geometric shapes/curvatures. In the depicted embodiment, energy window **207** is adjacent to protrusions **204** and **251a,** however other embodiments are contemplated in which an energy window may be positioned elsewhere on the modular TDM shaft **202** or modular TDM tip **201** of the wand, and still be considered adjacent to protrusions **204** or **251a.** However, if an energy window was placed on modular TDM shaft **202,** such an energy window would not be considered adjacent to protrusions **204** or **251a.** It is contemplated that in alternative embodiments, either one or both of the energy windows may be omitted.

The depicted embodiment comprises a plurality of axial protrusions **204** (axially meaning at least substantially parallel to an axis of a corresponding TDM shaft). This embodiment further comprises a plurality of non-axial protrusions, such as **251a** along the right side of the tip and a plurality of non-axial protrusions positioned along the left side of the tip. The tip further comprises two non-axial corner or transitional protrusions **254.** The tip further comprises a plurality of recessions, such as **253a.** One or more of the recessions may further comprise a lysing element, such as **253a** & **205.**

In the depicted embodiment, modular TDM 200 comprising a modular TDM tip **201** with 3 different shaped lysing elements. Lysing element **205** is concave, lysing element **258.2** is straight as are the other lysing elements on the same side, lysing element **253a** is convex, as are the other lysing elements on its same side. Non-axial protrusions **251a** and the axial protrusion opposite it across the axial midline point in substantially opposite directions. In the depicted embodiment, the double lines on the lysing elements indicate a bevel that is sharpened. In other embodiments, the bevel is not required to be sharp. In other embodiments the lysing element may be a wire. In some embodiments, all the lysing elements are of the same shape. In some contemplated embodiments, one or more of the lysing elements may differ from the rest in shape and/or other characteristics.

In some embodiments, one or more sensors such as for example sensors **210** and **214** may be positioned on the device. The sensors **210** and **214** may comprise any of the sensors described in the specification herein. Other embodiments may comprise one or more sensors on any other suitable location on the TDM, including but not limited to on the protrusions or otherwise on the tip, and on the shaft. Such sensors may comprise for example thermal sensors, photoelectric sensors, photo optic sensors, and/or cameras, etc. In some embodiments, one or more sensors may be used to monitor the local post passage electrical impedance or thermal conditions that may exist near the distal tip of the shaft or on the tip. Some embodiments may also comprise one or more sensors incorporating MEMS (Micro Electro-Mechanical Systems) technology, such as MEMS gyroscopes, accelerometers, galvanometers, piezoelectrics, mechanical scanning elements, diffractive elements, acousto-optic elements, capacitive sensor arrays and the like. Such sensors may be positioned at any number of locations on the TDM, including within the handle in some embodiments. In some embodiments, sensor **214** may comprise fiberoptic elements. In an embodiment, the sensor can be configured to sense a temperature of tissue adjacent to the apparatus during one or methods described herein. The temperature sensor may alternatively be configured to sense a temperature of one or more fluids adjacent to the apparatus such as for example tissue fluids and/or fluids introduced by the surgeon.

Modular TDM tip **201** may further comprise Internal Tissue Optical Motion Sensor (ITOMSensor) **219.** ITOMSensor **219** may comprise one or more lenses and/or windows for emitting EMR and may further comprise one or lenses and/or windows for capturing or receiving reflected EMR.

In order to improve the ability of the TDM to track movement with respect to an internal tissue or region of tissue, such as the fatty undersurface of the skin, some embodiments may comprise an ITOMSensor comprising an electromagnetic emission source and a photodetector device. In, alternative embodiments, the internal tissue optical motion sensor may comprise a non-coherent light source. In, the depicted embodiment, the ITOMSensor comprises a coherent light source. Some such embodiments may comprise a motion detection algorithm configured to extract peaks & nulls for detected speckled light intensity patterns. One example of such an algorithm is disclosed in US patent 7,876,307 titled 'Motion Detection Mechanism for Laser Illuminated Optical Mouse Sensor' which is hereby incorporated by reference in its entirety Other examples of an optical sensor that may be used as an ITOMSensor are disclosed in US Patent Application publication number 2013/0016041 titled 'High Resolution Mouse' which is hereby incorporated by reference in its entirety. Other examples an optical sensor that may be used as an ITOMSensor are disclosed in US Patents: US7791590B1 titled: 'Optical Mouse With Uniform Level Detection & US 5,578,813 titled: 'Freehand Image Scanning Device Which Compensates For NonLinear Movement' & US 5,644,139 titled: 'Navigation For Detecting Movement Of Navigation Sensors Relative To An Object' & US5,786,804 titled: 'Method and System For Tracking Attitude,'. One or more steps, features, elements, or components disclosed in any of the above referenced documents which have been incorporated herein by reference may be combined with any other step disclosed in any of the other steps, features, elements, or components disclosed in any of the other documents disclosed herein incorporated by reference herein as would be apparent to one of ordinary skill in the art.

An ITOMSensor may comprise a source of non-coherent light which may be part of the motion sensor or located elsewhere on the surgical tool for illuminating a tissue at a low angle of incidence, a two dimensional array of photodetectors, each of the photo detectors producing an output in response to light reflected from surface irregularities in the tissue, and circuitry (which may be in the surgical tool or located remotely) to track movement of the housing relative to the work surface by comparing at least some of the photo detector outputs sensed at a first time with at least some of the photo detector outputs sensed at a second time if a particular condition in the photo detector outputs is identified. Motion produces successive frames of translated patterns of pixel information, which may be compared to determine the direction and distance moved.

One method for motion detection that may be used in connection with one or more embodiments disclosed herein may be based on based on the "Peak/Null Motion Detection" algorithm described in the International Patent Application WO_03/049018.

According to the "Peak/Null Motion Detection" algorithm a distinction is made between edges according to their "direction" which may be referred to as edge direction data. In some embodiments based upon the Peak/Null Motion Detection Algorithm, the ITOMSensor may comprise a coherent light source and a photodetector array. In connection with such embodiments the ITOMSensor may operate by illuminating under a determined gradient using a coherent light source a portion of tissue at a determined flash rate; detecting by means of the photodetector array a reflected speckled light intensity pattern from the illuminated portion of the internal tissue surface for a first flash; detecting a second reflected speckled light intensity pattern of the illuminated portion of the internal tissue surface for a second flash; extracting motion features of two different types from the detected first and second speckled light intensity patterns; determining a measurement of the relative motion between the surgical tool and the illuminated portion of the internal tissue surface based on a comparison of motion features extracted. In some embodiments, before the step of determining the measurement a preliminary step may be performed to modify or adjust the gradient under which the surface is illuminated. In some such implementations, the gradient may be modified or adjusted by adjusting the position of the coherent light source and/or moving an optical lens that may be used to obtain an optical gradient. In some implementations, the step of extracting motion features may comprise comparing light intensity using an offset between adjacent pixels derived from speckled light intensity patterns and extracting motion features using this comparison. In some further implementations, an intensity threshold may be defined without extracting motion features; such an intensity threshold may be adjustable. In other implementations, a plurality of intensity thresholds may be defined. In some embodiments, the method may comprise keeping only pairs of neighboring Peaks and Nulls and ignoring individually occurring Peaks and Nulls.

In some embodiments, ITOMSensor **219** may comprise for example one or more elements described in the aforementioned reference. In alternative embodiments ITOMSensor 219 may be located elsewhere on modular TDM **200** such as for example on shaft **202.** In some embodiments, ITOMSensor **219** may be coupled with lysing elements and/or energy window to facilitate controlled delivery of energy to tissues. For example, some embodiments may be configured to deliver a preset amount of energy such that when the device is moved faster a larger amount of energy is delivered per unit time, and when the device is moved more slowly the amount of energy is automatically decreased so as to provide a more uniform or at least semi-uniform (at least within a predetermined range) delivery of energy per unit area. In some embodiments, motion **219 sensor** may further or alternatively be coupled with sensor **210** and/or **214** for example, in embodiments comprise a temperature sensor; temperature data may be used along with data from ITOMSensor **219** in order to adjust energy output to energy window **207** and/or lysing elements.

Temperature and impedance values may be tracked on a display screen or directly linked to a microprocessor capable of signaling control electronics to alter the energy delivered to the tip when preset values are approached or exceeded. Typical instrumentation paths are widely known, such as thermal sensing thermistors, and may feed to analog amplifiers which, in turn, feed analog digital converters leading to a microprocessor. In some embodiments, internal or external ultrasound measurements may also be taken during a procedure with the TDM. Sensors that may be useful include thermal sensors, photoelectric or photo optic sensors, cameras, etc. Temperature sensors that may be useful in connection with embodiments disclosed herein may comprise, but are not limited to, resistance temperature sensors, such as carbon resistors, film thermometers, wire-wound thermometers, or coil elements. Some embodiments may comprise thermocouples, pyrometers, or non-contact temperature sensors, such as total radiation or photoelectric sensors.

In some embodiments, one or more electromagnetic delivery elements **215** may be positioned on tip or shaft. Other embodiments may comprise one or more electromagnetic delivery elements on any other suitable location on the TDM, including but not limited to on the protrusions or otherwise on the tip, and on the shaft. Electromagnetic delivery elements that may be useful include: LEDs, LASERs, fiberoptics, filaments, photoelectric materials, infrared emitters, etc. One or more additional conduits **212.3** may be used to transmit data from one or more locations on the modular TD **200** such as for example sensors **210, 214, 216, 219;** conduit **212.3** may further be configured to deliver signals and/or energy to modular TD **200.** In alternative embodiments separate conduits may be used for delivery of signals and/or energy from conduits used to extract data from modular TD.

In embodiments of tips with at least some non-axial placement of protrusion and or relative recessions, surgeons may implement the use of a fanning motion which may comprise a 'windshield wiper' motion.

In the embodiment depicted in FIGs. 2a,b antenna **218** comprises an RFID TAG. In embodiments in which antenna **218** comprises an RFID tag, the RFID tag may comprise a RFID transponder. In other embodiments the RFID tag may comprise a passive tag. It should be understood that although antenna **218** is not depicted in every one of the other figures, any of the embodiments described herein may include one or more such locations. Other embodiments may comprise one or more antennas on any other suitable location on the TDM, including but not limited to on the protrusions or otherwise on the tip, and on the shaft. In some embodiments an RFID transponder or other antenna may comprise a microchip such as a microchip having a rewritable memory. In an embodiment the tag is millimeter sized. In some embodiments a reader generates an alternating electromagnetic field which activates the antenna/RFID transponder and data is sent via frequency modulation. In an embodiment, the position of the antenna/RFID tag is determined by an alternating electromagnetic field in the ultra-high frequency range. The position may be related to a 3 dimensional mapping of the subject. In an embodiment the reader may generate an alternating electromagnetic field. In a further embodiment the alternating electromagnetic field may be in the shortwave (13.56MHz) or UHF (865-869MHz) frequency.

In some embodiments, a modular TDM **200** may be attached to a robotic arm. In some embodiments modular TDM tip **201** and portion of modular TDM shaft **202** may be attached to a robotic arm. In some embodiments modular TDM tip **201** and a portion of modular TDM shaft **202** and/or a portion of modular TDM plug **203** may be attached to a robotic arm.

Said modular TDM are not intended to be restricted to symmetry and/or pattern and/or dimension. In other embodiments said modular TDM may be asymmetrical or lacking protrusions and/or lysing elements on one side or another.

One or more electrosurgical conduits may extend from **200.** For example, in the embodiment depicted in Figs 2a,b conduit **212** may extend to source of electrosurgical energy and may use connector **212.2** to plug into the source. A separate sensor conduit may be used to send and/or receive one or more signals, energy or related data or materials to be used in connection with one or more of the sensors on the device.

In the depicted embodiment, electrosurgical 'pencil' comprises an electrosurgical 'pencil' shaft **202.1,** electrosurgical 'pencil' handle **203.1** & rocker switch **203.2** located on or about 'pencil' handle **203.1** and/or 'pencil' shaft **202.1** which may control the electrosurgical energy such as suitable electrosurgical waveform derived from an electrosurgical generator (not seen in this view) brought into the handle via conduit **211.2.** In some embodiments, modular TDM **200** is modular in that it may removable from electrosurgical 'pencil' shaft **202.1.** More particularly, modular TDM **200** comprises a means for removably coupling the tip with an electrosurgical 'pencil' shaft **202.1** at **268.** In the depicted embodiment, this coupling means comprises a modular TDM tip plug **203.** In some embodiments, modular TDM tip plug **203** may be threaded to facilitate a secure coupling between modular TDM **200** and 'pencil' shaft **202.1.** However, in other embodiments, the coupling means may comprise a recess configured to receive a plug formed on the shaft. In still other embodiments, the coupling means may comprise a snap-fit coupling, a friction fit coupling, a bayonet clip, etc.

With regards to the term 'electrosurgical pencil,' the following is taken from the publication of Bovie Medical "Understanding Electrosurgery" © 2010 written by Genard McCauley page 7: "Monopolar electrosurgery has the means of delivering energy to the tissue through several modalities (modes of operation): pure cut, blended cut, desiccation or pinpoint) and spray (or fulguration). The delivery system of the monopolar electrosurgical generator can be a hand controlled pencil (reusable or disposable) or a foot controlled pencil. A number of accessories can be adapted to the foot control output jack to deliver energy through a number of instruments.

In the depicted embodiment modular TDM tip plug **203** is configured to be received within a corresponding electrosurgical 'pencil' shaft recess **269** formed within electrosurgical 'pencil' shaft **202.1.** In some embodiments, elements within modular TDM tip plug **203** and/or recess **269** may be electrically connected with electrical elements within 'pencil' shaft **202.1** and/or 'pencil' handle **203.1** which are in turn connected with 'pencil' switch **203.2** and/or conduit **211.2.** In some embodiments, modular TDM tip plug **203** may be configured to electrically couple modular TDM tip **201** with 'pencil' shaft **202.1.** In this manner, in embodiments comprising, for example, lysing elements, electricity from a power source may be transmitted through the coupling between modular TDM tip plug **203** and 'pencil' recess **269** to allow for energizing the lysing elements.

In some embodiments, modular TDM **200** may be disposable as well, such that a surgeon can place an appropriate tip on the shaft and remove and dispose of the tip after surgery. Alternatively or additionally, a plurality of different tips may be provided, each of which may be disposable, or may be configured for sterilization and re-use, and an appropriate tip may be selected as needed for a particular surgery.

In the depicted embodiment, modular TDM tip **201** comprises a plurality of protrusions **204,** some of which are non-axial, and a plurality of recessions that may contain lysing elements **205** positioned therebetween, as described above. In some embodiments, a modular TDM and/or a modular TDM tip comprising only axial protrusions may be swapped for modular TDM tip **201** as desired to suit a particular surgical procedure.

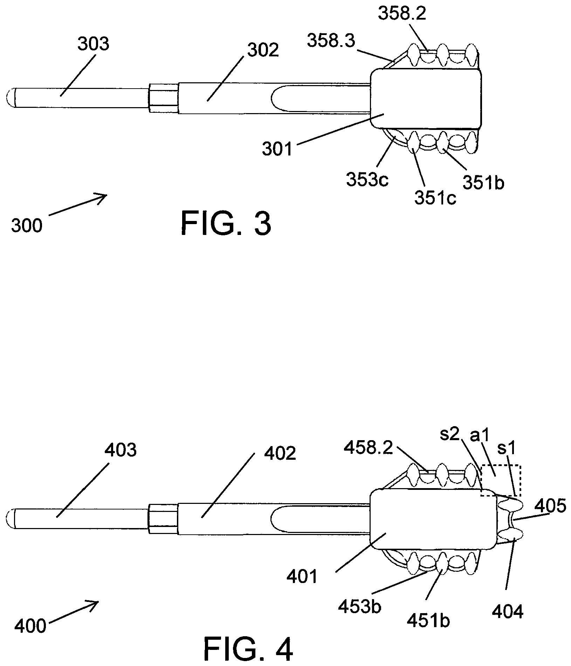

FIG. 3 is an upper plan view illustrating an embodiment of a modular Tissue Dissector (modular TD) **300** comprising a modular TD tip **301,** a modular TD shaft **302** and a modular TD plug **303,** wherein the tip is lacking in axial protrusions and lysing elements. In the depicted embodiment, protrusions and lysing elements are however oriented in non-axial directions. In other words, protrusions **351b**, **351c**, & **353c** are not oriented in a direction of the axis of modular TD shaft **302.** The embodiment depicted in FIG. 3 also lacks in transitional protrusions. Transitional protrusions, axial protrusions, non-axial protrusions, axial lysing elements, non-axial lysing elements and lysing elements of differing geometries are discussed in more detail elsewhere in this application. Embodiments having only non-axial protrusions may be useful in 'open' surgical procedures wherein a surgeon may predominately move the TD in a side to side procedure; such embodiments may also be useful where a blunt non-energized probe front is able to separate and/or pass through and/or around a particular type of tissue; such embodiments may also be easier to manufacture from a cost and/or other perspective. Such an embodiment lacking in axial protrusions may be more difficult to move in a forward direction through, for example, more fibrous and/or dense tissues. In the depicted embodiment, non-axial lysing element **358.2** is straight as are the other lysing elements such as **358.3** on the same side; lysing element **353c** is convex, as are the other lysing elements on its same side. In other contemplated embodiments, a variety of geometrically shaped lysing elements and/or protrusions may co-exist side by side. For example, in some embodiments, two adjacent protrusions may differ in shape and or size, 2 adjacent lysing elements may differ in length and or shape and/or TD tip **301** may otherwise be nonsymmetrical in one or more views..In some embodiments, the number of protrusions per side may range from 2 to 10. In some embodiments the number of protrusions per side may range from 1 to 99. In some embodiments, the modular TD tip without axial protrusions may measure and/or range in sizes similar to previously discussed embodiments in this application.

In some embodiments, non-axial protrusion such as **351b** may extend at an angle of between zero degrees and 30 degrees of a normal to the direction in which the axes of shaft **302** and/or tip **301** point. It is contemplated that it may be desirable for some implementations and/or embodiments to provide non-axial tips extending in a direction or directions falling within this range in order to, for example, allow a surgeon to effectively perform both a to and fro, and a side-to-side ("windshield wiper") motion using the TD.

FIG. 4 is an upper plan view illustrating an embodiment of a modular Tissue Dissector (modular TD) **400** with a modular TD tip **401,** a modular TD shaft **402** and a modular TD plug **403,** wherein the tip is lacking in transitional protrusions and transitional lysing elements. Protrusions and lysing elements, however, may be oriented in axial and/or 'normal' (perpendicular) directions. In other words, non-axial protrusion **451b** is substantially oriented in a 'normal' to direction (substantially perpendicular to) the axis of modular TD shaft **402;** and axial protrusion **404** axes are substantially parallel to the axes of modular TD shaft **402** and/or modular TD tip **401.** Transitional protrusions, axial protrusions, non-axial protrusions, axial lysing elements, non-axial lysing elements and lysing elements of differing geometries are discussed in more detail elsewhere in this application. Embodiments having only non-axial and axial protrusions may be useful in 'open' surgical procedures wherein a surgeon may predominately move the TD predominately side to side and/or forward directions without moving in intermediate angles for a particular procedure; such embodiments may also be useful where tissues are relatively soft or tissue planes are more easily maintained and/or well defined. Such embodiments may also be a bit easier to manufacture from a cost and/or other perspective. Such an embodiment lacking in transitional protrusions may be more difficult to move in a directions ranging around 45 degrees from the modular TD shaft axis. In the depicted embodiment, non-axial lysing element **458.2** is straight and opposing side lysing elements, such as **453b,** are convex; whereas axially directed lysing element **405** is concave. In other contemplated embodiments, a variety of geometrically shaped lysing elements and/or protrusions may co-exist side by side. For example, in some embodiments, two adjacent protrusions may differ in shape and/or size, 2 adjacent lysing elements may differ in length and/or shape and/or TD tip **401** may otherwise be nonsymmetrical in one or more views. In some embodiments, non-axial protrusion such as **451b** may extend at an angle of between zero degr**ees** and 30 degrees of a normal to the direction in which the axes of shaft **402** and/or tip **401** point. It is contemplated that it may be desirable for some implementations and/or embodiments to provide non-axial tips extending in a direction or directions falling within this range in order to, for example, allow a surg**eon** to effectively perform both a to and fro, and a side-to-side ("windshield wiper") motion using the TD. In some embodiments, the number of protrusions per side may range from 2 to 10. In some embodiments the number of protrusions per side may range from 1 to 99. In some embodiments, the modular TD tip without axial protrusions may measure and/or range in sizes similar to previously discussed embodiments in this application. In the depicted embodiment, **a1** is the area of a rectangle representing a space remaining if there is no transitional protrusion and/or lysing element; **s1** represents the side parallel to the axis of the modular TD shaft whereas **s2** represents the side perpendicular to **s1**. In some contemplated embodiments, **a1** may contain axial or transitional protrusions pointing at angles within about 30 degrees of **s1** and may populate most of the area depicted in **a1.** In some embodiments, **a1** may contain non-axial or transitional protrusions pointing at angles within about 30 degrees of **s2** populating most of area **a1.** In some embodiments, the same options discussed with reference to **a1** may similarly be positioned on a space on the opposite side of modular TD tip **401** relative to a1.

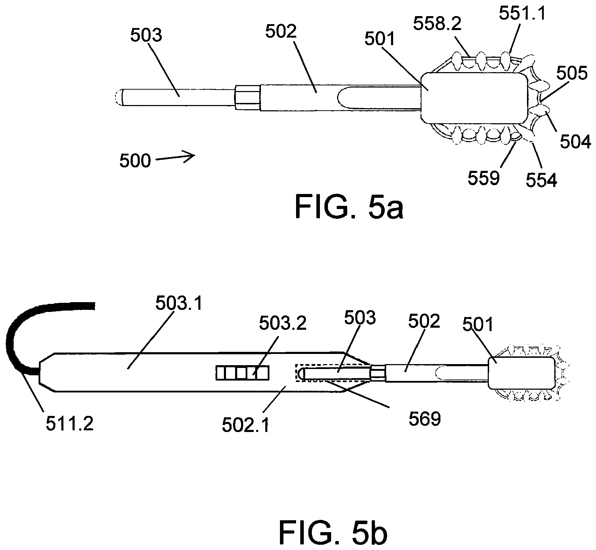

FIG. 5a is an upper plan view illustrating an embodiment of a modular Tissue Dissector (modular TD) **500** comprising a modular TD tip **501,** a modular TD shaft **502** and a modular TD plug **503,** wherein the tip lacks a means for spot coagulation. Notably, the depicted embodiment lacks a spot coagulating lysing element. The depicted embodiment also comprises axial protrusion **504,** transitional protrusion **554** and non-axial protrusion **551.1,** non-axial, straight lysing element **558.2,** concave axial lysing element **505** and convex lysing element **559.** In the depicted embodiment convex lysing element **559** does not protrude substantially relative to other electrosurgically energizable lysing elements; these features may discourage use of the lysing elements for spot coagulation. In the depicted embodiment, convex lysing element **559** may also be somewhat shielded from significant tissue exposure on forward motion to restrict its ability to be forwardly pushed toward a bleeding area to spot coagulate said area; as well an approach from an non-axial motion may be difficult in that the closest non-axial protrusion may also provide some shielding. Transitional protrusions, axial protrusions, non-axial protrusions, axial lysing elements, non-axial lysing elements and lysing elements of differing geometries are discussed in more detail elsewhere in this application. Embodiments lacking protruding spot coagulation lysing elements may be useful in 'open' surgical procedures wherein little bleeding is expected; such embodiments may also be a bit easier to manufacture from a cost and/or other perspective. In other contemplated embodiments, a variety of geometrically shaped lysing elements and/or protrusions may co-exist side by side. For example, in some embodiments, two adjacent protrusions may differ in shape and/or size, 2 adjacent lysing elements may differ in length and/or shape and/or TD tip **501** may otherwise be nonsymmetrical in one or more views.

FIG. 5b is an upper plan view of a Modular TD **500** that may be removable from an electrosurgical 'pencil' shaft and/or an electrosurgical 'pencil' handle **503.1.** Rocker switch **503.2** may control the electrosurgical energy such as a suitable waveform derived from an electrosurgical generator (not seen in this view) brought into the handle via conduit **511.2.**

In the depicted embodiment modular TDM tip plug **503** is configured to be received within a corresponding electrosurgical 'pencil' shaft recess **569** formed within electrosurgical 'pencil' shaft **502.1.** In some embodiments, elements within modular TDM tip plug **503** and/or electrosurgical 'pencil' shaft recess **569** may be electrically connected with electrical elements within 'pencil' shaft **502.1** and/or 'pencil' handle **503.1** which are in turn connected with 'pencil' switch **503.2** and/or conduit **511.2.**

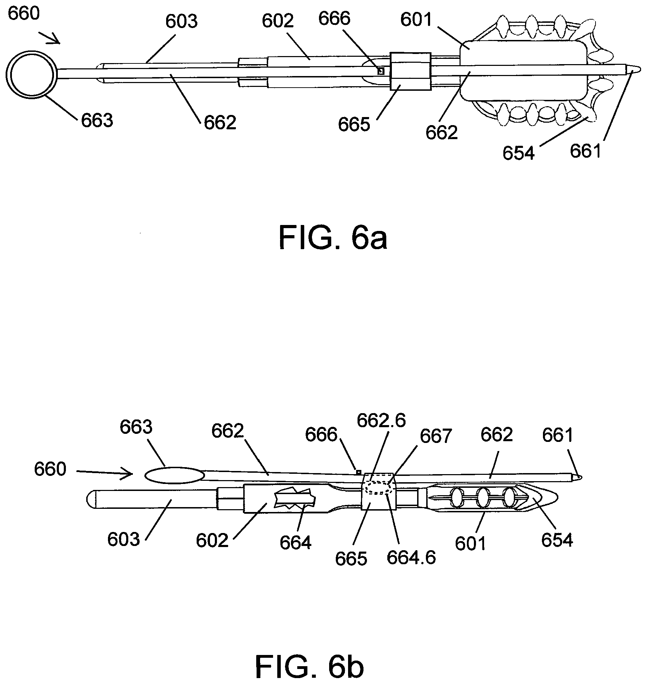

FIG. 6a is an upper plan view of an embodiment of modular TD with a spot coagulator (SC). Modular TD comprises a Modular TD tip **601,** a Modular TD shaft **602,** a Modular TD plug **603** and spot coagulator **660**. In the depicted embodiment, Modular TD tip **601** comprises transitional protrusions **654**. Spot coagulator **660** comprises an SC tip **661**, SC shaft **662** and SC handle **663**. In the depicted embodiment, modular TD shaft **602** may comprise an insulating and/or supportive coating and/or cover overlying a modular TD shaft conductive core. In the depicted embodiment, SC shaft **662** is slidably coupled to the modular TD shaft **602** of the modular TD by SC coupler **665.** SC coupler **665** is one example of a means for moveably coupling a SC with a surgical tool. SC coupler **665** may be configured to allow SC shaft **662** to slide relative to SC coupler **665** and modular TD shaft **602.** In the depicted embodiment, SC coupler **665** is rigidly affixed to SC shaft **662.** In contemplated embodiments, SC coupler **665** may be configured to slide relative to TD shaft **602.** In some embodiments, SC coupler **665** may comprise a sterilizable plastic and/or polymer and/or ceramic and/or polytetrafluoroethylene and/or other non-conductive and/or insulating material. In some embodiments, SC coupler **665** may comprise an integral part of modular TD shaft **602.** In other embodiments SC coupler **665** may comprise a separate component that may be coupled with modular TD shaft **602** by way of for example form fitting, snap fitting, an adhesive, welding, and the like. One or more passageways in SC coupler **665** may allow the SC shaft **662** to pass therethrough. In some contemplated embodiments, SC coupler **665** may be formed from and/or be an integral part of modular TD shaft **602;** in some of these embodiments, SC coupler **665** may comprise the same insulating materials of modular TD shaft **602.** In other contemplated embodiments, SC coupler **665** may be formed from and/or be an integral part of SC shaft **662;** in some of these embodiments, SC coupler **665** may comprise the same insulating materials of SC shaft **662.** The more distal end of the SC is the SC tip **661** and the more proximal (toward the surgeon) end of the SC is SC handle **663.** The term handle does not necessarily specify it is for the entire hand of the surgeon as just one finger is possible to control the SC in some embodiments. In the embodiment depicted in FIGS. 6a,b, the SC handle **663** comprises a ring that may be operated by a single finger of a surgeon. In this depicted embodiment the SC tip **661 e**xtends from the SC shaft **662** and is conductive and not insulated along at least a portion of the tip. In some embodiments, the entire tip may be conductive. So as not to have the surgeon set down the TD or TDM, to pick up other instrumentation from the surgical tray to stem bleeding blood vessel(s), it may be beneficial during some surgical procedures to have spot coagulator coagulation capabilities within the same instrument.

FIG 6b is a depiction of an embodiment revealing a 'break away' phantom in the modular TD shaft **602** which shows a portion **664** of the metal center of the shaft as it passes from the TD plug area to TD tip. In some embodiments, the modular TD plug **603** may comprise a continuation of the metal center of modular TD shaft **602;** in some embodiments, the diameter may be noncircular and/or irregular on a macroscopic and/or microscopic scale to prevent unwanted rotation. In the depicted embodiment limiting ridge **666** may be present on or about SC shaft **663** to prevent further movement of the SC tip **661** a given distance beyond the modular TD tip **601.** In the depicted embodiment the SC tip is restricted to 10mm protrusion beyond the distalmost portion of modular TD tip **601,** which may comprise the end of one of the protrusions on modular TD tip **601.** In other contemplated embodiments, a bend in the SC shaft or SC coupler passageway and/or size mismatch and/or tether, etc., may also be used to limit the distance SC tip **661** may protrude beyond the distal end of modular TD tip **601.** In other contemplated embodiments, no elements may restrict the working movement range of the SC shaft **662.**

FIG 6b also depicts in phantom 3 elements: internal components of SC coupler **665** including SC shaft conductive opening **662.6** in SC shaft **662,** and modular TD shaft conductive opening **664.6** in modular TD shaft **602,** and SC coupler conductive spring **667.** In the depicted embodiment, SC shaft **662** may comprise one or more non-insulated area(s) (usually on the underside) that may be brought into contact with SC coupler conductive spring **667** which may electrically couple with the modular TD shaft conductive opening **664.** For example, some embodiments may comprise a plurality of adjacent conductive rings or other non-insulated areas such that SC tip **661** may be positioned at a plurality of preset distances from modular TD tip. SC coupler conductive spring **667** may comprise a metal and/or other conductive material such as metal-coated/impregnated plastic and/or polymer and may have a variety of shapes. SC shaft conductive opening **662.6** and/or modular TD shaft conductive opening **664.6** may comprise metal portions of their respective shafts lacking overlying insulation in the area of the opening. In contemplated embodiments, SC coupler conductive spring **667** may be an integral part of SC coupler **665** and/or modular TD shaft **602.** In the depicted embodiment, the SC coupler conductive spring **667,** may comprise a flexible metal having an oval cross section that may be welded and/or affixed to modular TD shaft **602** by any means known in the art.

An implementation using the depicted embodiment may involve pushing distally SC handle **663** which forces SC shaft **662** distally through SC coupler **665** whereupon SC shaft conductive opening **662.6** is brought into direct contact with SC coupler conductive spring **667** which itself may be in direct contact with modular TD shaft conductive opening **664.6.** Thus electrosurgical energy such as suitable electrosurgical waveform will be delivered, when the electrosurgical generator is activated, via modular TD plug 603, into modular TD shaft **602,** into modular TD shaft conductive opening **664.6** into SC coupler conductive spring **667** into SC shaft conductive opening **662.6** into SC shaft **662** and thereupon to SC tip **661** and then into target tissue. In the depicted embodiment, the SC tip **661** extends from the SC shaft **662** and is conductive and not insulated along at least a portion of the tip. In some embodiments, the entire tip is conductive. In the depicted embodiment SC shaft **662** may comprise stainless steel and may be round in cross-section. In the depicted embodiment, modular TD shaft **602** may comprise an electrically insulating and/or supportive coating and/or cover overlying a conductive material and/or metal axial core. The electrical insulator may comprise, for example, porcelain, ceramics, glass-ceramics, plastics, various halogenated carbon molecules, polytetrafluoroethylene, carbon, graphite, and graphite-fiberglass composites and the like. In some embodiments, the conductive material may comprise: steel, nickel, alloys, palladium, gold, tungsten, silver, copper, platinum and/or any other conductive metal that does not give off toxic residua at operating temperatures. In other contemplated embodiments, the conductive material may comprise cermets and the like. In the depicted embodiment, SC tip **661** is shaped like the frustum of a cone. In some embodiments, SC shaft **662** may be oval, flat, rectangular or geometric in cross-section or substantially flattened. In alternative embodiments, SC tip **661** may be pointed, bullet shaped, or geometric in cross section; more angulate and/or pointed tips may disperse electrical energy more readily and allow greater precision than larger, more rounded tip designs. In the depicted embodiments of the SC shaft **662,** the electrical insulator may comprise polytetrafluoroethylene. In alternative embodiments the electrical insulator may comprise polyether etherketone and/or polysulfone and/or another electrically nonconductive polymers (with thermal stability in the operating range) and/or materials that are both electrically non-conductive and of low thermal conductivity. In contemplated embodiments the electrical insulator may comprise, for example, porcelain, ceramics, glass-ceramics, plastics, various halogenated carbon molecules, polytetrafluoroethylene, carbon, graphite, and graphite-fiberglass composites.

Although the depicted embodiment shows a manually deployed SC, other contemplated embodiments may allow deployment to be (including but not limited to): motorized and/or spring activated and/or screw driven and/or ratchet style and/or cog style and/or pneumatic and/or hydraulic, etc. Although, the embodiment depicted in Fig. 6b shows the SC shaft **662** pointing about axially (relative to the modular TD axis) and/or passing about over the middle of the front of the modular TD tip **601,** in other contemplated embodiments, SC shaft **662** may point at an angle within about 30degrees of the axis of the modular TD and/or may pass over any other points on the modular TD tip **601** besides the middle front of the modular TD tip. In other contemplated embodiments, SC shaft **662** may point at angles greater than 30degrees of the axis of the modular TD. In some embodiments, SC shaft **662** may be flexible and its forward axis redirected upon passing through SC coupler **665;** in some embodiments SC coupler may pivot and/or rotate and/or have one or more non-axial passageways for SC shaft **662** that may redirect the axis and/or pointing of the forward part of SC shaft **662.** For example, some embodiments may comprise a plurality of SC coupler passageways for SC shaft **662** each pointing in a different direction. In such embodiments one or more such passageways may be axial and one or more may be non-axial. In the depicted embodiment, the SC tip **661** and SC shaft **662** together may measure about, 55mm in length and about 3mm in maximal thickness; the SC handle **663** may measure about 20mm in diameter and about 3mm in maximal thickness; the overall SC **660** may measure about 75mm in length. In embodiments wherein an electrical insulation and/or polymeric insulating coating are present on such parts, for example on SC shaft **662** and/or SC handle **663,** such insulation may measure about 0.5mm in thickness. In some embodiments, the insulation thickness may range from about 0.1mm to 3mm. Embodiments are contemplated wherein sizes of about one-fifth to about five times these dimensions may have possible uses. It is also contemplated in some veterinary embodiments; tip sizes of about one-tenth to 20 times the aforementioned dimensions may also have possible uses.

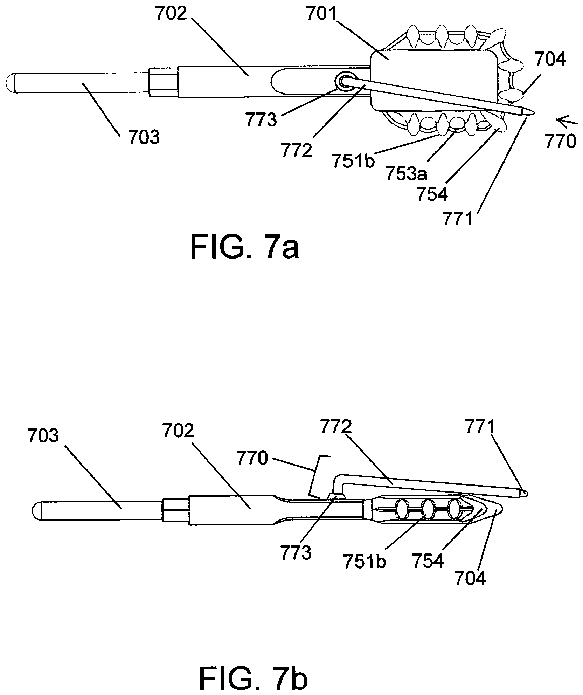

FIG. 7a is an upper plan view of an embodiment of modular TD with a spot coagulator boom. In the depicted embodiment, the Modular TD comprises a Modular TD tip **701,** a Modular TD shaft **702,** a Modular TD plug **703** and spot coagulator boom (SCB) **770.** The depicted embodiment also comprises axial protrusion **704,** transitional protrusion **754** and non-axial protrusion **751b**, and non-axial convex lysing element **753a**. Transitional protrusions, axial protrusions, non-axial protrusions, axial lysing elements, non-axial lysing elements and lysing elements of differing geometries are discussed in more detail elsewhere in this application. In other contemplated embodiments, a variety of geometrically shaped lysing elements and/or protrusions may co-exist side by side. For example, in some embodiments, two adjacent protrusions may differ in shape and/or size, 2 adjacent lysing elements may differ in length and/or shape and/or TD tip **701** may otherwise be nonsymmetrical in one or more views. In the depicted embodiment, modular TD shaft **702** may comprise an electrically insulating and/or supportive coating and/or cover overlying a conductive material and/or metal axial core; the electrical insulator may comprise, for example, porcelain, ceramics, glass-ceramics, plastics, various halogenated carbon molecules, polytetrafluoroethylene, carbon, graphite, and graphite-fiberglass composites and the like. SCB tip **771** shapes may comprise those discussed in other embodiments in this application. Spot coagulator boom **770** comprises a SCB tip **771,** SCB shaft **772** and SCB base **773.** In the depicted embodiment, the SCB tip **771** extends from the SCB shaft **772** and is conductive and not insulated along at least a portion of the tip. In some embodiments, the entire tip is conductive. In the depicted embodiment, SCB Base **773** permits SCB shaft **772** to pivot about base. In some embodiments, SCB shaft **772** may pivot 360 degrees and in other embodiments, it may be limited to a specific arc, for example some embodiments may be configured to rotate about 180 degrees. Some embodiments may be configured to automatically couple an electric connection at a particular position, for example, some embodiments may be configured such that the spot coagulation boom (SCB) **772** is not electrically connected when the spot coagulator extends backwards toward the modular TD plug **703** but upon reaching a threshold amount of rotation the SCB **770** automatically connects with an electrical source. Alternatively, rotation of the SCB shaft **772** may be independent of the electrical coupling, in such embodiments, a switch may be provided if desired to couple and decouple electrosurgical energy to SCB tip **771.** It is contemplated in some embodiments that a snap or hook may be deployed on the modular TD shaft **702** and/or electrosurgical 'pencil' that may not only insulate, but provide storage for SCB tip **771** but may clean tip on docking, and may also prevent from inadvertently deploying or coming loose during dissection in close quarters (areas of limited surgical workspace, for example between organs or in a 'pocket' of tissues) or more dense tissues.

In the depicted embodiment of FIG. 7b, SCB shaft **772** is angled with respect to the modular TD such that in an operational position as shown in Fig. 7b, tip **771** is angled downward such that SCB tip **771** extends beyond the distal tip of the modular TD and may intersect the axis of the modular TD shaft **702.** In such embodiments, when rotated around to a storage position, SCB shaft **772** may extend at a greater angle away from the axis of the modular TDM shaft **702** to provide additional clearance between SCB shaft **772** and modular TDM shaft **702.** Such embodiments may be desirable in certain procedures in such that SCB tip **771** may be in a better position for spot coagulation procedures than embodiments in which SCB shaft **772** is parallel to the modular TD shaft **702.** Some embodiments may be configured such that SCB shaft **772** is rotatable to discrete number of positions about the base **773** or otherwise with respect to modular TD shaft **702.** In alternative embodiments SCB base **773** may be configured to pivot with SCB shaft **772** about modular TD shaft **702.** In other contemplated embodiments, SCB **770** may be coupled with other portions of the modular TD such as modular TD tip **701** for example.

In the depicted embodiment, SCB tip **771** and SC shaft **772** together may measure about 30mm in length and about 2mm in maximal thickness; SCB base **773** may measure about 4mm in diameter and about 3mm in height. In embodiments wherein an electrical insulation and/or polymeric insulating coating is present on such parts, for example SCB shaft **772** and/or SCB base **773** such insulation may measure about 0.5mm in thickness. In some embodiments, the insulation thickness may range from 0.1mm to 3mm. Embodiments are contemplated wherein sizes of about one-fifth to about five times these dimensions may have possible uses. It is also contemplated in some veterinary embodiments; tip sizes of about one-tenth to 20 times the aforementioned dimensions may also have possible uses.

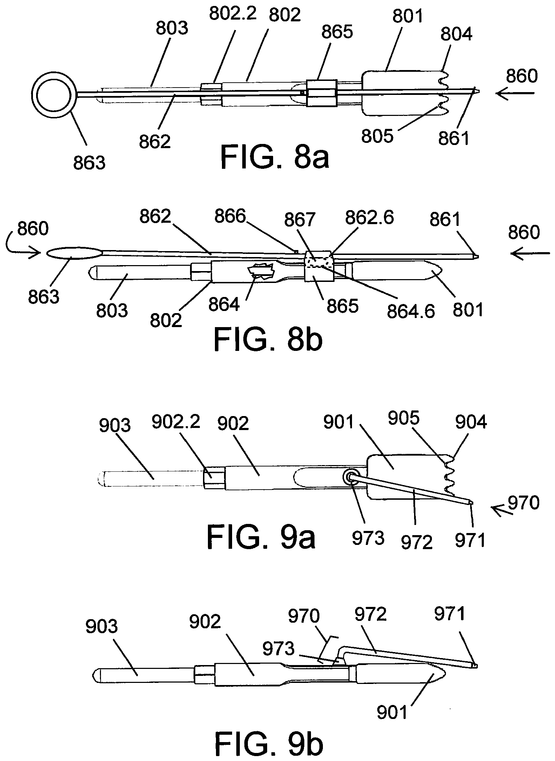

FIGs. 8a,b depict an embodiment that differs from the embodiment depicted in FIGs. 6a,b in that it comprises only axial protrusions **804** and accompanying lysing elements **805.** Each of the other elements depicted in FIGs. 8a,b may be identical to the corresponding elements shown in FIGs. 6a,b and are referenced by like numerals (numbers higher by 200). For example, in FIGs. 8a,b: Modular TD comprises a Modular TD tip **801,** a Modular TD shaft **802,** a Modular TD plug **803** & {spot coagulator (SC) **860** (further comprises: SC tip **861,** SC shaft **862** & SC handle **863**)}. SC coupler **865** is one example of a means for moveably coupling a SC with a surgical tool. SC coupler **865** may be configured to allow SC shaft **862** to slide relative to SC coupler **865** and modular TD shaft **802.** In the depicted embodiment, SC coupler **865** is rigidly affixed to SC shaft **862.** In contemplated embodiments, SC coupler **865** may be configured to slide relative to TD shaft **802.** In some embodiments, SC coupler **865** may comprise an integral part of modular TD shaft **802.** In other embodiments SC coupler **865** may comprise a separate component that may be coupled with modular TD shaft **802** by way of for example form fitting, snap fitting, an adhesive, welding, and the like.

FIG. 8b is a depiction of an embodiment revealing a 'break away' in the modular TD shaft **802** which shows a portion **864** of the metal center of the shaft; limiting ridge **866** may be present on or about SC shaft **862.** To limit or prevent any rotation of TD plug **803** within a shaft that is a source of electrosurgical energy, the section of SC shaft **802** may comprise SC shaft insert **802.2** that may take any geometric shape and be inserted into the source of electrosurgical energy mating with a complementary shape therein.

FIG 8b also depicts in phantom 3 elements: internal components of SC coupler **865** including SC shaft conductive opening **862.6** in SC shaft **862,** and modular TD shaft conductive opening **864.6** in modular TD shaft **802,** and SC coupler conductive spring **867.**

Although the depicted embodiment shows a manually deployed SC, other contemplated embodiments may allow deployment to be (including but not limited to): motorized and/or spring activated and/or screw driven and/or ratchet style and/or cog style and/or pneumatic and/or hydraulic, etc. Although, the embodiment depicted in Fig. 8b shows the SC shaft **862 p**ointing about axially (relative to the modular TD axis) and/or passing about over the middle of the front of the modular TD tip **801,** in other contemplated embodiments, SC shaft **862** may point at an angle within about 30degrees of the axis of the modular TD. In other contemplated embodiments, SC shaft **862** may point at angles greater than 30degrees of the axis of the modular TD. In some embodiments, SC coupler may pivot and/or rotate and/or have one or more non-axial passageways for SC shaft **862** that may redirect the axis and/or pointing of the forward part of SC shaft **862.** For example, some embodiments may comprise a plurality of passageways for SC shaft **862** each pointing in a different direction. In such embodiments one or more such passageways may be axial and one or more may be non-axial. In the depicted embodiment of FIGs 8a,b, measurements about those of similar parts in FIGs. 6a,b. Embodiments are contemplated wherein sizes of about one-fifth to about five times these dimensions may have possible uses. It is also contemplated in some veterinary embodiments; tip sizes of about one-tenth to 20 times the aforementioned dimensions may also have possible uses.

FIGs. 9a,b depict an embodiment that differs from the embodiment depicted in FIGs. 7a,b in that it comprises only axial protrusions **904** and accompanying lysing elements **905.** Each of the other elements depicted in FIGs. 9a,b may be identical to the corresponding elements shown in FIGs. 7a,b and are referenced by like numerals (numbers higher by 200). For example, FIGs. 9a,b: Modular TD comprises a Modular TD tip **901,** a Modular TD shaft **902,** a Modular TD plug **903** & {spot coagulator boom (SCB) **970** (further comprises: SCB tip **971,** SCB shaft **972** & SCB base **973**)}. In the depicted embodiment, SCB Base **973** permits SCB shaft **972** to pivot about base. In some embodiments, SCB shaft **972** may pivot 360 degrees and in other embodiments, it may be limited to a specific arc, for example some embodiments may be configured to rotate about 180 degrees. Some embodiments may be configured to automatically couple an electric connection at a particular position, for example, some embodiments may be configured such that the spot coagulation boom (SCB) **972** is not electrically connected when the spot coagulator extends backwards toward the modular TD plug **903** but upon reaching a threshold amount of rotation the SCB **970** automatically connects with an electrical source. Alternatively, rotation of the SCB shaft **972** may be independent of the electrical coupling, in such embodiments, a switch may be provided if desired to couple and decouple electrosurgical energy to SCB tip **971.** It is contemplated in some embodiments that a snap or hook may be deployed on the modular TD shaft **902** and/or electrosurgical 'pencil' that may not only insulate, but provide storage for SCB tip **971** but may clean tip on docking, and may also prevent from inadvertently deploying or coming loose during dissection in close quarters (areas of limited surgical workspace, for example between organs or in a 'pocket' of tissues) or more dense tissues. To limit or prevent any rotation of TD plug **903** within a shaft that is a source of electrosurgical energy, the section of SC shaft **902** may comprise SC shaft insert **902.2** that may take any geometric shape and be inserted into the source of electrosurgical energy mating with a complementary shape therein.

In the depicted embodiment, SCB shaft **972** is angled with respect to the modular TD. Some embodiments may be configured such that SCB shaft **972** is rotatable to discrete number of positions about the base **973** or otherwise with respect to modular TD shaft **902.** In alternative embodiments SCB base **973** may be configured to pivot with SCB shaft **972** about modular TD shaft **902.** In other contemplated embodiments, SCB **970** may be coupled with other portions of the modular TD such as modular TD tip **901** for example.

In the depicted embodiment of FIGs. 9a,b, measurements about those of similar parts in FIGs. 7a,b, Embodiments are contemplated wherein sizes of about one-fifth to about five times these dimensions may have possible uses. It is also contemplated in some veterinary embodiments; tip sizes of about one-tenth to 20 times the aforementioned dimensions may also have possible uses.

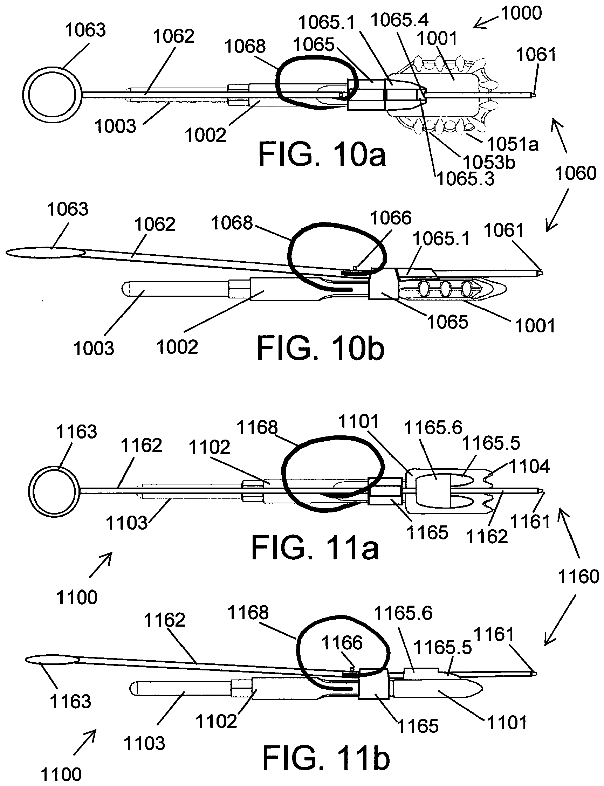

FIG. 10a is an upper plan view of an embodiment of modular TD with a spot coagulator (SC). Modular TD **1000** comprises a Modular TD tip **1001,** a Modular TD shaft **1002,** a Modular TD plug **1003** & spot coagulator **1060.** In the depicted embodiment, Modular TD tip **1001** comprises non-axial protrusions **1051a** and non-axial lysing segment **1053b**. Spot coagulator **1060 c**omprises an SC tip **1061,** SC shaft **1062,** SC handle **1063** & SC connecting conductor cable **1068.** In contemplated embodiments, connecting conductor cable may comprise one or more wires, conductive strips, etc. In contemplated embodiments, modular TD may comprise axial protrusions & lack non-axial or transitional protrusions. In the depicted embodiment, modular TD shaft **1002** may comprise an insulating and/or supportive coating and/or cover overlying a conductive material and/or metal axial core. In the depicted embodiment SC shaft **1062** is slidably coupled to the modular TD shaft **1002** of the modular TD by SC coupler **1065.** SC coupler **1065** is one example of a means for moveably coupling a SC with a surgical tool. SC coupler **1065** may be configured to allow SC shaft **1062** to slide relative to SC coupler **1065** and modular TD shaft **1002.** Alternatively SC coupler **1065** may be rigidly affixed to SC shaft **1062** in which case SC coupler **1065** may be configured to slide relative to TD shaft **1002.** In some embodiments, SC coupler **1065** may comprise a sterilizable plastic and/or polymer and/or ceramic and/or polytetrafluoroethylene and/or other non-conductive and/or insulating material. In some embodiments, SC coupler **1065** may comprise an integral part of modular TD shaft **1002.** In other embodiments SC coupler **1065** may comprise a separate component that may be coupled with modular TD shaft **1002** by way of for example form fitting, snap fitting, an adhesive, welding, and the like.

One or more passageways in SC coupler **1065** may allow the SC shaft **1062** to pass therethrough. In some contemplated embodiments, SC coupler **1065** may be formed from and/or be an integral part of modular TD shaft **1002;** in some of these embodiments, SC coupler **1065** may comprise the same insulating materials of modular TD shaft **1002.** In other contemplated embodiments, SC coupler **1065** may be formed from and/or be an integral part of SC shaft **1062;** in some of these embodiments, SC coupler **1065** may comprise the same insulating materials of SC shaft **1062.** The more distal end of the SC is the SC tip **1061** and the more proximal (toward the surgeon) end of the SC is SC handle **1063.** The term handle does not necessarily specify it is for the entire hand of the surgeon as just one finger is possible to control the SC in some embodiments. In the embodiment depicted in FIGS. 10a,b, the SC handle **1063** comprises a ring that may be operated by a single finger of a surgeon. In this depicted embodiment the SC tip **1061 e**xtends from the SC shaft **1062** and is conductive and not insulated along at least a portion of the tip. In some embodiments, the entire tip may be conductive.

FIG 10b is a side view of a modular TD **1000** & SC **1060** comprising connecting conductor cable **1068.** In some embodiments, the modular TD plug **1003** may comprise a continuation of the metal center of modular TD shaft **1002;** in some embodiments, the diameter may be noncircular and/or irregular on a macroscopic and/or microscopic scale to prevent unwanted rotation. In the depicted embodiment limiting ridge **1066** may be present on or about SC shaft **1063** to prevent further movement of the SC tip **1061** a given distance beyond the modular TD tip **1001.** In the depicted embodiment the SC tip is restricted to 10mm protrusion beyond the distalmost portion of modular TD tip **1001**, which may comprise the end of one of the protrusions on modular TD tip **1001.** In the depicted embodiment, connecting conductor cable **1068** may also restrict movement of SC tip **1061.** In other contemplated embodiments, a bend in the SC shaft or SC coupler passageway and/or size mismatch and/or tether, etc., may also be used to limit the distance SC tip **1061** may protrude beyond the distal end of modular TD tip **1001.** In other contemplated embodiments, no elements may restrict the working movement range of the SC shaft **1062.**

FIG 10b also depicts the attachment of SC connecting conductor cable **1068** to SC shaft **1062** and modular TD shaft **1003** and may conduct electrosurgical energy therebetween. In the depicted embodiment, SC connecting conductor cable **1068** may comprise one or more non-insulated area(s) (usually on the ends) that may be brought into electrical contact with SC shaft **1062** and TD shaft **1003.** In some embodiments, SC connecting conductor cable **1068** is rigidly affixed at the contact points by such methods as welding, soldering, gluing, tying, wrapping, twisting and by any other means known in the art. In the depicted embodiment, insulation covers the contact points; electrical insulation may prevent ionic body fluids from transmitting electrosurgical energy to an undesirable location on the patient. In the depicted embodiment, the insulation is polytetrafluoroethylene as it may have a favorable permittivity. In some embodiments, the electrical insulation may comprise, for example, various halogenated carbon molecules, polyether etherketone and/or polysulfone and/or another electrically nonconductive polymers (with thermal stability in the operating range) and/or materials that are both electrically non-conductive and of low thermal conductivity. In contemplated embodiments, the electrical insulator may comprise, carbon, graphite, and graphite-fiberglass composites and the like. SC connecting conductor cable **1068** may comprise a metal and/or other conductive material such as metal-coated/impregnated plastic and/or polymer and may have a variety of shapes.

Some embodiments may further comprise a SC sheath **1065.1** wherein at least a portion of SC may be positioned in the storage position. In some embodiments, SC sheath may extend from modular TD tip **1001.** In other embodiments SC sheath **1065.1** may extend from SC coupler **1065.** SC sheath may comprise an insulating material and may be configured to completely cover SC tip **1061** in the storage position. SC sheath **1065.1** may, in some embodiments, comprise a transparent material to allow a surgeon to visualize the position of SC tip **1061** within the sheath. Some embodiments may be further configured to clean SC tip **1061** upon withdrawal into SC sheath **1065.1** and/or upon protrusion through SC sheath **1065.1.** For example, in the depicted embodiment, SC sheath **1065.1** may also comprise an SC gate **1065.3,** which may be hinged from a polymer to allow SC opening **1065.4** to move which may help remove debris from SC tip **1061** on passage into and/or out of SC opening **1065.4.** In some embodiments, SC opening **1065.4** may be formed within SC gate **1065.3** and may be configured such that upon advancing and/or retracting SC tip **1061** SC gate **1065.3** pivots slightly to provide an interface with SC tip **1061** that scrapes or otherwise removes debris from SC tip **1061** upon advancing and/or retracting SC tip **1061** thru SC opening **1065.4.**

In some embodiments, one or more passageways may be formed in one or more locations on a tip or other area of a TDM to allow for an SC to be repositioned by a surgeon at a plurality of preconfigured positions.

In the depicted embodiment, the SC tip **1061** extends from the SC shaft **1062** and is conductive and not insulated along at least a portion of the tip. In some embodiments, the entire tip is conductive. In the depicted embodiment SC shaft **1062** may comprise stainless steel and may be round in cross-section. In the depicted embodiment, SC shaft **1063** may comprise an electrically insulating and/or supportive coating and/or cover overlying a conductive material and/or metal axial core. In some embodiments, the conductive material may comprise: steel, nickel, alloys, palladium, gold, tungsten, silver, copper, platinum and/or any other conductive metal that does not give off toxic residua at operating temperatures. In other contemplated embodiments, the conductive material may comprise cermets and the like. In some embodiments, the electrical insulator may comprise, for example, porcelain, ceramics, glass-ceramics, plastics, various halogenated carbon molecules, polytetrafluoroethylene, carbon, graphite, and graphite-fiberglass composites and the like. In alternative embodiments the electrical insulator may comprise polyether etherketone and/or polysulfone and/or another electrically nonconductive polymers (with thermal stability in the operating range) and/or materials that are both electrically non-conductive and of low thermal conductivity. In the depicted embodiment, SC tip **1061** is shaped like the frustum of a cone. In some embodiments, SC shaft **1062** may be oval, flat, rectangular or geometric in cross-section or substantially flattened. In alternative embodiments, SC tip **1061** may be pointed, bullet shaped, or geometric in cross section; more angulate and/or pointed tips may disperse electrical energy more readily and allow greater precision than larger, more rounded tip designs. In the depicted embodiments of the SC shaft **1062,** the electrical insulator may comprise polytetrafluoroethylene. In alternative embodiments the electrical insulator may comprise polyether etherketone and/or polysulfone and/or another electrically nonconductive polymers (with thermal stability in the operating range) and/or materials that are both electrically non-conductive and of low thermal conductivity. In contemplated embodiments the electrical insulator may comprise, for example, porcelain, ceramics, glass-ceramics, plastics, various halogenated carbon molecules, polytetrafluoroethylene, carbon, graphite, and graphite-fiberglass composites.

Although the depicted embodiment shows a manually deployed SC, other contemplated embodiments may allow deployment to be (including but not limited to): motorized and/or spring activated and/or screw driven and/or ratchet style and/or cog style and/or pneumatic and/or hydraulic, etc. Although, the embodiment depicted in Fig. 10b shows the SC shaft **1062** pointing about axially (relative to the modular TD axis) and/or passing about over the middle of the front of the modular TD tip **1001,** in other contemplated embodiments, SC shaft **1062** may point at an angle within about 30degrees of the axis of the modular TD and/or may pass over any other points on the modular TD tip **1001** besides the middle front of the modular TD tip. In other contemplated embodiments, SC shaft **1062** may point at angles greater than 30degrees of the axis of the modular TD. In some embodiments, SC shaft **1062** may be flexible and its forward axis redirected upon passing through SC coupler **1065;** in some embodiments SC coupler may pivot and/or rotate and/or have one or more non-axial passageways for SC shaft **1062** that may redirect the axis and/or pointing of the forward part of SC shaft **1062.** For example, some embodiments may comprise a plurality of passageways for SC shaft **1062** each pointing in a different direction. In such embodiments one or more such passageways may be axial and one or more may be non-axial. In the depicted embodiment of FIGs 10a,b, measurements about those of similar parts in FIGs. 6a,b, Embodiments are contemplated wherein sizes of about one-fifth to about five times these dimensions may have possible uses. It is also contemplated in some veterinary embodiments; tip sizes of about one-tenth to 20 times the aforementioned dimensions may also have possible uses.

FIGs. 11a,b depict an embodiment that differs from the embodiment depicted in FIGs. 10a,b in that it comprises only axial protrusions **1104** and accompanying lysing elements & also differs in that SC sheath has been replaced by SC shield **1165.5** (SC shield may also further comprise SC shield cover **1165.6.** Each of the other elements depicted in FIGs. 11a,b may be identical to the corresponding elements shown in FIGs. 10a,b and are referenced by like numerals (numbers higher by 100). For example, in FIGs. 11a,b: Modular TD comprises a Modular TD tip **1101,** a Modular TD shaft **1102,** a Modular TD plug **1103** & {spot coagulator (SC) **1160** (further comprises: SC tip **1161,** SC shaft **1162** & SC handle **1163)}.** SC coupler **1165** is one example of a means for moveably coupling a SC with a surgical tool. SC coupler **1165** may be configured to allow SC shaft **1162** to slide relative to SC coupler **1165** and modular TD shaft **1102.** In the depicted embodiment, SC coupler **1165** is rigidly affixed to SC shaft **1162.** In contemplated embodiments, SC coupler **1165** may be configured to slide relative to TD shaft **1102.** In some embodiments, SC coupler **1165** may comprise an integral part of modular TD shaft **1102.** In other embodiments SC coupler **1165** may comprise a separate component that may be coupled with modular TD shaft **1102** by way of for example form fitting, snap fitting, an adhesive, welding, and the like.

FIG. 11b is a side view of a modular TD **1100** & SC **1160** comprising connecting conductor cable **1168.** In some embodiments, the modular TD plug **1103** may comprise a continuation of the metal center of modular TD shaft **1102.** In the depicted embodiment, SC comprises SC limiting ridge **1166,** SC connecting conductor cable **1168,** and SC shield **1165.5** (wherein at least a portion of SC may be positioned in the storage position). In the depicted embodiment, SC shield **1165.5** comprises two non-conductive polymeric ridges affixed to TD tip **1101** by adhesive; SC shield **1165.5** further comprises SC shield cover **1165.6.** In some embodiments, affixed SC shields **1165.5** may comprise electrical insulators including but not limited to polymeric insulators. In some embodiments, SC shields may be a direct extension and thus composition of a modular TD tip **1101.** SC shield cover **1165.6** may, in some embodiments, comprise a transparent material to allow a surgeon to visualize the position of SC tip **1161** within the sheath.

In the depicted embodiment of FIGs 11a,b, measurements about those of similar parts in FIGs. 10a,b, Embodiments are contemplated wherein sizes of about one-fifth to about five times these dimensions may have possible uses. It is also contemplated in some veterinary embodiments; tip sizes of about one-tenth to 20 times the aforementioned dimensions may also have possible uses.

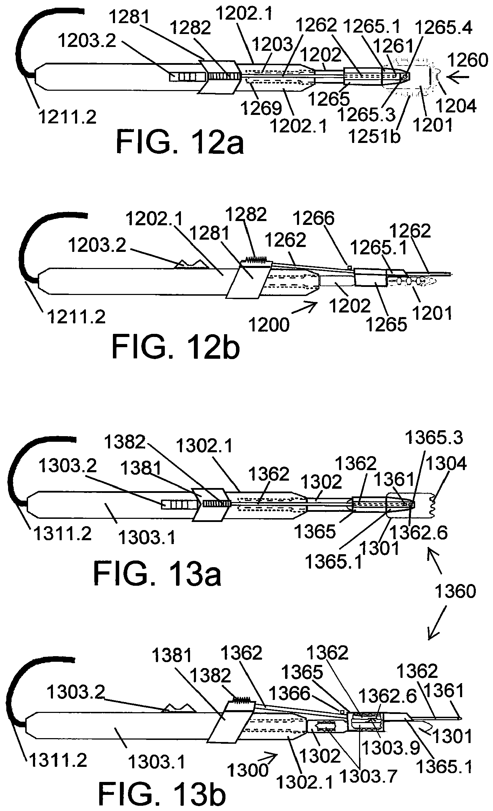

FIGS. 12a,b depict an embodiment comprising an axially advanceable SC **1260.** SC comprises SC shaft **1262,** SC tip **1261,** SC toggle **1281** and/or SC sheath **1265.1.** SC shaft may be coupled with modular TD by way of coupler **1265.** In this embodiment, toggle **1281** is configured both to advance the SC and to connect the SC with an electrical source at a particular point in its advancement. In contemplated embodiments, separate components may be used to advance the SC and to couple the SC with an electrical source. In the depicted embodiment, SC toggle comprises a sleeve. The modular TD **1200** depicted in this embodiment comprises a Modular TD tip **1201,** Modular TD shaft **1202,** & Modular TD plug **1203'.**

In some embodiments, the spot coagulator toggle may be configured to move at least a portion of the spot coagulator with respect to a portion of a surgical device, such as a tip comprising protrusions and lysing segments, between a plurality of operational positions and a storage position, or between at least one operational position and at least one storage position.

In some embodiments, a first conductive element and a second conductive element may be provided. The first conductive element may be configured to electrically couple to the second conductive element in an operational configuration to allow the spot coagulator tip to deliver energy for coagulating a blood vessel. The first conductive element may be electrically insulated from the second conductive element in a storage configuration to prevent the spot coagulator tip from delivering energy.

In some embodiments, the spot coagulator may be configured to provide for a plurality of operational positions such that at least one conductive element of the spot coagulator couples with at least one other conductive element such that the spot coagulator can deliver energy for coagulating a blood vessel at any of a plurality of operational positions.

In some embodiments, a plurality of conductive elements may be provided and each of the conductive elements may correspond to one of the plurality of operational positions. For example, at least one of a first and a second conductive element may comprise an insulator and a plurality of conducting sections. Each of the conducting sections may comprise an opening in the insulator, such as a bare portion to expose the underlying conductor, to allow the conducting sections to make contact with another conductive element. Each of the conducting sections may correspond to one of the plurality of operational positions such that electrical energy may be deliver to a spot coagulator tip at each, and only at each, of the operational positions.

In some embodiments, the spot coagulator tip may be axially movable with respect to a shaft of a TDM such that the spot coagulator can be advanced to one or more operational configurations and retracted to one or more storage configurations.

The modular TD **1200** depicted in this embodiment has non-axial protrusions. The depicted embodiment also comprises axial protrusions, such as **1204**, and non-axial protrusions such as **1251b** including transitional protrusions. Transitional protrusions, axial protrusions, non-axial protrusions, axial lysing elements, non-axial lysing elements and lysing elements of differing geometries are discussed in more detail elsewhere in this application. Modular TD **1200** is modular in that it is removable from an electrosurgical 'pencil' shaft **1202.1** and/or an electrosurgical 'pencil' handle. Rocker switch **1203.2** may control the electrosurgical energy such as a suitable waveform derived from an electrosurgical generator (not seen in this view) brought into the handle via conduit **1211.2.** In the depicted embodiment modular TDM tip plug **1203** is configured to be received within a corresponding electrosurgical 'pencil' shaft recess **1269** formed within electrosurgical 'pencil' shaft **1202.1.** In some embodiments, elements within modular TDM tip plug **1203** and/or electrosurgical 'pencil' shaft recess **1269** may be electrically connected with electrical elements within 'pencil' shaft **1202.1** and/or 'pencil' handle which are in turn connected with 'pencil' switch **1203.2** and/or conduit **1211.2.**

So as not to have the surgeon set down the TD or TDM, to pick up other instrumentation from the surgical tray to stem bleeding blood vessel(s), it may be beneficial during some surgical procedures to have spot coagulator coagulation capabilities within the same instrument.

In the depicted embodiment, modular TD shaft **1202** may comprise an electrically insulating and/or supportive coating and/or cover overlying a conductive material and/or metal axial core. In some embodiments, the conductive material may comprise: steel, nickel, alloys, palladium, gold, tungsten, silver, copper, platinum and/or any other conductive metal that does not give off toxic residua at operating temperatures. In other contemplated embodiments, the conductive material may comprise cermets and the like. In some embodiments, the electrical insulator may comprise, for example, porcelain, ceramics, glass-ceramics, plastics, various halogenated carbon molecules, polytetrafluoroethylene, carbon, graphite, and graphite-fiberglass composites and the like. In alternative embodiments the electrical insulator may comprise polyether etherketone and/or polysulfone and/or another electrically nonconductive polymers (with thermal stability in the operating range) and/or materials that are both electrically non-conductive and of low thermal conductivity. SC **tip** 1261 shapes may comprise those discussed in other tip embodiments in this application. In the depicted embodiment SC shaft **1262** is slidably coupled to the modular TD shaft **1202** of the modular TD by SC coupler **1265.** SC coupler **1265** is one example of a means for moveably coupling a SC with a surgical tool. SC coupler **1265** may be configured to allow SC shaft **1262** to slide relative to SC coupler **1265** and modular TD shaft **1202.** In the depicted embodiment, SC coupler **1265** is rigidly affixed to TD shaft **1202.** In contemplated embodiments, SC coupler **1265** may be configured to slide relative to TD shaft **1202.** In such embodiments, SC coupler **1265** may be rigidly affixed to SC shaft **1262.** In some embodiments, SC coupler **1265** may comprise a sterilizable plastic and/or polymer and/or ceramic and/or polytetrafluoroethylene and/or other non-conductive and/or insulating material. In some embodiments, SC coupler **1265** may comprise an integral part of modular TD shaft **1202.** In contemplated embodiments, SC coupler **1265** may comprise an integral part of modular TD tip **1201.** In other embodiments SC coupler **1265** may comprise a separate component that may be coupled with modular TD tip **1201** by way of for example form fitting, snap fitting, an adhesive, welding, and the like. One or more passageways in SC coupler **1265** and/or SC sheath **1265.1** may allow the SC shaft **1262** to pass therethrough. In some contemplated embodiments, SC coupler **1265** may be formed from and/or be an integral part of modular TD shaft **1202;** in some of these embodiments, SC coupler **1265** may comprise the same insulating materials of modular TD shaft **1202.** In other contemplated embodiments, SC coupler **1265** may be formed from and/or be an integral part of SC shaft **1262;** in some of these embodiments, SC coupler **1265** may comprise the same insulating materials of SC shaft **1262.** Some contemplated embodiments may be lacking in SC sheath **1265.1.**

Coupler **1265** may be configured to allow SC shaft **1262** to be advanced or retracted axially therethrough. The SC depicted in these figures may be coupled with electrosurgical 'pencil' shaft **1202.1** by way of modular TD plug **1203.** SC toggle 1281 may be slidably coupled along 'pencil' shaft **1202.1** such that SC tip **1261** may be advanced axially to an operational position and retracted axially to a storage position. SC tip **1261** may retract into SC sheath **1265.1** via SC opening **1265.4.** In the depicted embodiment, SC sheath may also comprise SC gate **1265.3** which may be hinged from a polymer to allow SC opening **1265.4** to move which may help remove debris from SC tip **1261** on passage into and/or out of SC opening **1265.4.** In some embodiments, SC opening **1265.4** may be formed within SC gate **1265.3** and may be configured such that upon advancing and/or retracting SC tip **1261** SC gate **1265.3** pivots slightly to provide an interface with SC tip **1261** that scrapes or otherwise removes debris from SC tip **1261** upon advancing and/or retracting SC tip **1261** thru SC opening **1265.4.** Passage of SC shaft through SC opening may be facilitated by such insulations as polytetrafluoroethylene. In some embodiments, the edges of SC opening **1265.4** may be serrated and/or irregularly edged and/or possess bristles and/or other texture that may facilitate dislodgement of electrocoagulation char and/or carbon to clean SC tip **1261.** Some contemplated embodiments lack SC gate **1265.3** and have only opening **1265.4.** SC shaft **1262** may be limited in excursion by limiting ridge **1266.** In the depicted embodiment limiting ridge **1266** may comprise a ridge of SC shaft **1262** insulation that may not fit through SC coupler passageway. SC toggle **1281** may comprise grip **1282** to facilitate advancement and retraction of SC **1260.** Some embodiments may further comprise a SC sheath **1265.1** wherein at least a portion of SC may be positioned in the storage position. In some embodiments SC sheath may extend from modular TD tip **1201.** In other embodiments SC sheath **1265.1** may extend from SC coupler **1265.** SC sheath may comprise an insulating material and may be configured to completely cover SC tip **1261** in the storage position. SC sheath **1265.1** may, in some embodiments, comprise a transparent material to allow a surgeon to visualize the position of SC tip **1261** within the sheath. Some embodiments may be further configured to clean SC tip **1261** upon withdrawal into SC sheath **1265.1** and/or upon protrusion through SC sheath **1265.1.** In some contemplated embodiments SC sheath **1265.1** may be absent. SC toggle **1281** may, in some embodiments, comprise a transparent material to allow a surgeon to visualize the position of the front of the electrosurgical 'pencil' within the SC toggle. SC toggle **1281** may comprise, in cross section, a full or complete ring. Other embodiments may comprise, in cross section, a split ring. Such rings may be configured to accommodate 'pencil' shafts of varying cross sectional geometries and/or diameters and/or lengths. In the depicted embodiment, SC toggle **1281** is attached to the screw-threaded proximal end of conductive metal core of SC shaft **1262;** insulation may be removed in the threaded area of the SC shaft to improve the fit. In other contemplated embodiments, SC toggle **1281** may be attached to SC shaft **1262** by way of for example form fitting, snap fitting, an adhesive, welding, and the like. In some contemplated embodiments, SC toggle **1281** may be a continuation and/or comprise the insulation of SC shaft **1262.** SC **1260** may be electrically coupled with modular TD **1200** by an SC connecting contact and/or an SC shaft conductive opening (for example, as shown in FIG. 13 at **1303.9** & **1362.6**). The SC connecting contact may be configured to accommodate axial movement of SC shaft **1262** between the operational and storage positions. For example, some embodiments may comprise a plurality of adjacent conductive rings or other non-insulated areas such that SC tip **1261** may be positioned at a plurality of preset distances from modular TD tip. In some contemplated embodiments, toggle **1281** may be a part of modular TD shaft **1202** (and does not move relative to it) instead SC shaft **1262** may move through toggle **1281** and may be controlled by a thumb switch.

In the depicted embodiment, the SC tip **1261** extends from the SC shaft **1262** and is conductive and not insulated along at least a portion of the tip. In some embodiments, the entire tip is conductive. In the depicted embodiment SC shaft **1262** may comprise stainless steel and may be round in cross-section. In the depicted embodiment, SC shaft **1262** may comprise an electrically insulating and/or supportive coating and/or cover overlying a conductive material and/or metal axial core. In some embodiments, the conductive material may comprise: steel, nickel, alloys, palladium, gold, tungsten, silver, copper, platinum and/or any other conductive metal that does not give off toxic residua at operating temperatures. In other contemplated embodiments, the conductive material may comprise cermets and the like. In some embodiments, the electrical insulator may comprise, for example, porcelain, ceramics, glass-ceramics, plastics, various halogenated carbon molecules, polytetrafluoroethylene, carbon, graphite, and graphite-fiberglass composites and the like. In alternative embodiments the electrical insulator may comprise polyether etherketone and/or polysulfone and/or another electrically nonconductive polymers (with thermal stability in the operating range) and/or materials that are both electrically non-conductive and of low thermal conductivity. In the depicted embodiment, SC tip **1261** is shaped like the frustum of a cone. In some embodiments, SC shaft **1262** may be oval, flat, rectangular or geometric in cross-section or substantially flattened. In alternative embodiments, SC tip **1261** may be pointed, bullet shaped, or geometric in cross section; more angulate and/or pointed tips may disperse electrical energy more readily and allow greater precision than larger, more rounded tip designs.

Although the depicted embodiment shows a manually deployed SC, other contemplated embodiments may allow deployment to be (including but not limited to): motorized and/or spring activated and/or screw driven and/or ratchet style and/or cog style and/or pneumatic and/or hydraulic, etc. Although, the embodiment depicted in Fig. 12a shows the SC shaft **1262** pointing about axially (relative to the modular TD axis) and/or passing about over the middle of the front of the modular TD tip **1201,** in other contemplated embodiments, SC shaft **1262** may point at an angle within about 30 degrees of the axis of the modular TD and/or may pass over any other points on the modular TD tip **1201** besides the middle front of the modular TD tip. In other contemplated embodiments, SC shaft **1262** may point at angles greater than 30 degrees of the axis of the modular TD. In some embodiments, SC shaft **1262** may be flexible and its forward axis redirected upon passing through SC coupler **1265;** in some embodiments SC coupler may pivot and/or rotate and/or have one or more non-axial passageways for SC shaft **1262** that may redirect the axis and/or pointing of the forward part of SC shaft **1262.** For example, some embodiments may comprise a plurality of SC coupler and/or SC sheath passageways for SC shaft **1262** each pointing in a different direction. In such embodiments one or more such passageways may be axial and one or more may be non-axial. In other contemplated embodiments, SC coupler **1265** and/or SC sheath **1265.1** may have one or more pathways that may lead to SC shaft exiting at a similar range of angles from the axis of the modular TD.

In other contemplated embodiments, a variety of geometrically shaped lysing elements and/or protrusions may co-exist side by side. For example, in some embodiments, two adjacent protrusions may differ in shape and/or size, 2 adjacent lysing elements may differ in length and/or shape and/or TD tip **1201** may otherwise be nonsymmetrical in one or more views.

In the depicted embodiment, the SC tip **1261** and SC shaft **1262** together may measure about, 55mm in length and about 3mm in maximal thickness; the SC toggle **1281** may measure about 15mm in diameter and about 1mm in maximal thickness; the overall SC **1260** may measure about 75mm in length. In embodiments wherein an electrical insulation and/or polymeric insulating coating may be present on such parts, for example on SC shaft **1262 s**uch insulation may measure about 0.5mm in thickness. In some embodiments, the insulation thickness may range from about 0.1mm to 3mm. Embodiments are contemplated wherein sizes of about one-fifth to about five times these dimensions may have possible uses. It is also contemplated in some veterinary embodiments; tip sizes of about one-tenth to 20 times the aforementioned dimensions may also have possible uses.

An implementation using the depicted embodiment may involve the surgeon pushing distally SC toggle **1281** which forces SC shaft **1262** distally through SC coupler **1265** whereupon SC shaft conductive opening is brought into direct contact with SC connecting contact which itself is in direct or indirect contact with the conductive portion of modular TD shaft. Thus electrosurgical energy such as a suitable waveform will be delivered, when the electrosurgical generator is activated, via modular TD plug **1203,** into modular TD shaft **1202,** into SC shaft **1262** and thereupon to SC tip **1261** and then into target tissue.

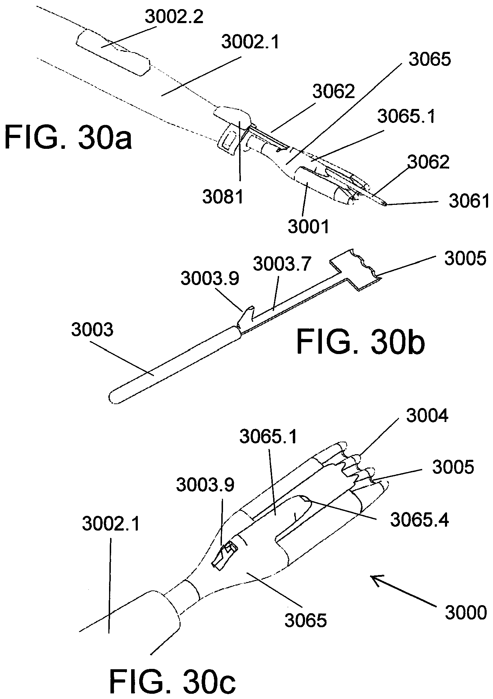

The embodiment depicted in FIGS. 12a and 12b may, in some embodiments, further comprise a protruding electrically-conductive element positioned on an electro-conductive portion of Modular TD shaft **1202** that is configured to electrically couple with a corresponding conductive element positioned on SC shaft **1262.** For example, some embodiments may comprise a protruding fin, as will be discussed and shown in conjunction with FIG. 30.

FIGs. 13a,b depict an embodiment that differs from the embodiment depicted in FIGs. 12a,b in that it comprises only axial protrusions **1304** and accompanying lysing elements. In addition, some of the elements in FIGs. 13b are shown in greater detail in connection with these figures than the corresponding elements in FIGs. 12a,b. Such elements are as follows: FIGs. 13b also depicts a breakaway showing elements that are present but not seen in the embodiment depicted in FIGs. 12a,b, including SC shaft conductive opening **1362.6** in SC shaft **1362,** modular TD shaft conductive core **1303.7,** and SC coupler conductive element **1303.9.** SC coupler conductive element **1303.9** comprises a protruding electrically-conductive element. More particularly, SC coupler conductive element **1303.9** comprises a spring, such as a leaf spring, that may be used to facilitate desired contact with a portion of the SC, such as SC shaft **1362.**

Each of the other elements depicted in FIGs. 13a,b may be identical to the corresponding elements shown in FIGs. 12a,b and are referenced by like numerals (numbers higher by 100). For example, FIGs. 13a,b depict an embodiment comprising an axially advanceable SC **1360.** SC comprises SC shaft **1362,** SC tip **1361,** SC toggle **1381,** SC coupler **1365** and/or SC sheath **1365.1.** The modular TD **1300** depicted in this embodiment comprises a Modular TD tip **1301,** Modular TD shaft **1302,** Modular TD plug **1303** (in phantom) and also comprises axial protrusions **1304** but lacks non-axial protrusions and lysing segments. The standard electrosurgical 'pencil' depicted, comprises shaft **1302.1,** 'pencil' switch **1303.2,** 'pencil' handle **1303.1** and/or conduit **1311.2.** SC tip **1361** may retract into SC sheath **1365.1** via SC opening **1365.4.** In some embodiments, SC opening **1365.4** may be formed within SC gate **1365.3.** SC shaft **1362** may be limited in excursion by limiting ridge **1366.** SC toggle **1381** may comprise grip **1382.** Although, the embodiment depicted in Fig. 13a shows the SC shaft **1362** pointing about axially (relative to the modular TD axis) and/or passing about over the middle of the front of the modular TD tip **1301,** in other contemplated embodiments, SC shaft **1362** may point at an angle within about 30 degrees of the axis of the modular TD and/or may pass over any other points on the modular TD tip **1301** besides the middle front of the modular TD tip. In other contemplated embodiments, SC shaft **1362** may point at angles greater than 30 degrees of the axis of the modular TD.

As shown in FIG. 13b breakaway, SC **1360** is electrically coupled with modular TD **1300** by SC connecting contact **1303.9** & SC shaft conductive opening **1362.6.** SC connecting contact **1303.9** may be configured to accommodate axial movement of SC shaft **1362** between the operational and storage positions. For example, some embodiments may comprise a plurality of adjacent conductive rings or other non-insulated areas such that SC tip **1361** may be positioned at a plurality of preset distances from modular TD tip. In the depicted embodiment, SC connecting contact **1303.9** is electrically coupled to the modular TD shaft **1362** conductive core **1303.7.** In contemplated embodiments, SC connecting contact **1303.9** may be electrically coupled to the modular TD tip **1301** and/or modular TD plug **1302.** In contemplated embodiments, the SC connecting contact **1303.9** may comprise a conductive piece emanating and/or projecting from the conductive core of modular TD shaft **1302,** for example a 'fin'; such pieces may be manufactured by stamping and/or welding or other method known in the art. An example of a protruding electrically-conductive element comprising a fin is shown and discussed in conjunction with FIG. 30 below. In some embodiments, the electrically-conductive element may be flexible, such as spring-loaded or spring-biased, to further facilitate desired electrical contact.

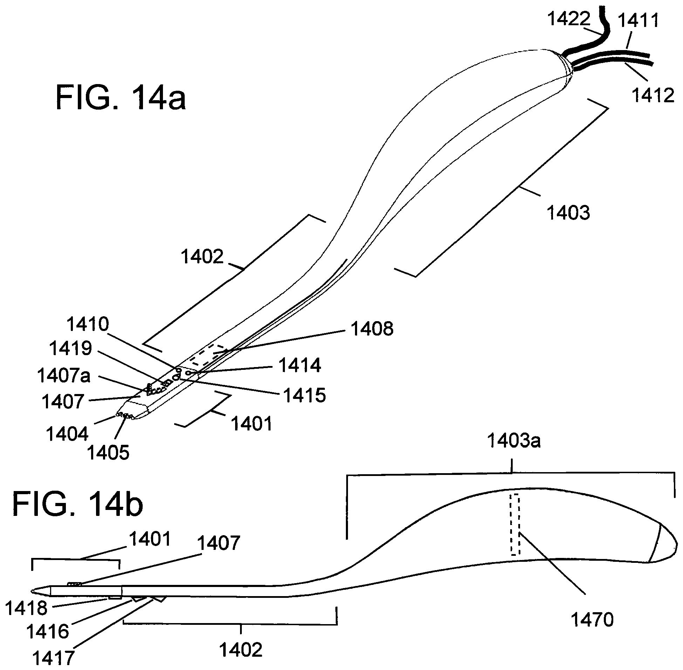

FIG. 14a is a perspective view of an embodiment of a TDM comprising a tip **1401,** a shaft **1402** and a handle **1403.** In the depicted embodiment, electrosurgical energy such as for example electro-coagulation and/or electro-cutting waveforms arrive in electrical conduits **1411** and/or **1412** and may travel by wiring through the handle and shaft to termini **1407a,** which are part of energy window **1407.** In the depicted embodiment, electrosurgically energized energy window **1407** may be present on the upper side of the device. Electro-cutting and electro-coagulation currents may be controlled outside the TDM via an electrosurgical generator, for example the Bovie Aaron 1250™ or Bovie Icon GP™. It should be noted that the term "energy window" is intended to encompass what is referred to as a planar-tissue-altering-window/zone in U.S. Patent No. 7,494,488 and, as described later, need not be electrosurgically energized in all embodiments. In some embodiments, the "energy window" may comprise a variety of other energy emitting devices, including radiofrequency, electromagnetic, thermochromic, intense pulsed light, LASER, thermal, microwave and/or ultrasonic. It should also be understood that the term "energy window" does not necessarily imply that energy is delivered uniformly throughout the region comprising the energy window. Instead, some energy window implementations may comprise a series of termini or other regions within which energy is delivered with interspersed regions within which no energy, or less energy, is delivered. This configuration may be useful for some implementations to allow for alteration of certain tissue areas with interspersed areas within which tissue is not altered, or at least is less altered. This may have some advantages for certain applications due to the way in which such tissue heals. A second energy window **1408** may also be included in some embodiments, and may comprise yet another electrical energy emitting area. In some embodiments, the "second energy window" may comprise a variety of other energy emitting devices, including radiofrequency, thermochromic, intense pulsed light, LASER, thermal, microwave and ultrasonic. It is contemplated that in alternative embodiments, either one or both of the energy windows may be omitted.

In the depicted embodiment, the tip **1401** may measure about 1cm in width and about 3mm in thickness. It is contemplated that in alternative embodiments, sizes of about one-fifth to about five times these dimensions may also have possible uses. In some contemplated veterinary embodiments, tip sizes of about one-tenth to 20 times the aforementioned dimensions may also have possible uses. In some embodiments, the tip can be a separate piece that is secured to shaft by a variety of methods such as a snap mechanism, mating grooves, plastic sonic welding, etc. Alternatively, in some other embodiments, the tip can be integral or a continuation of shaft made of similar metal or materials. In some embodiments, the tip may comprise materials that are both electrically non-conductive and of low thermal conductivity; such materials might comprise, for example, ceramics, porcelain, glass-ceramics, plastics, various halogenated carbon molecules, polytetrafluoroethylene, carbon, graphite, graphite-fiberglass composites and the like. In some embodiments, the tip may be constructed of a support matrix of an insulating material (for example, ceramic, glass, alumina, zirconia).

In the depicted embodiment, external conduits **1411** and/or **1412** (which may also contain electrical control wires to aid in device operation) may connect to electrically conductive elements to bring RF electrosurgical energy from an electrosurgical generator down the shaft **1402** to electrically conductive lysing elements **1405** mounted in the recessions in between the protrusions **1404.** In some embodiments, the protrusions may comprise bulbous protrusions. Partially hidden from direct view in FIGs. 14a,b, and located in the grooves defined by protrusions **1404** are electrically conductive tissue lysing elements **1405,** which, when powered by an electrosurgical generator, effects lysing of tissue planes on forward motion of the device. Lysing elements may be located at the termini of conductive elements.

In the depicted embodiment, the tip **1401** may comprise, partially or completely, concentrically laminated or annealed-in wafer layers of materials that may include plastics, silicon, glass, glass/ceramics, cermets or ceramics. Lysing elements **1405** may also comprise, partially or completely, a cermet material. In an alternative embodiment, the tip may comprise insulation covered metals and/or electroconductive materials.

The tip shown in the depicted embodiment has four relative protrusions and three relative recessions & a monopolar tip conductive element. All of the axes of the relative protrusions of the tip depicted in this embodiment extend at least substantially parallel to the axis of the shaft of the TDM (as viewed from Top). In embodiments of tips of such axially oriented protrusions and/or relative recessions, surgeons may use methods of defining and or dissecting a target area by entering through an incision and then moving the TDM tip in a primarily axial direction forward and backward and reorienting the TDM after the backstroke in a spokewheel pattern the TDM to access tissues adjacent to earlier strokes.

In some embodiments, shaft **1402** may range in cross-section for example, oval, flat, rectangular, geometric or substantially flattened. In some embodiments, smoothing of the edges of the shaft may reduce friction on the skin surrounding the entrance wound. In some further embodiments, the shaft may comprise metal, plastic and/or other material. In alternative embodiments, the shaft interior may be completely occupied or hollowed containing wires, electrical conductors, fluid/gas pumping or suctioning conduits, fiber-optics, or insulation. In the depicted embodiment, shaft **1402** may be about 15cm in length and/or handle **1403** may be about 10cm in length. In some embodiments, the shaft may have a length of about 10-20cm. In some embodiments, the handle may have a length of about 8-18cm. In some embodiments, shaft plastics, such as polytetrafluoroethylene may act as insulation about wire or electrically conductive elements. In some embodiments, the shaft may alternatively be made partially or completely of concentrically laminated or annealed-in wafer layers of materials that may include plastics, silicon, glass, glass/ceramics, ceramics carbon, graphite, graphite-fiberglass composites. Depending upon the intended uses for the device, an electrically conductive element internal to shaft may be provided to conduct electrical impulses or RF signals from an external power/control unit (such as a Valleylab™ electrosurgical generator) to another energy window **1408.**

In the depicted embodiment, energy windows **1407** and/or **1408** may comprise substantially planar shapes. In alternative embodiments, energy windows **1407** and/or **1408** may take on other cross-sectional shapes that may correspond with a portion of the shape of the shaft, such as arced, stair-step, or other geometric shapes/curvatures. In the depicted embodiment in FIGs. 14a,b energy window **1407** is adjacent to protrusions **1404.** However, in other contemplated embodiments, an energy window may be positioned elsewhere on the shaft **1402** or tip **1401** of the wand, and still be considered adjacent to protrusions **1404.** For example, in an embodiment lacking energy window **1407,** but still comprising energy window **1408,** energy window **1408** would still be considered adjacent to protrusion **1404.** However, if an energy window was p**lace**d on handle **1403,** such an energy window would not be considered adjacent to the protrusions **1404.** In some embodiments, the electrosurgically energized window waveforms may be further pulsed at varying rates, by interpolating gating circuitry at some point external to the electrosurgical generator by standard mechanisms known in the art that may range from about 1 per second to about 60 per second. In some embodiments, the rate may vary from about 1 per second to about 150 per second. In some embodiments, the electrosurgically energized tip current can be further pulsed at varying rates by gating circuitry within the electrosurgical generator by standard mechanisms known in the art.

In some embodiments, the electrically conductive lysing elements portion of the tip may arise from a plane or plate of varying shapes derived from the aforementioned materials by methods known in the manufacturing art, including but not limited to additive manufacturing, cutting, stamping, pouring, molding, filing and/or sanding. In some embodiments, the electrically conductive lysing elements **1405** may comprise an insert attached to a conductive element in the shaft or continuous with a formed conductive element coursing all or part of the shaft. In some embodiments, lysing elements may be continuous and continue to extend all the way around a tip. In some embodiments lysing elements may be discrete and do not extend all the way around a tip. For example, in some embodiments each of the lysing elements may comprise a separate piece of material. In other embodiments, each of the lysing elements may comprise a portion of a single plate, wire or other piece of material. In some embodiments, one or more electrically conductive elements or wiring in conduit **1411** and/or **1412** brings RF electrosurgical energy down the shaft to electrically conductive lysing elements **1405** associated in part with the recessions. In an embodiment, the electrosurgical energy via conduit **1411** is predominately electro-cutting and/or a blend.

In some embodiments, the electrically conductive element or wiring may be bifurcated to employ hand switching if an optional finger switch is located on handle. The electrically conductive element or wiring leading from the shaft into the handle may be bundled with other electrical conduits or energy delivering cables, wiring and the like and may exit the proximal handle as insulated general wiring to various generators (including electrosurgical), central processing units, lasers and other sources as have been described herein.

In some embodiments, the plate making up lysing elements **1405** may be sharpened or scalloped or made to slightly extend outwardly from the tip recessions into which the plate will fit. Alternatively, in some embodiments, since cutting or electrical current may cause an effect at a distance without direct contact, the lysing elements may be recessed into the relative recessions or grooves defined by the protrusions **1404** or, alternatively, may be flush with protrusions **1404.** In some further adjustable embodiments, locations of the electrically conductive lysing elements with respect to the protrusions may be adjusted by diminutive screws and/or ratchets. In some further adjustable embodiments, locations of the electrically conductive lysing elements with respect to the protrusions may be adjusted by MEMS or microelectronics. In the depicted embodiment, lysing element plate is about 0.5mm in thickness. In other embodiments, the plate thickness may range from 0.1mm to 3mm. Plate bevel sharpness may range to varying degrees on its forward facing surface; however, sharp edges may increase the efficiency with which electricity will pass from an edge & may increase the aggressiveness of electrosurgical cutting of a target tissue.

In some implementations, the electrosurgical current for the electrically conductive lysing elements may comprise monopolar "cutting," "blend" &/or "coag" selections. In some embodiments, the electrosurgical current for the electrically conductive lysing elements may be delivered to the tip lysing conductor in a continuous fashion or, alternatively, a pulsed fashion. Surgeons may control the presence of current by a foot pedal control of the electrosurgical generator and/or by button control on the shaft and/or handle. The amount of electrosurgical current and waveform may be modified by standard interfaces or dials on the electrosurgical generator. For monopolar embodiments, the electrosurgical generator is connected to a grounding or dispersive plate which may be placed elsewhere in contact with the patient's body, such as the thigh. Such circuitry may be controlled and gated/wired from the cutting current delivery system of the electrosurgical generator. Acceptable electrosurgical generators may include Valley Lab Force 1 B™ with maximum P-P voltage of 2400 on "cut" with a rated load of 300 Ohms and a maximum power of 200 Watts, 35 maximum P-P voltage of 5000 on "coagulate" with a rated load of 300 Ohms, and a maximum power of 75 Watts ValleyLab Force 4 has a maximum P-P voltage of 2500 on "cut" with a rated load of 300 Ohms and a maximum power of 300 Watts, 750kHz sinusoidal waveform output, maximum P-P voltage of 9000 on "coagulate" with a rated load of 300 Ohms and a maximum power of 120 Watts using a 750kHz damped sinusoidal with a repetition frequency of 31 kHz.

In the depicted embodiment, tip **1401** may comprise materials that are both electrically non-conductive and of low thermal conductivity such as porcelain, epoxies, ceramics, glass-ceramics, plastics, or varieties of polytetrafluoroethylene. In an alternative embodiment, the tip may comprise multilayer wafer substrates comprised of bonded conductive strips and ceramics. Suitable conductive materials may comprise those already described for tip manufacture. In alternative embodiments, the tip may be made from metals or electroconductive materials that are completely or partially insulated. In the depicted embodiment, positioned in the relative recessions of the tip are planar, crescent shaped (concave), stainless steel, electrically conductive tissue lysing elements **1405** (usually hidden from view at most angles). Note the relative protrusions and relative recessions may not be completely visible from certain viewing angles. In contemplated embodiments, lysing element shapes may be convex, straight and/or virtually any geometric shape. In contemplated embodiments, electrically conductive lysing elements may be in the shape of a plate, plane and/or wire. In contemplated embodiments, electrically conductive lysing elements may comprise conductive materials may comprise but are not limited to steel, nickel, alloys, palladium, gold, tungsten, titanium, silver, copper, and platinum and the like. In contemplated embodiments, electrically conductive lysing elements may comprise a conductive material and/or metal that does not melt under operating conditions or give off toxic residua. In alternative embodiments the geometry of the tip area may comprise protrusions that are not oriented along the axis of the shaft (for example, as seen in FIGs. 3, 4, 5 herein, **351b**, **451b**, **551.1**). Certain metals may become oxidized after repeated use, thus impeding electrical flow and function. Some embodiments may comprise a low cost, disposable, and one-time-use device. However, in some embodiments intended for multiple uses, the tip's electrically conductive tissue lysing elements be protected or coated with materials that include, but are not limited to, Silverglide™ non-stick surgical coating, platinum, palladium, silver, gold and rhodium. Varying the amount of protective coating allows for embodiments of varying potential for obsolescence capable of either prolonging or shortening instrument life. Some embodiments may be configured to be modular and/or comprise disposable tips such that a surgeon can place an appropriate tip for a particular surgery on the shaft. Alternatively or additionally one or more of the tips may be disposable such that a surgeon may dispose of the tip after performing surgery and install a new tip for subsequent surgeries or a continuation of the current surgery with a new tip.

In alternative embodiments, the electrically conductive lysing elements may be bifurcated or divided into even numbers at the relative recessions, insulated and energized by wiring to an even number of electrical conduits in a bipolar fashion and connected to the bipolar outlets of the aforementioned electrosurgical generators. Rings partly or completely encircling the shaft of the hand unit can be linked to a partner bipolar electrode at the tip or on the energy window. Such bipolar versions may decrease the energy necessary to electrically modify certain tissues, especially thicker tissues. In alternative embodiments, the lysing elements may be divided into odd numbers yet still allow for bipolar flow between two or more elements as those of ordinary skill in the art would appreciate.

In some embodiments, one or more suction/vacuum ports **1417** may be provided on or about the tip or distal shaft. The port(s) may be fluidly coupled with a vacuum; the vacuum may comprise a pump or a negative pressure chamber or a syringe at the end of a fluid conduit. Other embodiments may comprise one or more suction/vacuum ports on any other suitable location on the TDM, including but not limited to on the protrusions or otherwise on the tip, and on the shaft. In some embodiments, a fluid delivery port **1416** may be provided. In some embodiments the fluid delivery port may be coupled with a pump or high pressure fluid. In some embodiments the port may be perpetually open such that fluid may be delivered therethrough upon actuation of a pump or fluid pressure system. In other embodiments the port may be closed and selectively opened to deliver fluid therethrough. Other embodiments may comprise one or more fluid ports on any other suitable location on the TDM, including but not limited to on the protrusions or otherwise on the tip, and on the shaft. Fluid ports that may be useful may comprise channels within the TDM, polymer lines, hoses, etc. Fluids that may emanate from the outlet may comprise ionic fluids such as saline, medicines (including but not limited to antibiotics, anesthetics, antineoplastic agents, bacteriostatic agents, etc.), non-ionic fluids, and or gasses (including but not limited to nitrogen, argon, air, etc.). In some embodiments fluids may be under higher pressures or sprayed. It should be understood that although these elements (**1416** & **1417**) are not depicted in every one of the other figures, any of the embodiments described herein may include one or more such elements.

In some embodiments, a vibration means **1470** may be positioned in the handle. Other embodiments may comprise one or more vibration means on any other suitable location on the TDM, including but not limited to on the protrusions or otherwise on the tip, and on the shaft. Examples of suitable vibration means may include piezoelectric materials, ultrasonic motors with stators, piezoelectric actuators, vibration motor such as an off-center weight mounted on a gear, etc. Some vibration means may be configured to emit ultrasound in the 20-40kHz range. Yet other vibration means may include electromagnet drivers with a frequency of operation in the range of 150-400Hz. In some embodiments, one or more vibration means may be used to provide additional forces which may facilitate passage of the TDM. In some embodiments, one or more vibration means may be used to reduce debris on the electrosurgical or other components of the TDM. In a further embodiment, a vibration means may be directly or indirectly connected to one or more of the lysing elements. Some vibration means may help to decrease and/or remove debris. In some embodiments use of a vibration means may, also or alternatively, be used to assist in migrating the TDM through tissue during the procedure. In some such embodiments, it is thought that use of a vibration means having a lower frequency may be particularly useful for assisting in such migration. In addition, positioning the vibration means closer to a handle of the TDM may facilitate such migration as well. By contrast, positioning the vibration means on or near the tip, and/or using a higher frequency vibrations means may be particularly useful for preventing buildup of debris on the tip.

In some embodiments, one or more electromagnetic delivery elements **1415** may be positioned on tip or shaft. Other embodiments may comprise one or more electromagnetic delivery elements on any other suitable location on the TDM, including but not limited to on the protrusions or otherwise on the tip, and on the shaft. Electromagnetic delivery elements that may be useful include: LEDs, LASERs, fiberoptics, filaments, photoelectric materials, infrared emitters, etc.

The sensors **1410** and **1414** may comprise any of the sensors described in the specification herein. Other embodiments may comprise one or more sensors on any other suitable location on the TDM, including but not limited to on the protrusions or otherwise on the tip, and on the shaft. Sensors that may be useful include thermal sensors, photoelectric or photo optic sensors, cameras, etc. In some embodiments, one or more sensors may be used to monitor the local post passage electrical impedance or thermal conditions that may exist near the distal tip of the shaft or on the tip. Some embodiments may also comprise one or more sensors incorporating MEMS (Micro Electro-Mechanical Systems) technology, such as MEMS gyroscopes, accelerometers, galvanometers, piezoelectrics, mechanical scanning elements, diffractive elements, acousto-optic elements, capacitive sensor arrays and the like. Such sensors may be positioned at any number of locations on the TDM, including within the handle in some embodiments. In some embodiments, sensor **1414** may comprise fiberoptic elements. In an embodiment, the sensor can be configured to sense a temperature of tissue adjacent to the apparatus. The temperature sensor may alternatively be configured or sense a temperature of one or more fluids adjacent to the apparatus such as for example tissue fluids and/or fluids introduced by the surgeon.

Temperature and impedance values may be tracked on a display screen or directly linked to a microprocessor capable of signaling control electronics to alter the energy delivered to the tip when preset values are approached or exceeded. Typical instrumentation paths are widely known, such as thermal sensing thermostats, and may feed to analog amplifiers which, in turn, feed analog digital converters leading to a microprocessor. In some embodiments, internal or external ultrasound measurements may also provide information which may be incorporated into a feedback circuit. In an embodiment, an optional mid and low frequency ultrasound transducer may also be activated to transmit energy to the tip and provide additional heating and may additionally improve lysing. In some embodiments, a flashing visible light source, for example, an LED, can be mounted on the tip may show through the tissues and/or organs to identify the location of the device.

Temperature and impedance values may be tracked on a display screen or directly linked to a microprocessor capable of signaling control electronics to alter the energy delivered to the tip when preset values are approached or exceeded. Typical instrumentation paths are widely known, such as thermal sensing thermistors, and may feed to analog amplifiers which, in turn, feed analog digital converters leading to a microprocessor. In some embodiments, internal or external ultrasound measurements may also provide information which may be incorporated into a feedback circuit. In an embodiment, an optional mid and low frequency ultrasound transducer may also be activated to transmit energy to the tip and provide additional heating and may additionally improve lysing. In some embodiments, a flashing visible light source, for example, an LED, can be mounted on the tip may show through the tissues and/or organs to identify the location of the device.

In some embodiments, one or more electromagnetic delivery elements **1415** may be positioned on tip or shaft. Other embodiments may comprise one or more electromagnetic delivery elements on any other suitable location on the TDM, including but not limited to on the protrusions or otherwise on the tip, and on the shaft. Electromagnetic delivery elements that may be useful include: LEDs, LASERs, fiberoptics, filaments, photoelectric materials, infrared emitters, etc.

TDM tip **1401** may further comprise Internal Tissue Optical Motion Sensor (ITOMSensor) **1419.** ITOMSensor **1419** may comprise one or more lenses and/or windows for emitting EMR and may further comprise one or lenses and/or windows for capturing or receiving reflected EMR. In some contemplated embodiments, TDM shaft **1402** may instead comprise ITOMSensor **1419.**

In order to improve the ability of the TDM to track movement with respect to an internal tissue or region of tissue, such as the fatty undersurface of the skin, some embodiments may comprise an ITOMSensor comprising an electromagnetic emission source and a photodetector device. In, alternative embodiments, the internal tissue optical motion sensor may comprise a non-coherent light source. In, the depicted embodiment, the ITOMSensor comprises a coherent light source. Some such embodiments may comprise a motion detection algorithm configured to extract peaks & nulls for detected speckled light intensity patterns. One example of such an algorithm is disclosed in US patent 7,876,307 titled 'Motion Detection Mechanism for Laser Illuminated Optical Mouse Sensor'. Other examples of an optical sensor that may be used as an ITOMSensor are disclosed in US Patent Application publication number 2013/0016041 titled 'High Resolution Mouse'. Other examples an optical sensor that may be used as an ITOMSensor are disclosed in US Patents: US7791590B1 titled: 'Optical Mouse With Uniform Level Detection & US 5,578,813 titled: 'Freehand Image Scanning Device Which Compensates For NonLinear Movement' & US 5,644,139 titled: 'Navigation For Detecting Movement Of Navigation Sensors Relative To An Object' & US5,786,804 titled: 'Method and System For Tracking Attitude,'.

An ITOMSensor may comprise a source of non-coherent light which may be part of the motion sensor or located elsewhere on the surgical tool for illuminating a tissue at a low angle of incidence, a two dimensional array of photodetectors, each of the photo detectors producing an output in response to light reflected from surface irregularities in the tissue, and circuitry (which may be in the surgical tool or located remotely) to track movement of the housing relative to the work surface by comparing at least some of the photo detector outputs sensed at a first time with at least some of the photo detector outputs sensed at a second time if a particular condition in the photo detector outputs is identified. Motion produces successive frames of translated patterns of pixel information, which may be compared to determine the direction and distance moved.

One method for motion detection that may be used in connection with one or more embodiments disclosed herein may be based on based on the "Peak/Null Motion Detection" algorithm described in the International Patent Application WO_03/049018.

According to the "Peak/Null Motion Detection" algorithm a distinction is made between edges according to their "direction" which may be referred to as edge direction data. In some embodiments based upon the Peak/Null Motion Detection Algorithm, the ITOMSensor may comprise a coherent light source and a photodetector array. In connection with such embodiments the ITOMS may operate by illuminating under a determined gradient using a coherent light source a portion of tissue at a determined flash rate; detecting by means of the photodetector array a reflected speckled light intensity pattern from the illuminated portion of the internal tissue surface for a first flash; detecting a second reflected speckled light intensity pattern of the illuminated portion of the internal tissue surface for a second flash; extracting motion features of two different types from the detected first and second speckled light intensity patterns; determining a measurement of the relative motion between the surgical tool and the illuminated portion of the internal tissue surface based on a comparison of motion features extracted. In some embodiments, before the step of determining the measurement, preliminary step(s) (may be executed) consisting of modifying/adjusting the gradient under which the surface is illuminated In some embodiments, the method may comprise keeping only pairs of neighboring Peaks and Nulls and ignoring individually occurring Peaks and Nulls.

In some embodiments Internal Tissue Optical Motion Sensor **1419** may comprise for example one or more elements described in the aforementioned reference. In alternative embodiments ITOMSensor **1419** may be located elsewhere on modular TDM **1400** such as for example on shaft **1402.** In some embodiments, ITOMSensor **1419** may be coupled with lysing elements and/or energy window to facilitate controlled delivery of energy to tissues. For example, some embodiments may be configured to deliver a preset amount of energy such that when the device is moved faster a larger amount of energy is delivered per unit time, and when the device is moved more slowly the amount of energy is automatically decreased so as to provide a more uniform or at least semi-uniform (at least within a predetermined range) delivery of energy per unit area. In some embodiments, motion **1419 sensor** may further or alternatively be coupled with sensor **1410** and/or **1414** for example, in embodiments comprise a temperature sensor; temperature data may be used along with data from ITOMSensor **1419** in order to adjust energy output to energy window **1407** and/or lysing elements.

In the depicted embodiment, **1418** represents an antenna configured to deliver a signal to a receiver unit. In some embodiments, antenna **1418** may comprise radiofrequency identification (RFID) TAG. In some embodiments the RFID tag may comprise an RFID transponder. In other embodiments the RFID tag may comprise a passive tag. It should be understood that antenna **1418** is not depicted in every one of the other figures; any of the embodiments described herein may comprise one or more such elements. Other embodiments may comprise one or more antenna on any other suitable location on the TDM, including but not limited to on the protrusions or otherwise on the tip, and on the shaft. In embodiments in which antenna **1418** comprises an RFID transponder, the RFID transponder may comprise a microchip, such as a microchip having a rewritable memory. In some embodiments, the tag may measure less than a few millimeters. In some embodiments a reader may generate an alternating electromagnetic field which activates the RFID transponder and data may be sent via frequency modulation. In an embodiment, the position of the RFID tag or other antenna may be determined by an alternating electromagnetic field in the ultra-high frequency range. The position may be related to a 3 dimensional mapping of the subject. In an embodiment the reader may generate an alternating electromagnetic field. In some such embodiments, the alternating electromagnetic field may be in the shortwave (13.56MHz) or UHF (865-869MHz) frequency. Examples of potentially useful systems and methods for mapping/tracking a surgical instrument in relation to a patient's body may be found in U.S. Patent Application Publication No. 2007/0225550 titled "System and Method for 3-D Tracking of Surgical Instrument in Relation to Patient Body.

In some embodiments, a transmission unit may be provided that may generate a highfrequency electromagnetic field configured to be received by an antenna of the RFID tag or another antenna. The antenna may be configured to create an inductive current from the electromagnetic field. This current may activate a circuit of the tag, which may result in transmission of electromagnetic radiation from the tag. In some embodiments, this may be accomplished by modulation of the field created by the transmission unit. The frequency of the electromagnetic radiation emitted by the tag may be distinct from the radiation emitted from the transmission unit. In this manner, it may be possible to identify and distinguish the two signals. In some embodiments, the frequency of the signal from the tag may lie within a range of the frequency of the radiation emitted from the transmission unit. Additional details regarding RFID technology that may be useful in connection with one or more embodiments discussed herein may be found in, for example, U.S. Patent Application Publication No. 2009/0281419 titled "System for Determining the Position of a Medical Instrument".

In other embodiments, antenna **1418** may comprise a Bluetooth antenna. In such embodiments, multiple corresponding Bluetooth receivers at known locations may be configured to sense signal strengths from the Bluetooth antenna **1418** and triangulate such data in order to localize the signal from the Bluetooth antenna **1418** and thereby locate the TDM within a patient's body. Other embodiments may be configured to use angle-based, electronic localization techniques and equipment in order to locate the antenna **1418.** Some such embodiments may comprise use of directional antennas, which may be useful to increase the accuracy of the localization. Still other embodiments may comprise use of other types of hardware and/or signals that may be useful for localization, such as WIFI and cellular signals, for example.

One or more receiver units may be set up to receive the signal from the tag. By evaluating, for example, the strength of the signal at various receiver units, the distances from the various receiver units may be determined. By so determining such distances, a precise location of the TDM relative to a patient and/or a particular organ or other surgical site on the patient may be determined. In some embodiments, a display screen with appropriate software may be coupled with the RFID or other localization technology to allow a surgeon to visualize at least an approximate location of the tag/antenna, and therefore TDM, relative to the patient's body.

Some embodiments may be further configured such that data from the antenna(s) may be used in connection with sensor data from the TDM. For example, some embodiments of TDMs comprising one or more sensors may be further configured with one or more RFID tags. As such, data from the one or more sensors may be paired or otherwise used in connection with data from the one or more RFID tags or other antennas. For example, some embodiments may be configured to provide information to a surgeon regarding one or more locations on the body from which one or more sensor readings were obtained. To further illustrate using another example, information regarding tissue temperature may be combined with a location from which such tissue temperature(s) were taken. In this manner, a surgeon may be provided with specific information regarding which locations within a patient's body have already been treated in an effective manner and thus which locations need not receive further treatment using the TDM.

In some such embodiments, a visual display may be provided comprising an image of the patient's body and/or one or more selected regions of a patient's body. Such a system may be configured so as to provide a visual indication for one or more regions within the image corresponding to regions of the patient's tissue that have been sufficiently treated. For example, a display of a patient's liver may change colors at locations on the display that correspond with regions of the liver that have experienced a sufficient degree of fibrosis or other treatment. Such regions may, in some embodiments, be configured such that pixels corresponding to particular regions only light up after the corresponding tissue in that region reaches a particular threshold temperature.

Such sensors **1410** and/or **1414** may be coupled with an antenna, which may send and/or receive one or more signals to/from a processing unit. Alternatively, or additionally, data from such sensors resulting from tissue and/or fluid analysis using such sensors may be stored locally and transmitted later. As yet another alternative, such a signal may be transmitted following surgery. In such implementations, the signals need not necessarily be transmitted wirelessly. In fact, some embodiments may be configured to store data locally, after which a data module, such as a memory stick, may be removed from the TDM and uploaded to a separate computer for analysis.

In some embodiments tip **1401** may be attached to a robotic arm. In some embodiments, tip **1401** and portion of shaft **1402** may be attached to a robotic arm. In some embodiments, tip **1401 an**d/or a portion of shaft **1402** and/or a portion shaft and/or portion of handle **1403** may be attached to a robotic arm. In some embodiments, the robotic arm may comprise one or more motors such as a screw-drive motor, gear motor, hydraulic motors, etc. In some embodiments the robotic arm system may comprise worm gearheads, video cameras, motor control circuits, monitors, remote control devices, illumination sources, tactile interface, etc.

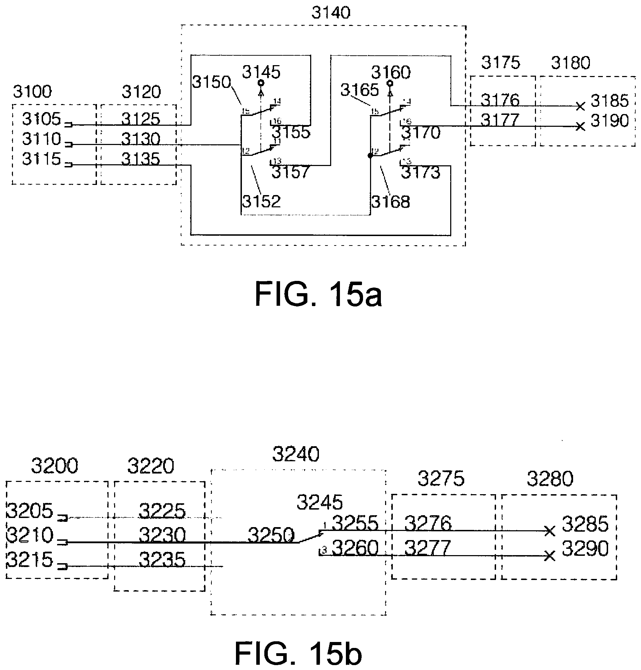

Figure 15a, depicts an embodiment wherein the electrical wiring for the TDM comprises a connector **3100,** a three-conductor cable **3120,** a first DPST (Double-pole, Single-throw) switch **3145** that may be used to deliver a coagulation waveform ("COAG DPST switch"), a second DPST switch **3160** that may be used to deliver a cutting waveform ("CUT DPST switch"), a shaft **3175** with two conductors **3176** and **3177,** and a tip **3180** comprised of a first electrode **3185** that may be used for tissue modification and/or coagulation ("COAG electrode"), and a second electrode **3190** that may be used for cutting tissue and/or tissue coagulation ("CUT electrode"). It is well known in the art that to complete a monopolar circuit either electrode may make contact with the patient who is in contact with a conductive return pad connected to the energy source. The three-conductor cable **3120** comprises one RF conductor **3130,** one CUT switch conductor **3135,** and one COAG switch conductor **3125.** The RF conductor **3130** extends from the RF pin **3110,** positioned in the connector **3100,** to the RF pins **3150** & **3152** of the COAG DPST switch **3145** and the RF pins **3165** & **3168** of the CUT DPST switch **3160** which may be positioned in the handle **3140.** The CUT switch conductor **3135** extends from the CUT switch pin **3115,** positioned in the connector **3100,** to the CUT switch pin **3173** of the CUT DPST switch **3160** which may be positioned in the handle **3140.** The COAG switch conductor **3125** extends from the COAG switch pin **3105,** positioned in the connector **3100,** to the COAG switch pin **3155** of the COAG DPST switch **3145** which may be positioned in the handle **3140.** The COAG electrode conductor **3176** positioned in the shaft **3175** extends from the COAG electrode **3185** positioned in the tip **3180,** to the COAG electrode pin **3157** of the COAG DPST switch **3145.** The CUT electrode conductor **3177,** positioned in the shaft **3175,** extends from the CUT electrode **3190** positioned in the tip **3180,** to the CUT electrode pin **3170** of the CUT DPST switch **3160.** When the COAG DPST switch **3145** is engaged, the RF pins **3150** & **3152** are electrically short circuited to the COAG switch pin **3155** and the COAG electrode pin **3157** simultaneously. When the CUT DPST switch **3160** is engaged, the RF pins **3165** & **3168** are electrically short circuited to the CUT switch pin **3173** and the CUT electrode pin **3170** simultaneously. When the COAG DPST switch **3145** is not engaged, there is electrical isolation between the short-circuited RF pins **3150** & **3152,** the COAG switch pin **3155,** and the COAG electrode pin **3157.** When the CUT DPST switch **3160** is not engaged, there is electrical isolation between the short-circuited RF pins **3165** & **3168,** the CUT switch pin **3173,** and the CUT electrode pin **3170.** When the connector **3100** is plugged into an electrosurgical generator (for example, a Valley Lab Force FX) and the COAG DPST switch **3145** is engaged, RF energy is passed through the RF conductor **3130** to the COAG electrode **3185.** Alternatively, if the CUT DPST switch **3160** is engaged while the connector **3100** is plugged into an electrosurgical generator, RF energy is passed through the RF conductor **3130** to the CUT electrode **3190.** In this manner, the RF energy delivery can be turned on and off from the handle **3140.** In other embodiments, the conductor cable **3120** comprises two conductors to accommodate electrosurgical generators utilizing two-pronged connectors or may comprise more conductors to accommodate other attributes of the device. In other embodiments, the DPST switches **3145** & **3160** may be positioned on the conductor cabling **3120** between the connector **3100** and the handle **3140;** in such embodiments the electrode conductors **3176** & **3177** leading to the electrodes **3185** & **3190** may extend through or around the shaft **3175** & handle **3140** & may comprise at least part of conductor cabling **3120.** In other embodiments, the shaft **3175** may contain more than 2 conductors. In alternative embodiments, each conductor within the conductor cable **3120** may be contained in its own cabling. In an alternative embodiment, each switch may have more than 2 positions.

In another embodiment depicted in Figure 15b, the electrical wiring for the TDM comprises a connector **3200,** a conductor cable **3220** that may comprise one or more conductors, a SPDT (Single-Pole, Double-Throw) switch **3245,** a shaft **3275** with two conductors **3276** and **3277,** a tip **3280** comprised of a first electrode **3285** that may be used for tissue modification and/or coagulation ("COAG electrode"), and a second electrode **3290** that may be used for cutting tissue and/or tissue coagulation ("CUT electrode"). It is well known in the art that to complete a monopolar circuit either electrode may make contact with the patient who is in contact with a conductive return pad connected to the energy source. The conductor cable **3220** comprises at least one RF conductor **3230** and may comprise one CUT switch conductor **3235,** and/or one COAG switch conductor **3225.** The RF conductor **3230** may extend from the RF pin **3210,** positioned in the connector **3200,** to the RF pin **3250** of the SPDT switch **3245** positioned in the handle **3240.** The CUT switch conductor **3235** may extend from the CUT switch pin **3215,** positioned in the connector **3200,** to the handle **3240.** The COAG switch conductor **3225** may extend from the COAG switch pin **3205,** positioned in the connector **3200,** to the handle **3240.** Alternatively, the COAG switch conductor **3225,** the CUT switch conductor **3235,** the COAG switch pin **3205,** and the CUT switch pin **3215** may be omitted from this embodiment. The COAG electrode conductor **3276** positioned in the shaft **3275** may extend from the COAG electrode **3285** positioned in the tip **3280,** to the COAG electrode pin **3255** of the SPDT switch **3245.** The CUT electrode conductor **3277,** positioned in the shaft **3275,** may extend from the CUT electrode **3290** positioned in the tip **3280** to the CUT electrode pin **3260** of the SPDT switch **3245.** The SPDT switch **3245** may be manipulated between two positions. In one switch position (the "COAG position") the RF pin **3250** may be electrically short circuited to the COAG electrode pin **3255.** In the other switch position (the "CUT position"), the RF pin **3250** may be electrically short circuited to the CUT electrode pin **3260.** This embodiment may be used in conjunction with another switch such as a foot switch (for example, the Valley Lab Monopolar Footswitch, E6008) which may be coupled with an electrosurgical generator (ESU) to activate/deactivate the RF energy delivered to the TDM. In some embodiments, if the foot switch is a two-pedal variant, the type of waveform that is delivered to the RF conductor **3230** when the connector **3200** is plugged into an ESU may be determined by which pedal on the footswitch is engaged. In this manner, this embodiment enables both the CUT and COAG waveform from the ESU to be delivered to the CUT electrode **3290** and/or the COAG electrode **3285** depending on the SPDT switch **3245** position. In other embodiments, the conductor cable **3220** comprises 4 or more conductors to accommodate other attributes of the device. In other embodiments, the SPDT switch **3245** may be positioned on the conductor cabling **3220** between connector **3200** and the handle **3240;** in such embodiments the electrode conductors **3276** and **3277** leading to the electrodes **3285** and **3290** may extend through or around the shaft **3275** and the handle **3240** and may comprise at least part of the conductor cabling **3220.** In other embodiments, the shaft **3275** may contain more than 2 conductors. In alternative embodiments, each conductor within the conductor cable **3220** may be contained in its own cabling. In an alternative embodiment, the switch may have more than 2 positions.

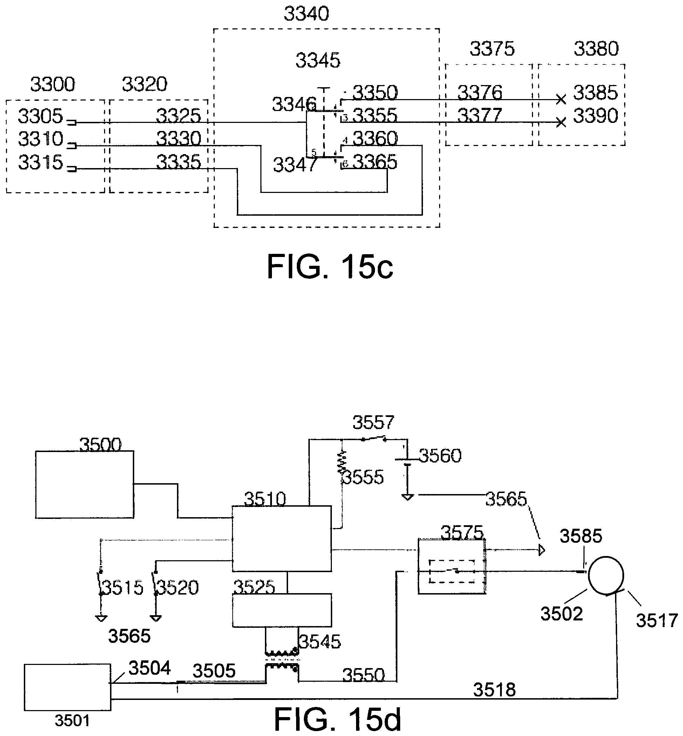

In another embodiment depicted in Figure 15c, the electrical wiring for the TDM comprises a connector **3300,** a three-conductor cable **3320,** a DPDT (Double-pole, Double-throw) switch **3345,** a shaft **3375** with two conductors **3376** and **3377,** a tip **3380** comprised of a first electrode **3385** that may be used for tissue modification and/or coagulation ("COAG electrode"), and a second electrode **3390** that may be used for cutting tissue and/or tissue coagulation ("CUT electrode"). It is well known in the art that to complete a monopolar circuit either electrode may make contact with the patient who is in contact with a conductive return pad connected to the energy source. The three-conductor cable **3320** comprises one RF conductor **3325,** one CUT switch conductor **3330,** and one COAG switch conductor **3335.** The RF conductor **3325** extends from the RF pin **3305,** positioned in the connector **3300,** to the RF pins **3346** & **3347** of the DPDT switch **3345** which may be positioned in the handle **3340.** The CUT switch conductor **3330** extends from the CUT switch pin **3310,** positioned in the connector **3300,** to the CUT switch pin **3365** of the DPDT switch **3345.** The COAG switch conductor **3335** extends from the COAG switch pin **3315,** positioned in the connector **3300,** to the COAG switch pin **3360** of the DPDT switch **3345.** The COAG electrode conductor **3376,** positioned in the shaft, **3375** extends from the COAG electrode **3385** which may be positioned in the tip **3380,** to the COAG electrode pin **3350** of the DPDT switch **3345.** The CUT electrode conductor **3377,** positioned in the shaft **3375,** extends from the CUT electrode **3390** which may be positioned in the tip **3380,** to the CUT electrode pin **3355** of the DPDT switch **3345.** The DPDT switch **3345** may be manipulated between three positions: position one, position two, or position three. In position one, the RF pins **3346 & 3347** may be electrically short circuited to the COAG switch pin **3360** and the COAG electrode pin **3350** simultaneously. In position two, the RF pins **3346 & 3347** may be electrically short circuited to the CUT switch pin **3365** and the CUT electrode pin **3355** simultaneously. In position three, there is electrical isolation between/among the short-circuited RF pins **3346 & 3347,** the COAG switch pin **3360,** the COAG electrode pin **3350,** the CUT switch pin **3365,** and the CUT electrode pin **3355.** When the connector **3300** is plugged into an electrosurgical generator (for example, a Valley Lab Force FX) and the DPDT switch **3345** is in position one, RF energy of a 'coag' waveform passes through the RF conductor **3325** to the COAG electrode **3385.** Alternatively, if the DPDT switch **3345** is in position two while the connector **3300** is plugged into an electrosurgical generator, RF energy of a 'cutting' or 'blended' waveform passes through the RF conductor **3325** to the CUT electrode **3390.** If the DPDT switch **3345** is in position three, no RF energy is delivered from the electrosurgical generator to the CUT or COAG electrodes **3385 & 3390.** In this manner, RF energy delivery can be turned on and off from the handle **3340** with one switch. In other embodiments, the conductor cable **3320** comprises two conductors to accommodate electrosurgical generators utilizing two-pronged connectors or comprises more conductors to accommodate other attributes of the device. In other embodiments, the DPDT switch **3345** may be positioned on the conductor cabling **3320** between the connector **3300** and the handle **3340;** in such embodiments the electrode conductors **3376** and **3377** leading to the electrodes **3385** and **3390** may extend through or around the shaft **3375** and handle **3340** and may comprise at least part of conductor cabling **3320.** In other embodiments, the shaft **3375** may contain more than 2 conductors. In alternative embodiments, each conductor within the conductor cable **3220** may be contained in its own cabling. In an alternative embodiment, the switch may have more than 2 positions.

The embodiment depicted in Figure 15d may be combined with the embodiments shown in Figures 15a, 15b, 15c to form the electrical wiring for the TDM. This embodiment may comprise a display **3500,** a PEM (Programmable Electronic Device) **3510,** an increment switch **3515,** a decrement switch **3520,** a filter circuit **3525,** a current sense transformer **3545,** a pull-up resistor **3555,** a power switch **3557,** a battery **3560,** and a RF relay **3575.** The circuit depicted in Figure 15d may be positioned in the source of RF energy **3501,** in the device handle, or as a separate enclosure between the source of energy **3501** and the connector of the RF conductor cable **3505,** or in line with the RF conductor (here **3505)** shown in Figures 15a, 15b, and 15c. This embodiment may utilize a PEM **3510** and relay **3575** to deterministically switch the RF energy on and off in order to modulate the RF energy delivered by the electrosurgical generator to electrode **3585.** The frequency modulation or duty cycle may be user selectable by engaging the increment switch **3515** or the decrement switch **3520.** The user selectable pulse width modulation may range from continuously on (no modulation) to a predetermined time based pulse width (e.g., 200Hz) with a duty cycle that may range from 1% to 100%, and a respective value may be visible on the display **3500** to indicate the selected setting. Once the power switch **3557** is engaged, the microcontroller switches the RF relay **3575** on and off with a modulation selected by the user. The battery **3560** may be included to provide power to the microcontroller **3510,** the filter circuit **3525,** the display **3500,** and the relay **3575.** The current sense transformer **3545** (which could also be housed in PEM **3510)** and the filter circuit **3525** may be included to facilitate feedback to the microcontroller **3510** about the electrical current delivered through the RF conductor **3550.** As electrical current passes through the RF conductor, a current is induced on the current sense transformer **3545,** which may then be scaled and filtered by the filter circuit **3525** in order to be used by the PEM **3510** to determine the status of the electrical current flowing through the RF conductor **3550.** It is well known in the art that to complete a monopolar circuit electrode **3585** may make contact with the patient **3502** who is in contact with a conductive return pad **3517** connected to the energy source **3501** via conductor **3518.** It is further well known in the art the use of signal grounds **3565** to complete various circuits.

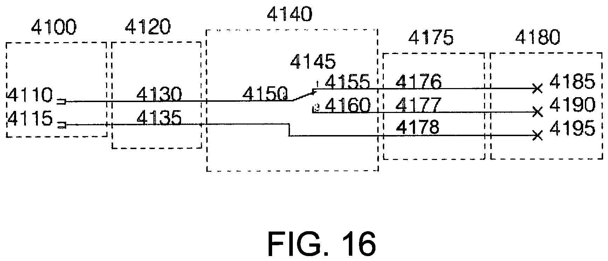

In another embodiment depicted in Figure 16, the electrical wiring for the TDM comprises a connector **4100,** a two-conductor cable **4120,** a SPDT (Single-Pole, Double-Throw) switch **4145,** a shaft **4175** with three conductors **4176, 4177,** and **4178,** a tip **4180** comprised of a first electrode **4185** that may be used for tissue modification and/or coagulation ("COAG electrode"), a second electrode **4190** that may be used for cutting tissue and/or tissue coagulation ("CUT electrode"), and a third electrode **4195** ("return electrode"). The two-conductor cable **4120** is comprised of RF conductor one **4130** and RF conductor two **4135.** RF conductor one **4130** extends from RF pin one **4110,** positioned in the connector **4100,** to the Switch RF pin **4150** of the SPDT switch **4145** which may be positioned in the handle **4140.** The COAG electrode conductor **4176** positioned in the shaft **4175** extends from the COAG electrode **4185** may be positioned in the tip **4180,** to the COAG electrode pin **4155** of the SPDT switch **4145.** The CUT electrode conductor **4177,** positioned in the shaft **4175,** extends from the CUT electrode **4190** positioned in the tip **4180,** to the CUT electrode pin **4160** of the SPDT switch **4145.** The RF conductor two **4135,** starting in the connector **4100,** extends from RF pin two **4115** that may be positioned in the connector **4100** or within another connector plugged into the electrosurgical generator, to the return electrode **4195** positioned in the tip **4180** or on the shaft **4175.** The SPDT switch **4145** can be manipulated between two positions. In one switch position the switch RF pin **4150** may be electrically short circuited to the COAG electrode pin **4155.** In the other switch position, the RF pin **4150** may be electrically short circuited to the CUT electrode pin **4160.** This embodiment requires the use of a foot switch (such as the Valley Lab Bipolar Footswitch, E6008) in conjunction with an electrosurgical generator (ESU) to activate/deactivate the RF energy delivered to the TDM. This embodiment allows RF energy from the ESU to be delivered to the CUT electrode **4190** or the COAG electrode **4185** depending on the selected SPDT switch **4145** position. If the footswitch is engaged, electric current is able to flow between the selected electrode (CUT electrode **4190** or COAG electrode **4185**) and the return electrode **4195,** provided the return electrode **4195** and one of the other electrodes both make physical contact with a contiguous, electrically conductive material (As well, plasma generation may extend the reach of the CUT or COAG electrode to effectively enable contact with the contiguous, electrically conductive material). In this embodiment, the TDM may be utilized as a bipolar device with a selectable electrode configuration. In some embodiments, the surface area that comprises the return electrode **4195** which makes contact with the electrically conductive material is optimally chosen to minimize the current density with the objective of minimizing the heating on the surface of the return electrode **4195.** In other embodiments, the conductor cable **4120** comprises more than two conductors to accommodate other attributes of the device. In other embodiments, the SPDT switch **4145** may be positioned on the conductor cabling **4120** between the connector **4100** and the handle **4140;** in such embodiments the electrode conductors **4176** and **4177** leading to the electrodes **4185** and **4190** may extend through or around the shaft **4175** and handle **4140** and may comprise at least part of conductor cabling **4120.** In other embodiments, the shaft **4175** may contain more than 3 conductors. In alternative embodiments, each conductor within the conductor cable **4120** may be contained in its own cabling. In an alternative embodiment, the switch **4145** may have more than 2 positions.

An alternative embodiment of the bipolar electrical wiring for the TDM may include a circuit that modulates the energy delivered at the electrodes similar to that described in Figure 15d.

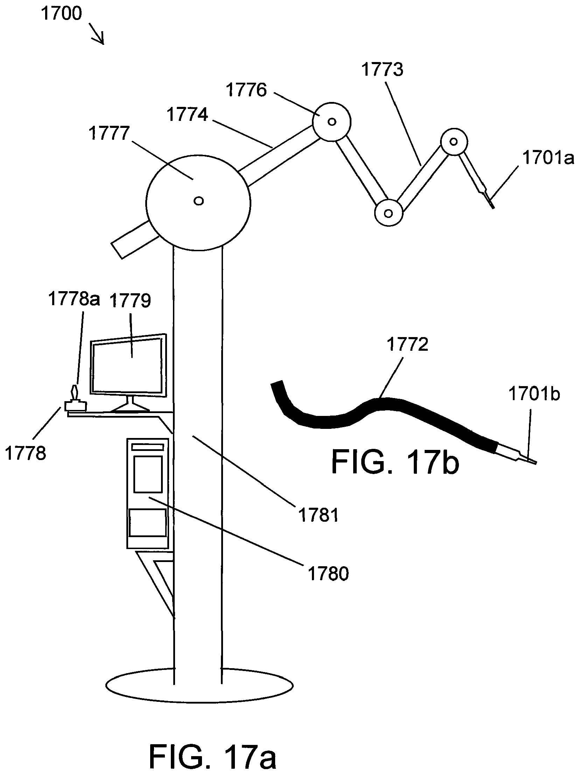

An embodiment of a system **1700** for performing robotic surgery using a modular TD is depicted in FIG. 17a. System **1700** may comprise a tissue dissecting and modifying wand (TDM) **1701a.** TDM **1701a** may comprise a tissue dissecting and modifying wand (TDM) that may, as described elsewhere herein, comprise a plurality of protrusions with one or more recessions positioned therebetween. TDM **1701a** may be coupled with one or more robotic surgery components, such as a surgical arm.

In some embodiments, TDM **1701a** may comprise a shaft, a tip, and/or a handle, as described elsewhere in this disclosure. In such embodiments, TDM **1701a** may be selectively coupled to a robotic arm such that the TDM **1701a** can either be used by hand, or coupled with one or more robotic surgery components to allow a surgeon to perform a surgical procedure with the TDM **1701a** remotely and/or indirectly. In other embodiments, the TDM may be configured to be integrally coupled with, or otherwise non-selectively coupled with, one or more robotic surgery components. In such embodiments, it may not be necessary to configure the TDM **1701a** with a handle and/or shaft. In other words, in some embodiments, the TDM **1701a** may comprise only a tip.

In some embodiments, the robotic surgery system **1700** may comprise one or more motors, such as a screw-drive motor, gear motor, hydraulic motors, etc. In some embodiments, the robotic surgery system **1700** may comprise worm gearheads, video cameras, motor control circuits, monitors, remote control devices, illumination sources, tactile interface, etc. In the embodiment depicted in FIG. 17a, TDM **1700** comprises a TDM tip **1701a** that is positioned at the end of a robotic arm. This robotic arm comprises a plurality of arm segments **1773** with corresponding joints **1776** positioned therebetween. A primary joint **1777** may be positioned to support and articulate together each of the arm segments **1773** and smaller joints **1776.** Primary joint has a primary arm segment **1774** that extends therefrom. Finer movements of the robotic arm may then be accomplished using one or more of the smaller joints **1776.**

A stand 1781 may also be provided to support the various robotic arms. In some embodiments, stand 1781 may also be configured to support a monitor 1779 and/or other display, input, or control components, such as a control element 1778. In some embodiments, control element 1778 may comprise a hand control toggle 1778a. In other embodiments, control element 1778 may comprise a keyboard, mouse, touchscreen display, virtual reality system, control pad, or the like. Monitor 1779 and/or control element 1778 may be communicatively coupled with a central processing unit 1780.

Central processing unit 1780 may comprise, for example, one or more microprocessors and/or other electronic components, such as data connectivity elements, memory, non-transitory computer readable media, etc. In some embodiments, central processing unit 1780 may comprise a general-purpose computer. Central processing unit 1780 may further comprise a machine-readable storage device, such as non-volatile memory, static RAM, dynamic RAM, ROM, CD-ROM, disk, tape, magnetic storage, optical storage, flash memory, or another machine-readable storage medium.

FIG. 17b illustrates an alternative embodiment of a robotic arm **1772** that may be used with system **1700.** Robotic arm **1772** comprises an endoscopic snake-like robotic arm **1772** and also comprises a TDM **1701b** positioned at its distal end. As with the embodiment of FIG. 7a, TDM **1701b** may be selectively coupled to robotic arm **1772** or, alternatively, may be integrally or otherwise non-selectively coupled to robotic arm **1772.** Further details regarding robotic surgery components that may be useful in connection with the various embodiments disclosed herein may be found in the following U.S. Patent Nos.: 4,259,876 titled Mechanical Arm, 4,221,997 titled Articulated Robot Arm and Method Of Moving Same, 4,462,748 titled Industrial Robot, 4,494,417 titled Flexible Arm, Particularly a Robot Arm, 4,631,689 titled Multi-Joint Arm Robot Apparatus, 4,806,066 titled Robotic Arm, 5,791,231 titled Surgical Robotic System and Hydraulic Actuator Therefore, 7,199,545 titled Robot For Surgical Applications, 7,316,681 titled Articulated Surgical Instrument For Performing Minimally Invasive Surgery With Enhanced Dexterity and Sensitivity, 8,182,418 titled Systems and Methods for Articulating An Elongate Body, 8,224,485 titled Snaking Robotic Arm With Movable Shapers.

Any of the embodiments of TD discussed herein including, but not limited to, the embodiments discussed with Figs 1-30 may be used in conjunction with one or more of the robotic surgery elements disclosed in connection with Figures 17a-b.

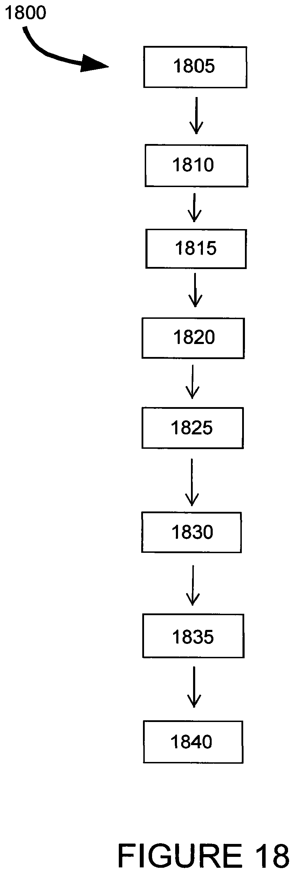

FIG. 18 depicts a flow chart of an implementation of an energy emission - sensor feedback loop **1800** according to this disclosure. Step **1805** may comprise: setting one or more temperatures (a desired maximal temperature threshold, or a range). In other implementations one or more such temperatures may be preset by the manufacturer. Step **1810** may comprise setting one or more energy levels to lysing area and/or energy windows (a desired maximal energy threshold, or a range). In other implementations energy levels may be preset by the manufacturer. Step **1815** may comprise passing the TD through or by the target tissue area. Step **1820** may comprise applying electrosurgical energy at lysing elements. Step **1825** may comprise applying energy at the energy window(s). In some implementations energy may be only applied at the lysing elements. In other implementations, energy may only be applied to the energy window(s). Step **1830** may comprise gathering sensor data, such as temperature data. Step **1835** may comprise comparing sensor data to one or more set temperature levels. Step **1840** may comprise, if the sensed temperature exceeds the threshold, reducing the amount of energy delivered through the lysing elements and/or the energy window(s).

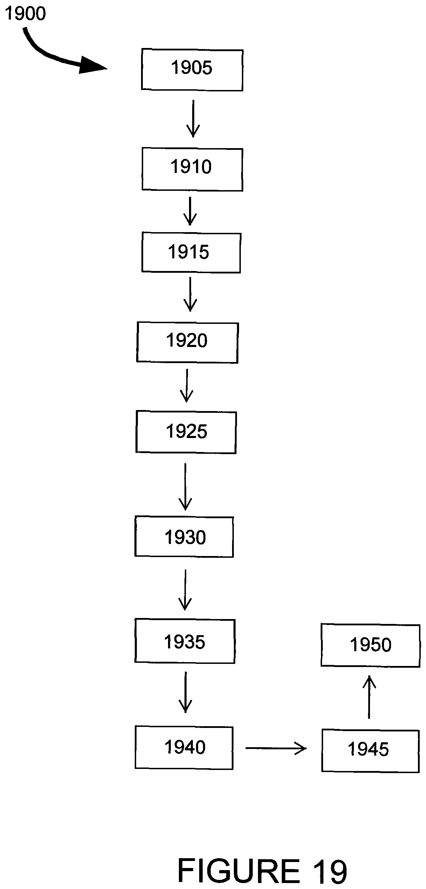

FIG. 19 illustrates an implementation of a method **1900** according to this disclosure for accessing an organ with the assistance of a TD. In some implementations, surgeon(s) may need to access tissue and/or an organ to repair or to treat it. In some implementations, the skin surrounding the anticipated entrance wound for the surgical area may be cleansed by, for example, with isopropyl alcohol (degreaser) followed by a germicide, for example, chlorhexidine scrub. Then, a local anesthetic may be applied (such as by injecting) 1% lidocaine + 1:100,000 adrenaline to the skin.

Step **1905** may comprise forming an incision. In some implementations, step **1905** may comprise making the incision using the same surgical tool, such as a TD, used to perform every step in method **1900.** In other implementations, step **1905** may be performed by another surgical instrument such as a scalpel. In some implementations, the incision may be minimally invasive. In other implementations, the incision may be open and/or invasive Step **1905** may be performed with, for example, a #15 Bard-Parker™ Scalpel. This incision may be deepened by scalpel, scissors or other surgical instrument to enter the desired body structure or cavity. For larger approaches, such as open abdominal surgery or trauma surgery step **1905** may comprise the initial skin opening or body cavity opening steps of such a procedure. In some implementations, step **1905** may comprise making the skin incision using the lysing elements of the TD. Step **1910** may comprise: applying one or more fluids to other tissue layers. In some implementations, step **1910** may comprise applying fluids to the target tissue(s). In some implementations, step **1910** may comprise applying fluids to the tissues to be traversed en route to the target tissue, in addition to, or as an alternative to applying fluids directly to the target tissue(s). In some implementations, the fluid(s) may comprise water. In some implementations, the fluid(s) may comprise an ionic fluid, such as a saline solution. The fluid(s) may be applied to the tissue via, for example, injection, or TDM fluid port or via a separate cannula or catheter or via pouring or via spray. In some implementations, the fluid(s) may comprise an ionic fluid and an anesthetic, such as a tumescent anesthesia. Non-ionic fluids may be used in other implementations; such fluids may become more ionic by diffusion of some of the patients' ions present in the surgical field. In some implementations step **1910** may comprise applying one or more fluids that serve as an ionic fluid, and/or an anesthetic, and/or adrenaline. In some such implementations, the fluid(s) may comprise a Klein Formula. In some implementations, the Klein formula and amount used may be about 100cc of Klein Formula with saline, 0.1% lidocaine, epinephrine 1:1,000,000, and NaHCO3 @5meq/L of saline).

Step **1915** may comprise: passing the TD and/or TDM through the various layers of tissue to create a path to a target organ. In some implementations, creating a path to a target organ or other target tissue may comprise creating a path from the incision to the target organ or other target tissue and/or creating a path around the target organ or other target tissue to allow for access to other regions of the target organ or other target tissue. In some implementations step **1915** may further comprise activating the lysing elements and/or energy window to reduce bleeding or tissues traversed on the way to the target organ. In some implementations, the lysing elements and/or energy window may be used to induce fibrosis along the path, including along a path that may traverse the perimeter of the target organ/tissue. In some implementations, step,may comprise visualizing the anticipated path for the TD using for example an endoscope, a fiberoptic or camera, an ITOMSensor, RFID and/or other antenna. The fiberoptic, camera and/or RFID tag may be positioned on the TD. In some implementations, such a device or devices may be positioned on the TD. In other implementations such a device or devices may be separate from the TD. 'ITOMSensor step' 1920 may comprise tracking the movement of the surgical tool with respect to an internal body tissue. In some implementations step **1920** may comprise use of image processing technology such as described herein (such as discussed before in Figs 2 & 14. In some implementations, heat may be produced or energy may otherwise be released in the tissues through which the TD is passed. In some implementations, heating portions of the tissues the TD passes by may be undesirable. As such, in some implementations, undesirable heating of such layers may be mitigated by applying a cooling step antecedent and or concurrent with energy delivery with the TD. Such steps may comprise use of one or more cooling fluids delivered via the TD or one or more separate catheters &/or cannulas &/or endoscopes. Such cooling mechanism(s) may comprise for example, a closed water bag. Such a bag may be at a temperature of less than 37°C. In some implementations, cooling objects such as fluid or gel filled bags may be used that may range in temperature between about 1°C to about 20°C. In some such implementations, the fluid or gel may be about 15°C. Other cooling mechanisms may comprise a dynamic cooling system wherein a cool liquid or gel is actively pumped into or through a contact cooling object. Step **1925** may comprise identifying important blood vessels, nerves, ducts, organs or other anatomy in the area surrounding the target tissue. Step **1930** may comprise spot coagulating one or more blood vessels, for example using spot coagulator **662** from previous FIG. 6a. Step **1930** may be repeated as needed throughout method **1900** to coagulate blood vessels as they are exposed and/or traumatized during a surgical procedure. Step **1935** may comprise: adding additional fluids of the types previously described to the target and/or surrounding tissues via the TD port(s) or via one or more separate catheters &/or cannulas &/or endoscopes. Step **1940** may comprise: expanding one or more regions of the path to the target tissue. In some implementations, step **1940** may comprise expanding one or more path(s) from the incision to the target tissue. In some implementations, step **1940** may comprise expanding a region around the target tissue such as for example, via a fanning motion. In some implementations, one or more of the other steps described herein using the TD may also be performed with a fanning motion. In implementations using TDs with axially oriented protrusions, such a fanning motion may comprise a to and fro spokewheel pattern. In implementations using TDs with non-axially oriented protrusions, such a fanning motion may comprise a side-to-side fanning motion; one example of a fanning motion using a TD having at least one nonaxially oriented protrusion may comprise a 'windshield wiper' motion. In some implementations, step **1940** may further comprise activating the energy to the TD for example the energy to the lysing elements and/or one or more energy windows. In some implementations, step **1945** may comprise spot coagulating one or more additional blood vessels that may be warranted to spot coagulate at this moment, for example using spot coagulator **662** from previous FIG. 6a. In some implementations, step **1945** may comprise repeating spot coagulation when more bleeding vessels are encountered and/or observed. In some implementations achieving hemostasis may be accomplished by cautery, electrifying, ligating, or chemical methods. In some implementations, the lysing element and/or the energy window can be used to achieve the hemostasis. In some implementations, one or more other devices and/or suture and/or surgeon's hands may be used to achieve hemostasis for larger vessels. Step **1950** may comprise: removing the TD with power off and suturing the wound in the standard fashion. In some implementations, the tissues traversed may require closure by suturing and/or stapling. In some implementations, organs and/or organ systems that the TD may be useful to access may include but not limited to muscle, and/or parotid, and/or salivary gland, and/or thyroid, and/or lung, and/or heart, and/or gastrointestinal, and/or liver, and/or pancreas, and/or spleen, and/or gallbladder, and/or kidney, and/or adrenal, and/or prostate, and/or ovary, and/or uterus, and/or bladder, and/or vascular, and/or lymph nodes and/or skeleton, and/or lung.

Any of the embodiments of TDM and/or TD discussed herein including, but not limited to, the embodiments discussed with Figs 1-29, etc. may be used in conjunction with one or more of the steps disclosed in connection with Figure 19.

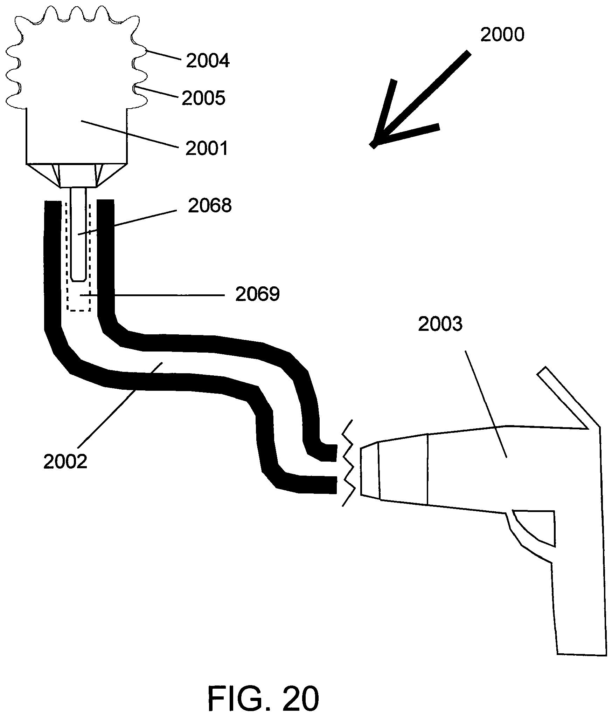

FIG. 20 depicts an embodiment of a modular TD **2000** comprising a tip **2001,** a shaft **2002,** and an endoscope handle **2003.** In some embodiments, shaft **2002** comprises a flexible shaft. Tip **2001** is modular in that it is removable from flexible shaft **2002.** More particularly, tip **2001** comprises a means for removably coupling the tip with a shaft at **2068.** In the depicted embodiment, this coupling means comprises a tip plug **2068.** In some embodiments, tip plug **2068** may be threaded to facilitate a secure coupling between modular tip **2001** and shaft **2002.** However, in other embodiments, the coupling means may comprise a recess configured to receive a plug formed on the shaft. In still other embodiments, the coupling means may comprise a snap-fit coupling, a friction fit coupling, a bayonet clip, etc.

In the depicted embodiment, tip plug **2068** is configured to be received within a corresponding recess **2069** formed within shaft **2002.** In some embodiments, tip plug **2068** may be configured to electrically couple tip **2001** with shaft **2002.** In this manner, in embodiments comprising, for example, lysing elements, electricity from a power source may be transmitted through the coupling between plug **2068** and recess **2069** to allow for energizing the lysing elements. Other embodiments may be configured to transfer additional electricity, data, or materials through such coupling. For example, in embodiments comprising one or more sensors on tip **2001,** a signal from such sensor(s) may be transmitted through shaft **2002** by way of the coupling means **2068.**

In some embodiments, tip **2001** may be disposable as well, such that a surgeon can place an appropriate tip on the shaft and remove and dispose of the tip after surgery. Alternatively or additionally, a plurality of different tips may be provided, each of which may be disposable, or may be configured for sterilization and re-use, and an appropriate tip may be selected as needed for a particular surgery.

In the depicted embodiment, tip **2001** comprises a plurality of protrusions **2004,** some of which are non-axial, and a plurality of recessions **2005** positioned therebetween, as described above. In some embodiments a tip comprising only axial protrusions may be swapped for tip **2001** as desired to suit a particular surgical procedure.

Any of the embodiments of TD discussed herein including, but not limited to, the embodiments discussed with Figs 1-30, etc. may be used in conjunction with one or more of the steps disclosed in connection with Figure 20.

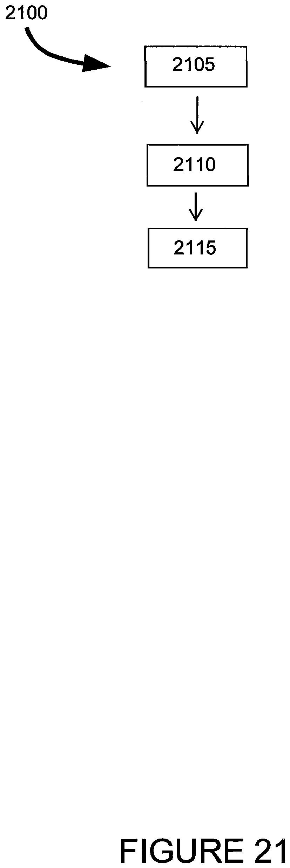

FIG. 21 depicts a flow chart of an implementation of a method 2100 for separating and/or modifying tissue using a TD. In this particular implementation, the use of combined data from the tissue dissecting and modifying wand generated from at least the temperature sensor and the ITOMSensor(s) may be used to provide suitable feedback to a user during treatment. In some implementations, the TD may comprise a tip comprising a plurality of protrusions. One or more lysing elements may be positioned between at least two adjacent protrusions among the plurality of protrusions. A temperature sensor may be positioned on the TD. The temperature sensor may be configured to sense a temperature of at least one of tissue and fluid adjacent to the TD during an operation. The fluid of which a temperature reading is taken may comprise, for example, fluid from adjacent tissue(s) and/or fluid introduced during the procedure by way of the TD and/or another device or procedure. The TD may also comprise an ITOMSensor positioned on the TD. In some implementations, the ITOMSensor(s) may be positioned on the tip and/or distal end of the shaft. The ITOMSensor(s) may be configured to provide speed of motion data regarding the TD, during an operation or procedure. Although method **2100** is shown in the figure beginning with step **2105,** it should be understood that any of the preliminary steps described above in connection with other implementations may be performed in method **2100** as well. For example, one or more of steps (**1905-1940**) from method **1900** may be performed in method **2100** if desired. Similarly, one or more other steps of any of the other implementations described herein may also be included in the method depicted in Figure 21. In some implementations, step **2105** may comprise: receiving data from the TD temperature sensor. Step **2110** may comprise receiving data from the ITOMSensor. Step **2115** may comprise combining the data generated from at least the temperature sensor and the ITOMSensor (s). In some implementations, the data from the temperature sensor and the ITOMSensor (s) may be combined before it is received. In other words, a step of "receiving combined data from the tissue dissecting and modifying wand generated from at least the temperature sensor and the ITOMSensor (s) may comprise receiving precombined data (data from the temperature sensor and the ITOMSensor (s) that was combined before it was received) or, alternatively, may comprise separately receiving temperature data and ITOMSensor (s) data that may be combined to allow for one or more particular features or functionalities. The combined data may be used to allow a surgeon or other user to determine one or more regions within a patient's body that have been adequately treated using the TD. For example, in some implementations, the combined data may allow a user to determine whether one or more regions within a patient's body have been sufficiently treated, and/or to modulate one or more aspects of energy output to maintain desired treatment parameter(s). In some implementations, determining whether one or more regions within a patient's body have been sufficiently treated may comprise visualizing one or more regions within a patient's body during treatment. This may be accomplished, for example, by creating an image corresponding with one or more regions of a patient's body. In some implementations, for TD comprising lysing elements but lacking an energy window, the combined data may be processed and/or electromagnetic lysing element output parameters automatically modulated. In some implementations, for TD comprising lysing elements and an energy window, the combined data may be processed &/or electromagnetic lysing element output parameters automatically modulated &/or energy window output parameters automatically modulated. For example, in implementations wherein an LASER energy window is present the following may be modulated: LASER power &/or wavelength &/or beam intensity &/or pulse duration &/or pulse rate &/or emission rate, For example, in implementations wherein an RF/electrosurgical energy window is present the following may be modulated: power &/or waveform &/or voltage &/or amperage &/or pulse duration &/or pulse rate &/or duty cycle. In some implementations, such adequately treated regions may correspond with regions comprising tissue that has reached a predetermined threshold temperature.

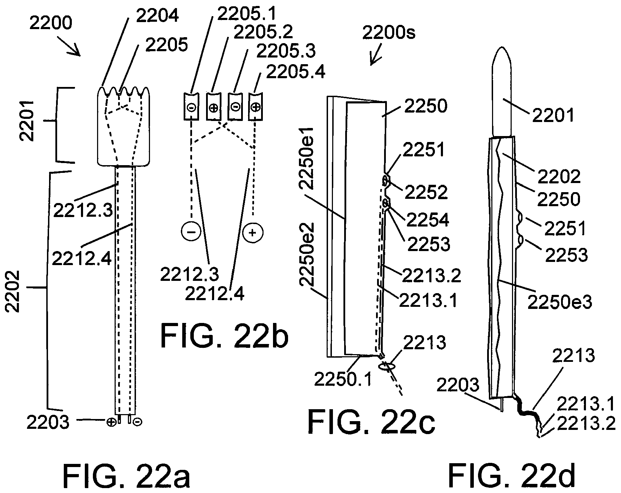

FIG. 22a depicts an embodiment of a modular bipolar Tissue Dissector (modular bipolar TD) **2200** comprising a modular bipolar TD tip **2201,** a modular bipolar TD shaft **2202** and a modular bipolar TD plug **2203.** In the depicted embodiment, protrusions **2204** and lysing elements **2305** are oriented an axial direction. An external power cord may bring RF electrosurgical energy from an electrosurgical generator to modular bipolar TD plug **2203** (which is electrically connected) to modular bipolar TD shaft **2202** and thus to electrically conductive lysing elements **2205,** mounted in the recessions in between protrusions, such as protrusions **2204.** In some embodiments, the protrusions may comprise bulbous protrusions. The tip shown in this embodiment has five relative protrusions **2204,** four lysing elements **2205** and four relative recessions pointing along the main axis of the modular bipolar TD. In other embodiments, the modular bipolar TD tip may have one or more non-axial protrusions and one or more non-axial relative recessions. In some embodiments, the tip may have between 3 and 100 axial and/or non-axial protrusions and/or relative recessions. It should be understood that the number of protrusions need not match the number of lysing elements or recessions. In some embodiments, lysing elements may be located at the termini of conductive elements. In some embodiments, lysing elements may also be made partially or completely of a cermet material. In an embodiment, the modular bipolar TD tip **2201** may measure about 15mm in width, about 18mm in length and about 5mm in maximal thickness; the modular bipolar TD shaft **2202** may measure about 6mm in diameter and/or about 60mm in length; the overall modular bipolar TD **2200** may measure about 15mm in width, 80mm in length and about 6mm in maximal thickness. Embodiments are contemplated wherein sizes of about one-fifth to about five times these dimensions may have possible uses. It is also contemplated, for example in some veterinary embodiments; tip sizes of about one-tenth to 20 times the aforementioned dimensions may also have possible uses. In some embodiments, wherein electrical insulation and/or polymeric insulating coating is present on such parts, for example modular bipolar TD shaft **2202,** such insulation may measure about 0.5mm in thickness; in some contemplated embodiments, the insulation thickness may range from 0.1mm to 3mm. In the depicted embodiment, electrical leads **2212.3** and **2212.4** may course from plug **2203** via shaft **2202** to energize various lysing elements located in bipolar TD tip **2201.** In some embodiments leads may comprise wires and/or conductive conduits. In other contemplated embodiments leads may be printed on via techniques that may be akin to those of MicroPen™ discussed elsewhere in this application.

FIG. 22b is an upper plan view of the four lysing elements of the embodiment depicted in FIG. 22a. In this embodiment, lysing elements may be comprised of even numbers of oppositely charged (when activated) individual lysing elements **2205.1, 2205.2, 2205.3** and **2205.4.** In this embodiment, individual bipolar TD lysing elements **2205.1, 2205.2, 2205.3** and/or **2205.4** may comprise surgical grade stainless steel positioned within all and/or a portion of one or more pieces of ceramic and/or other thermally resistant, non-conductive housing. In some embodiments, one or more individual lysing elements may comprise electroconductive materials including but not limited to cermets, steel, nickel, alloys, palladium, gold, tungsten, titanium, silver, copper, and/or platinum. In the depicted embodiment, the lysing elements may measure about 2mm in width, 5mm in length and about 0.5mm in thickness. In the depicted embodiment, the axial lysing elements are concave and crescentic in shape. However, in other contemplated embodiments lysing elements may comprise straight and/or convex and/or a variety of shapes. In other contemplated embodiments, lysing elements may vary from about one-fifth to about 20 times the aforementioned sizes and be of a variety of shapes. In the depicted embodiment, similarly charged individual lysing elements **2205.1**, **2205.3** are energized by 'negative' lead **2212.3**; similarly charged individual lysing elements **2205.2**, **2205.4** are energized by 'positive' lead **2212.4**.

In some contemplated embodiments, lysing elements carrying similar charges need not be individual but may be joined and/or continuous with one or more lysing elements stamped and/or made from a common piece. In other embodiments, lysing elements may be formed and/or joined from one or more conductive parts.

In some contemplated embodiments there need not be equal numbers of oppositely signed and/or charged individual lysing elements, for example, there may be 3 positive and 2 negative individual lysing elements. Uniformity of flux on activation may be achieved by modifying the size and/or position of lysing elements with respect to each other among other methods known in the art.

The relative static permittivity of some ceramics may range from about 5 to 10; this may cause some leakage of current in an undesirable path between closely approximated opposing electrodes during activation. Use of other materials, for example, those having over of relative static permittivities over 5 may undesirably alter the resultant plasma field. The relative static permittivity of the intervening materials housing the opposing electrodes may be enhanced by coating and/or surrounding and/or injection molding thermoresistant polymers of a low relative static permittivity into the housing and/or around one or more portions of bipolar lysing elements **2205** to reduce the effective static permittivity of the tip. In an embodiment, the thermoresistant polymer of low relative static permittivity 2.1 may be polytetrafluoroethylene. In other contemplated embodiments, thermoresistant polymers may include polyether etherketone (@3.3) and/or polysulfone (@3.1) and the like may be useful.

In the depicted embodiments, the electrical insulator comprises polytetrafluoroethylene. In other contemplated embodiments, the electrical insulator may comprise an electrically nonconductive polymer with a high melting temperature. In some embodiments, the nonconductive polymer may comprise for example, polyether etherketone and/or polysulfone, etc. In other contemplated embodiments, the electrical insulator may comprise an electrically nonconductive and/or thermally nonconductive polymer.

FIG 22c is a side view of a bipolar circuit activating foot-pedal bypass (BCAFPB) **2200s,** which may be coupled with a surgical tool such as modular bipolar TD **2200 as** depicted in FIG. 22d. In the embodiment depicted in Fig 22c the BCAFPB comprises a sticker. In some embodiments, the sticker may be configured to wrap around the surgical tool. BCAFPB 2200s is one example of a Means for Allowing Hand Control of a Footpedal Activated Surgical Tool (MAHCFAST). In some embodiments comprising a MAHCFAST, the foot control may be disabled in favor of the hand control. In other embodiments, the MAHCFAST may facilitate hand control without disabling foot control. Sticker **2200s** may comprise an adhesive backed wrapper or the like, pressure activated on-off switch **2252,** casing **2251** for switch **2252,** casing **2253** for a second switch and/or second switch **2254** (which may be used to control another function of the electrosurgical generator such as increasing or decreasing the power and/or other settings), electric lead **2213.1** (which connects to switch **2252**), and electric lead **2213.2** (which may connect to second switch **2254**) and opening **2250.1.** Electric leads **2213.1 & 2213.2** may be bound together in conduit **2213.** Sticker **2250** may comprise silicone and/or plastic and/or polyethylene and/or polyurethane and/or polypropylene and/or any other sterilizable and/or biocompatible polymer, etc. In the depicted embodiment, sticker **2250** comprises edges **2250e1** & **2250e2** & a sterile pressure sensitive acrylic adhesive. In some embodiments, sticker **2250** may be stretchable and/or elastic in order to fit more tightly over contours in the intended bipolar surgical instrument. In other contemplated embodiments, sticker **2250** may comprise pressure sensitive adhesives including but not limited to those based on: silicone, polyester, polyether & polyurethane. In other contemplated embodiments, sticker **2250** may be rigid and may require pleating along some of its axis in order to take up some extra slack following adhesion. Once applied to a surgical tool, edges **2250e1** and **2250e2** may for some surgical tools overlap; for other surgical tools the edges need not overlap.

A large portion of bipolar hand-held electrosurgical instruments are foot-pedal activated. Some surgeons may complain about having their feet restricted to the zone around the foot-pedal and/or about striking the wrong plate of the foot-pedal and/or having to find the foot-pedal during a lengthy procedure. Foot-pedal activation may also be disadvantageous for certain procedures since significant damage to vital organ(s) may happen in a fraction of a second if electrosurgical energy is applied erroneously. It may therefore be desirable for surgeons to have a method of hand control available over the foot-pedal circuitry; especially a control which is located adjacent and/or affixed to the bipolar instrument. It may be convenient to have the quicker hand response time available in a removable sheath that may accommodate and/or 'fit' over a one or more hand held bipolar instruments. Reported differences between eye-hand and eye-foot response times (0.28sec for hand versus 0.45sec for foot) may be in large part due to the extra distance nerve impulses must travel to the foot. (reference: J Amer Optom Asn, 2000; 71(12) 775-780). Metal bands and/or a glue seal and/or a tie off and/or another type of fastener may be located at any positions throughout **2250.1** to prevent continued movement of shaft **2202** within the sticker.

It is contemplated that BCAFPB may be customized to a particular surgical tool by way of shape and/or size and further that a variety of shapes and/or sizes may be provided, each of which may be designed to work in conjunction with one or more surgical bipolar tools. It is also contemplated that some BCAFPB **2250** may be cut to size prior to or during the operation. It is also contemplated that many alternative components and features may be provided for example, some embodiments may comprise in place of or in addition to pressure activated on off switch **2252** and or **2254** one or more other types of control elements such as flip switches, optically activated buttons, electrostatically operated buttons, and the like. In other contemplated embodiments the means for allowing hand control of a footpedal activated surgical tool may instead comprise a sheath, a clip, a ring, or the like that may be coupled with a bipolar surgical tool by any available means such as an adhesive, welding, hook and loop fastener and the like.

FIG. 22d is side view depicting an embodiment of a sticker **2200s** placed over bipolar TD shaft **2202.** In the depicted embodiment tip **2201** is fully exposed after application of sticker 2200s, however in other embodiments, sticker **2200s** may be configured to also partially cover tip **2201.** Similarly, although in the depicted embodiment sticker **2200s** fully covers shaft **2202,** in other embodiments, sticker **2200s** may only cover a portion of shaft **2202** and/or another portion of surgical tool **2200.** In the embodiment depicted in FIG. **22d****,** edges **2250e1** & **2250e2** overlap as shown at **2250e3,** the amount of overlap may vary depending upon the surgical tool.

As of the year 2000, the bipolar mode had traditionally been used primarily for coagulation, (reference: "The Biomedical Engineering Handbook, Electrosurgical Devices" J Eggleston, W Maltzahn, Ch 81, CRC Press 2000). However, more recent modifications to bipolar electrosurgical outputs may have facilitated the use of bipolar cutting instruments (reference: ValleyLab, Hotline, vol. 4, issue 4 pg. 1), examples of such outputs may include Macrobipolar settings (Reference: ValleyLab ForceTriad Users Guide 2006, chapter/sections: 9-13, 9-16, 9-24).

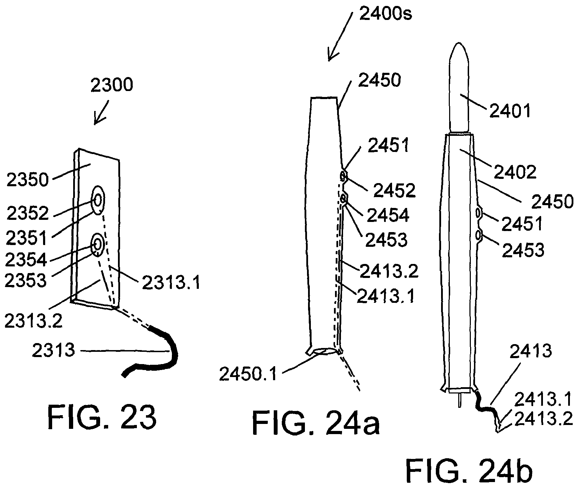

FIG. 23 depicts an embodiment of a BCAFPB comprising an adhesive backed pad. Adhesive backed pad **2300** should also be considered an example of a sticker. FIGs. 23 depicts an embodiment that differs from the embodiment depicted in FIGs. 22c,d in that it comprises a smaller adhesive pad **2300** and FIGs. 22 comprises a larger sticker. Each of the other elements depicted in FIGs. 23 may be identical to the corresponding elements shown in FIGs. 22c,d and are referenced by like numerals (numbers higher by 100). Pad **2300** may comprise an upper surface **2350.** An opposite lower surface (not shown) may comprise an adhesive for coupling pad **2300** with a surgical tool. One or more buttons or button encasements such as buttons **2352** & **2354** and encasements **2351** & **2353** may be positioned on upper surface **2350.** In alternative embodiments, one or more buttons **2352** & **2354** may be replaced by alternative control elements as discussed above. Electrical lead **2313.1** is electrically coupled with button **2352.** Similarly, electrical lead **2313.2** is electrically coupled with button **2354.** Leads **2313.1** & **2313.2** may be bound together in conduit **2313.**

FIGs. 24a,b depict an embodiment that differs from the embodiment depicted in FIGs. 22c,d in that FIGs. 24a,b comprises a sheath **2400** and FIGs. 22c,d comprises a sticker. Each of the other elements depicted in FIGs. 24a,b may be identical to the corresponding elements shown in FIGs. 22c,d and are referenced by like numerals (numbers higher by 200). For example, in FIGs. 24a,b sheath **2400s** comprises a tubular body **2450** comprising at least one open end **2450.1.** Sheath **2400s** also comprises casing **2451,** switch **2452,** second casing **2453**, second switch **2454,** corresponding surgical leads **2413.1** & **2413.2.** FIG. 24b depicts sheath **2400s** after it has been positioned over shaft **2402.** In the depicted embodiment, sheath **2400s** is configured to be put in to position such that tip **2401** is fully exposed. However in other embodiments sheaths may be configured to only partially expose a tip of a surgical tool.

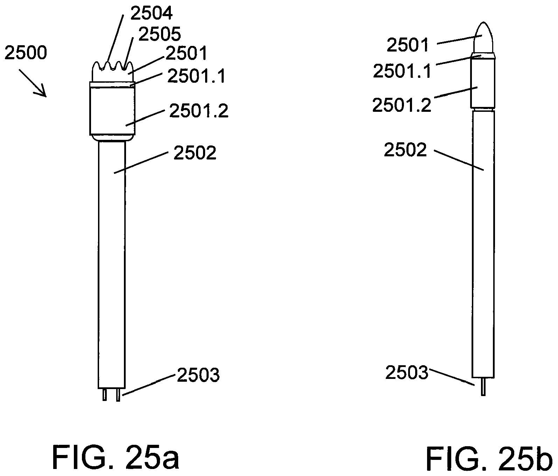

FIG. 25a depicts an embodiment of a modular common intermediate configuration Tissue Dissector (modular CiCTD) **2500** comprising a modular CiCTD tip **2501,** a modular CiCTD shaft **2502** and a modular CiCTD plug **2503.** Embodiments wherein both electrodes are located on the same probe and/or device, but the return electrode is larger than the active electrode may be termed by some surgeons as 'common intermediate configuration.'

In the depicted embodiment, protrusions **2504** and lysing elements **2505** are oriented an axial direction and return electrode **2501.2** may be located on the tip but electrically isolated from each of the lysing elements. An external power cord may bring RF electrosurgical energy from an electrosurgical generator to modular CiCTD plug **2503** (which is electrically connected) to modular CiCTD shaft **2502** and thus to electrically conductive lysing elements **2505,** mounted in the recessions in between protrusions, such as protrusions **2504.** In some embodiments, the protrusions may comprise bulbous protrusions. The tip shown in this embodiment has four relative protrusions **2504,** three lysing elements **2505** and three relative recessions pointing along the main axis of the CiCTD. In other embodiments, the modular CiCTD tip may have one or more non-axial protrusions and one or more non-axial relative recessions. In some embodiments, the tip may have between 3 and 100 axial and/or non-axial protrusions and/or relative recessions. It should be understood that the number of protrusions need not match the number of lysing elements or recessions. In some embodiments, lysing elements may be located at the termini of conductive elements. In some embodiments, lysing elements may also be made partially or completely of a cermet material. In some embodiments, return electrodes may also be made partially or completely of a cermet material. In an embodiment, the modular CiCTD tip **2501** may measure about 12mm in width, about 15mm in length and about 5mm in maximal thickness; the modular CiCTD shaft **2502** may measure about 6mm in diameter and/or about 60mm in length; the overall modular CiCTD **2500** may measure about 12mm in width, 75mm in length and about 6mm in maximal thickness. Embodiments are contemplated wherein sizes of about one-fifth to about five times these dimensions may have possible uses. It is also contemplated, for example in some veterinary embodiments; tip sizes of about one-tenth to 20 times the aforementioned dimensions may also have possible uses. In some embodiments, wherein electrical insulation and/or polymeric insulating coating is present on such parts, for example modular CiCTD shaft **2502,** such insulation may measure about 0.5mm in thickness; in some contemplated embodiments, the insulation thickness may range from 0.1mm to 3mm.

In upper plan view of the depicted embodiment of FIG. 25a, the visible CiCTD lysing element **2505** portions may be part of a larger piece of active electrode of surgical grade stainless steel lying within all and/or a portion of one or more pieces of ceramic and/or other thermally resistant, non-conductive housing. In some embodiments, one or more pieces of active electrode may be comprised of electroconductive materials including but not limited to cermets, steel, nickel, alloys, palladium, gold, tungsten, titanium, silver, copper, and/or platinum. In the depicted embodiment, the piece of active electrode comprises a plate of about 0.5mm in thickness. In other embodiments, lysing elements may be formed from one or more conductive parts. The relative static permittivity of some ceramics may range from about 5 to 10; this may cause some leakage of current in an undesirable path during activation of active electrode. In some embodiments, wherein a return electrode is positioned on the exterior of CiCTD tip **2501,** the relative static permittivity of the intervening materials housing the active electrode (and as may be the case in the CiCTD tip) may be enhanced by coating and/or surrounding and/or injection molding thermoresistant polymers &/or thermoresistant materials of a low relative static permittivity into the housing and/or around one or more portions of an active electrode piece comprising, at least in part, CiCTD lysing element **2505.** In an embodiment, the thermoresistant polymer of low relative static permittivity 2.1 is polytetrafluoroethylene. In other contemplated embodiments, thermoresistant polymers may comprise polyether etherketone (@3.3) and/or polysulfone (@3.1) and the like which may be useful. In other contemplated embodiments, thermoresistant materials may comprise polyether etherketone &/or PFTE with hollow glass microspheres.

In the depicted embodiments of FIGS. 25a & b, CiCTD tip return electrode 2501.2 may be located on one or more sides and/or edges of modular CiCTD tip 2501 In the depicted embodiment, CiCTD tip return electrode 2501.2 is an external band comprised of a biocompatible conductive metal, for example, surgical stainless steel, tightly fastened to the modular CiCTD tip 2501. In some embodiments, CiCTD return electrode is located on the external aspects of tip 2501 and/or shaft 2502. In some embodiments, CiCTD tip return electrode 2501.2 may be comprised of electroconductive materials including but not limited to cermets, steel, nickel, alloys, palladium, gold, tungsten, titanium, silver, copper, and platinum. In some embodiments, modular CiCTD tip may comprise materials that are electrically non-conductive and/or of low thermal conductivity; such materials may comprise, for example, porcelain, ceramics, glass-ceramics, plastics, varieties of polytetrafluoroethylene, carbon, graphite, and/or graphite-fiberglass composites, etc. In some embodiments, the modular TD tip may be constructed of a support matrix of an insulating material (e.g., ceramic or glass material such as alumina, zirconia). In some embodiments, the modular TD tip may comprise cermets.

In other contemplated embodiments, the modular TD tip may comprise polyether etherketone &/or polytetrafluoroethylene with hollow glass microspheres.

In some embodiments, the CiCTD tip return electrode **2501.2** may be in the shape of a plate and/or plane and/or may be made of any metal or alloy that does not melt under operating conditions or give-off toxic residua. In the depicted embodiment, the CiCTD tip return electrode **2501.2** comprises a band of surgical grade stainless steel comprising a thickness of 0.5mm and a thickness of about 1cm which may be wrapped around CICTD tip **2501.** Embodiments are contemplated of the CiCTD tip return electrode **2501.2** wherein sizes of about one-fifth to about five times these dimensions may have possible uses. It is also contemplated, for example in some veterinary embodiments; tip sizes of about one-tenth to 20 times the aforementioned dimensions may also have possible uses. In some embodiments, the CICTD tip has curviform edges thus reducing sharp edge formation on the overlying portions of the return electrode **2501.2.** Reducing sharp edges on the return electrode may mitigate unwanted electronic contact sequelae such as burns. In some embodiments, CiCTD tip return electrode **2501.2** may have any geometric shape including a wire, and may be positioned along any side or portion of the modular CiCTD tip **2501** and/or shaft **2502** excluding the recessions. It may be possible that sharp edges and/or corners and/or surface irregularities and/or surface defects on return electrode **2501.2** may contribute to a concentration of energy transfer when touched to a portion of the patient. Therefore, in some embodiments, it may be beneficial to have rounded and/or radiused edges and/or rounded corners. Surgeons may have a higher likelihood of an unwanted inadvertent 'touch' of adjacent patient tissue from the smaller of the sides; therefore in some embodiments, return electrode **2501.2** may be located on one or more of the larger substantially planar aspects of CiCTD tip **2501.** In the depicted embodiment, an electrical insulator **2501.1** covers the edge of the return electrode closest to the active electrodes. Electrical insulator **2501.1** may comprise a band positioned along the periphery of return electrode **2501.2.** In the depicted embodiments, the electrical insulator comprises polytetrafluoroethylene. In other contemplated embodiments, the electrical insulator may comprise an electrically nonconductive polymer with a high melting temperature. In some embodiments, the nonconductive polymer may comprise for example, polyether etherketone and/or polysulfone, etc. In other contemplated embodiments, the electrical insulator may comprise an electrically nonconductive and/or thermally nonconductive anti-stick polymer. In some embodiments a nonconductive coating may cover any portion of a return electrode and/or may have any shape. In some embodiments, the electrical insulator may cover about 20% of the return electrode surface. In other embodiments, the electrical insulator may cover a percentage of the return electrode that may range from 1% to 90%. In other embodiments, a portion and/or all of a return electrode may be covered with a porous substance of a lower relative static permittivity such that electrical charge transfer is reduced, such as alumina. Such a porous coverage may reduce the chance of a tissue burn from inadvertent touching and/or heating.

In some embodiments, electrodes may be printed onto a ceramic and/or nonconductive surface by various methods, for example using the Micropen™ of OhmCraft technique. In some embodiments, return electrodes **2501.2** may be printed onto a ceramic and/or nonconductive surface. In some embodiments, lysing elements comprising electrodes may be printed on or about the relative recessions.

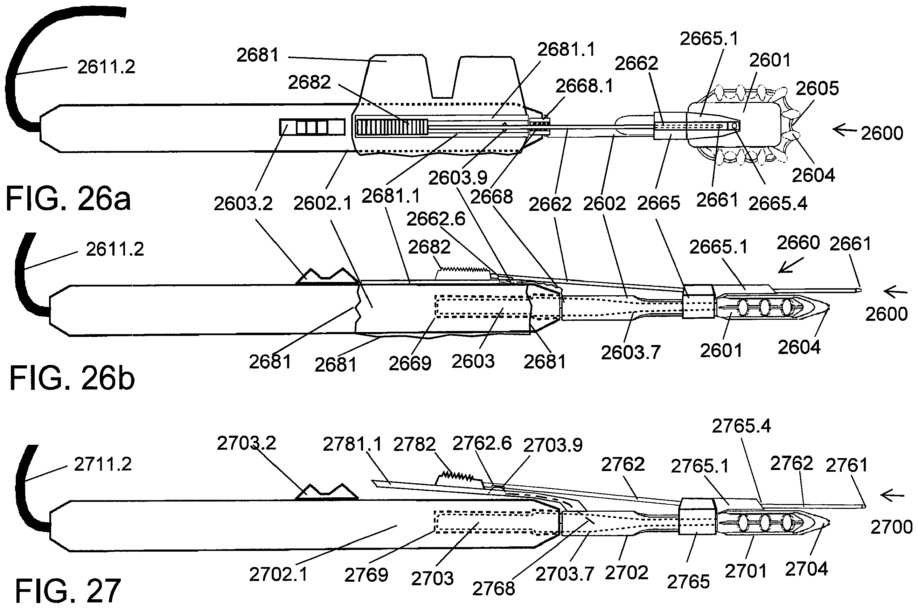

FIGS. 26a,b depict an embodiment comprising an axially advanceable SC **2600.** SC comprises SC shaft **2662,** SC tip **2661** and/or SC coupler **2665.** In the depicted embodiment, the Modular TD comprises a Modular TD tip **2601,** a Modular TD shaft **2602,** a Modular TD plug **2603.** The modular TD **2600** depicted in this embodiment has non-axial protrusions. In other embodiments, the modular TD may comprise axial protrusions and lack non-axial protrusions.

SC shaft may be coupled with modular TD by way of coupler **2665.** The depicted embodiment also comprises axial protrusions, such as **2604,** and axial lysing segments **2605** as well as non-axial & transitional protrusions. Transitional protrusions, axial protrusions, non-axial protrusions, axial lysing elements, non-axial lysing elements and lysing elements of differing geometries are discussed in more detail elsewhere in this application. Modular TD **2600** is modular in that it is removable from an electrosurgical 'pencil' shaft **2602.1** and/or an electrosurgical 'pencil' handle. Rocker switch **2603.2** may control the electrosurgical energy such as a suitable waveform derived from an electrosurgical generator (not seen in this view) brought into the handle via conduit **2611.2.** In the depicted embodiment modular TDM tip plug **2603** is configured to be received within a corresponding electrosurgical 'pencil' shaft recess **2669** formed within electrosurgical 'pencil' shaft **2602.1.** In some embodiments, elements within modular TDM tip plug **2603** and/or electrosurgical 'pencil' shaft recess **2669** may be electrically connected with electrical elements within 'pencil' shaft **2602.1** and/or 'pencil' handle which are in turn connected with 'pencil' switch **2603.2** and/or conduit **2611.2.**

So as not to have the surgeon set down the TD or TDM, to pick up other instrumentation from the surgical tray to stem bleeding blood vessel(s), it may be beneficial during some surgical procedures to have spot coagulator coagulation capabilities within the same instrument.

In the depicted embodiment, modular TD shaft **2602** may comprise an electrical insulator and/or thermally insulating and/or supportive coating and/or cover overlying a conductive material and/or metal axial core (shown in phantom) **2603.7;** In some embodiments, the conductive material may comprise: steel, nickel, alloys, palladium, gold, tungsten, silver, copper, platinum and/or any other conductive metal that does not give off toxic residua at operating temperatures. In other contemplated embodiments, the conductive material may comprise cermets and the like. In some embodiments, the electrical insulator may comprise, for example, porcelain, ceramics, glass-ceramics, plastics, various halogenated carbon molecules, polytetrafluoroethylene, carbon, graphite, and graphite-fiberglass composites and the like. In alternative embodiments the electrical insulator may comprise polyether etherketone and/or polysulfone and/or another electrically nonconductive polymers (with thermal stability in the operating range) and/or materials that are both electrically non-conductive and of low thermal conductivity. SC tip 2661 shapes may comprise those discussed in other tip embodiments in this application. In the depicted embodiment SC shaft **2662** is slidably coupled to the modular TD shaft **2602** of the modular TD by SC coupler **2665.** SC coupler **2665** is one example of a means for moveably coupling a SC with a surgical tool. SC coupler **2665** may be configured to allow SC shaft **2662** to slide relative to SC coupler **2665** and modular TD shaft **2602.** In the depicted embodiment, SC coupler **2665** is rigidly affixed to TD shaft **2662.** In contemplated embodiments, SC coupler **2665** may be configured to slide relative to TD shaft **2602.** In such embodiments, SC coupler **2665** may be rigidly affixed to SC shaft **2662.** In some embodiments, SC coupler **2665** may comprise a sterilizable plastic and/or polymer and/or ceramic and/or polytetrafluoroethylene and/or other non-conductive and/or insulating material. In some embodiments, SC coupler **2665** may comprise an integral part of modular TD shaft **2602.** In contemplated embodiments, SC coupler **2665** may comprise an integral part of modular TD tip **2601.** In other embodiments SC coupler **2665** may comprise a separate component that may be coupled with modular TD tip **2601** by way of for example form fitting, snap fitting, an adhesive, welding, and the like. One or more passageways in SC coupler **2665** and/or SC sheath **2665.1** may allow the SC shaft **2662** to pass therethrough. In some contemplated embodiments, SC coupler **2665** may be formed from and/or be an integral part of modular TD shaft **2602;** in some of these embodiments, SC coupler **2665** may comprise the same insulating materials of modular TD shaft **2602.** In other contemplated embodiments, SC coupler **2665** may be formed from and/or be an integral part of SC shaft **2662;** in some of these embodiments, SC coupler **2665** may comprise the same insulating materials of SC shaft **2662.** Some contemplated embodiments may lack SC sheath **2665.1.**

Coupler **2665** may be configured to allow SC shaft **2662** to be advanced or retracted axially therethrough. The SC depicted in these figures may be coupled with electrosurgical 'pencil' shaft **2602.1** by way of modular TD plug **2603.**

Means for selectively moving a SC (Spot Coagulator Moving Means = SCMM) **2681.1** (an example of such SCMM may include rails, grooves, tracks, ratchets, cables, arms, lines, etc.) may be affixed to 'pencil' shaft **2602.1.** SCMM **2681.1** may be affixed to 'pencil' shaft **2602.1** by means for rigidly affixing SCMM (MFRA-SCMM) **2681;** an example of such a means may include adhesive backed tapes, sheaths, films, adhesive backed foam pads, adhesive backed pads, hook & loop fastening systems, and the like. In the depicted embodiment, SC slidable toggle **2682** is affixed to SC shaft **2662** and moves with the distal (opposite of tip) end of the SC shaft between two rails **2681.1.** Moving SC slidable toggle **2682** toward the modular TD tip **2601** within the SCMM may allow SC tip **2661** to be advanced axially to an operational position and retracted axially to a storage position. SC tip **2661** may retract into SC sheath **2665.1** via SC opening **2665.4** when SC slidable toggle **2682** is moved rearward. Passage of SC shaft through SC opening **2665.4** may be facilitated by such insulations as polytetrafluoroethylene. In some embodiments, the edges of SC opening **2665.4** may be serrated and/or irregularly edged and/or possess bristles and/or other texture that may facilitate dislodgement of electrocoagulation char and/or carbon to clean SC tip **2661.** SC slidable toggle **2682** may comprise a grip, which may in some embodiments comprise a plurality of ridges, to facilitate advancement and retraction of SC shaft **2662.** Some embodiments may further comprise a SC sheath **2665.1** wherein at least a portion of SC may be positioned in the storage position. In some embodiments SC sheath may extend from modular TD tip **2601.** In other embodiments SC sheath **2665.1** may extend from SC coupler **2665.** SC sheath may comprise an insulating material and may be configured to completely cover SC tip **2661** in the storage position. SC sheath **2665.1** may, in some embodiments, comprise a transparent material to allow a surgeon to visualize the position of SC tip **2661** within the sheath. Some embodiments may be further configured to clean SC tip **2661** upon withdrawal into SC sheath **2665.1** and/or upon protrusion through SC sheath **2665.1.** In some contemplated embodiments SC coupler **2665** and/or SC sheath **2665.1** may be absent. MFRA-SCMM **2681** may be configured to accommodate 'pencil' shafts of varying cross sectional geometries and/or diameters and/or lengths. In the depicted embodiment, SC slidable toggle **2682** is attached to the screw-threaded proximal end of conductive metal core of SC shaft **2662;** insulation may be removed in the threaded area of the SC shaft to improve the fit. In other contemplated embodiments, MFRA-SCMM **2681** may be attached to SC shaft **2662** by way of for example form fitting, snap fitting, an adhesive, & the like.

SC **2600** is electrically coupled with modular TD by SC electrical contact **2603.9** which may comprise conductive metal button. In contemplated embodiments SC electrical contact may comprise a conductive spring and/or a terminus of a wire and/or terminus of a conductive strip and the like. SC electrical contact **2603.9** may be coupled to SC electrical conduit **2668** which may be permanently affixed or releasably affixed to modular TD plug **2603** and/or shaft **2602.** SC electrical conduit **2668** may be configured to accommodate pulling forces and/or movement of SC as it is placed on the electrosurgical pencil. For example, the depicted embodiment includes protective insulator **2668.1** which may be a plastic or other nonconductive film and/or coating that may fully wrap around the conductor & prevent the conductive strip within from being torn, for example, while the modular TDM and/or SC is being applied into the electrosurgical pencil.

An implementation using the depicted embodiment may involve the surgeon pushing distally SC slidable toggle **2682** which forces SC shaft **2662** distally through SC coupler **1265.** At a distal point on the movement of SC slidable toggle **2682,** whereupon SC shaft conductive opening **2662.6** is brought into direct contact with SC electrical contact **2603.9** which itself is in direct or indirect contact with the SC electrical conduit **2668** which itself is in direct or indirect contact with the conductive portion of modular TD shaft **2603.7.** Thus electrosurgical energy such as a suitable waveform will be delivered, when the electrosurgical generator is activated, via modular TD plug **2603,** into modular TD shaft **2602,** into SC shaft **2662** and thereupon to SC tip **2661** and then into target tissue. In the depicted embodiment, the SC tip **2661** extends from the SC shaft **2662** and is conductive and not insulated along at least a portion of the tip. In some embodiments, the entire SC tip **2661** is conductive. In the depicted embodiment SC shaft **2662** may comprise stainless steel and may be round in cross-section. In the depicted embodiment, SC shaft **2662** may comprise an electrically insulating and/or supportive coating and/or cover overlying a conductive material and/or metal axial core **2603.7.** In some embodiments, the conductive material may comprise: steel, nickel, alloys, palladium, gold, tungsten, silver, copper, platinum and/or any other conductive metal that does not give off toxic residua at operating temperatures. In other contemplated embodiments, the conductive material may comprise cermets and the like. In some embodiments, the electrical insulator may comprise, for example, porcelain, ceramics, glass-ceramics, plastics, various halogenated carbon molecules, polytetrafluoroethylene, carbon, graphite, and graphite-fiberglass composites and the like. In alternative embodiments the electrical insulator may comprise polyether etherketone and/or polysulfone and/or another electrically nonconductive polymers (with thermal stability in the operating range) and/or materials that are both electrically non-conductive and of low thermal conductivity. In the depicted embodiment, SC tip **2661** is shaped like the frustum of a cone. In some embodiments, SC shaft **2662** may be oval, flat, rectangular or geometric in cross-section or substantially flattened. In alternative embodiments, SC tip **2661** may be pointed, bullet shaped, or geometric in cross section; more angulate and/or pointed tips may disperse electrical energy more readily and allow greater precision than larger, more rounded tip designs. In the depicted embodiments of the SC shaft **2662,** the electrical insulator may comprise polytetrafluoroethylene.

Although the depicted embodiment shows a manually deployed SC, other contemplated embodiments may allow deployment to be (including but not limited to): motorized and/or spring activated and/or screw driven and/or ratchet style and/or cog style and/or pneumatic and/or hydraulic, etc. Although, the embodiment depicted in Fig. 26a shows the SC shaft 2662 pointing about axially (relative to the modular TD axis) and/or passing about over the middle of the front of the modular TD tip **2601,** in other contemplated embodiments, SC shaft 2662 may point at an angle within about 30 degrees of the axis of the modular TD and/or may pass over any other points on the modular TD tip **2601** besides the middle front of the modular TD tip. In other contemplated embodiments, SC shaft **2662** may point at angles greater than 30 degrees of the axis of the modular TD. In some embodiments, SC shaft **2662** may be flexible and its forward axis redirected upon passing through SC coupler **2665;** in some embodiments SC coupler may pivot and/or rotate and/or have one or more non-axial passageways for SC shaft **2662** that may redirect the axis and/or pointing of the forward part of SC shaft **1262.** For example, some embodiments may comprise a plurality of passageways for SC shaft **2662** each pointing in a different direction. In such embodiments one or more such passageways may be axial and one or more may be non-axial. In other contemplated embodiments, SC coupler **2665** and/or SC sheath **2665.1** may have one or more pathways that may lead to SC shaft exiting at a similar range of angles from the axis of the modular TD.

In other contemplated embodiments, a variety of geometrically shaped lysing elements and/or protrusions may co-exist side by side. For example, in some embodiments, two adjacent protrusions may differ in shape and/or size, 2 adjacent lysing elements may differ in length and/or shape and/or TD tip **2601** may otherwise be nonsymmetrical in one or more views. In the depicted embodiment, the SC tip **2661** and SC shaft **2662** together may measure about, 55mm in length and about 3mm in maximal thickness; the SCMM rails may measure 5mm wide & 30mm long; MFRA-SCMM may measure 30x20mm; the SC toggle **2682** may be capable of moving about 80% of the rail length; the overall SC **2660** may measure about 75mm in length. In embodiments wherein an electrical insulation and/or polymeric insulating coating may be present on such parts, for example on SC shaft **2662 s**uch insulation may measure about 0.5mm in thickness. In some embodiments, the insulation thickness may range from about 0.1mm to 3mm. Embodiments are contemplated wherein sizes of about one-fifth to about five times these dimensions may have possible uses. It is also contemplated in some veterinary embodiments; tip sizes of about one-tenth to 20 times the aforementioned dimensions may also have possible uses.

FIG 27 depicts an embodiment in which the SC **2700** may be an integral part of the modular TD. In the depicted embodiment, SC **2700** comprises: Spot Coagulator Moving Means (SCMM) **2781.1** which is rigidly affixed to TD shaft **2702,** SC shaft **2762,** SC tip **2761** and/or SC coupler **2765.** SC moving means **2781.1** may further comprise electric lead **2768** which may be further electrically coupled to modular TD shaft conductive axial core **2703.7.** SCMM **2781.1** may be configured to encase electric lead **2768** which may extend to SC electrical contact **2703.9.** Electrical contact **2703.9** may be configured to electrically couple with a corresponding electrical contact **2762.6** or conductive element, which may comprise a bare portion of an insulator of an electrical contact. Electrical contact **2762.6** may be positioned on or within SC shaft **2762.**

Some embodiments may be configured to extend in a shape that at least substantially matches a distal end of an existing surgical tool such as Bovie electrosurgical 'pencil'. Some embodiments may further be configured such that **2781.1** is malleable or flexible so as to allow a surgeon to conform the SC to the electrosurgical tool.

FIG. 27 depicts an embodiment that differs from the embodiment depicted in FIGs. 26a,b in that the embodiment of FIG 27 comprises: a Spot Coagulator Moving Means (SCMM) **2781.1** which is rigidly affixed to TD shaft **2702.** FIG. 27 depicts an embodiment that also differs from the embodiment depicted in FIGs. 26a,b in that the embodiment of FIG 27 lacks: means for rigidly affixing SCMM **2681** to modular TD shaft **2702.1** or modular TD handle. In some contemplated embodiments, an adhesive pad placed below SCMM may affix the SCMM to the modular TD handle with minimal rigidity. In other contemplated embodiments, the adhesive pad placed between the SCMM & modular TD handle affixes the two with strong rigidity.

Each of the other elements depicted in FIGs. 27 may be identical to the corresponding elements shown in FIGs. 26a,b and are referenced by like numerals (numbers higher by 100). For example, FIG. 27 depicts an embodiment comprising an axially advanceable SC **2700.** SC further comprises SC shaft **2762,** SC tip **2761** and/or SC coupler **2765.** In the depicted embodiment, the Modular TD comprises a Modular TD tip **2701,** a Modular TD shaft **2702,** a Modular TD plug **2703.** The modular TD **2700** depicted in this embodiment has non-axial protrusions & axial protrusions **2704.** The SC shaft may be coupled with modular TD by way of SC coupler **2765.** Modular TD **2700** is modular in that it is removable from an electrosurgical 'pencil' shaft **2702.1** and/or an electrosurgical 'pencil' handle. Rocker switch **2703.2** may control the electrosurgical energy such as a suitable waveform derived from an electrosurgical generator (not seen in this view) brought into the handle via conduit **2711.2.** In the depicted embodiment, modular TDM tip plug **2703** is configured to be received within a corresponding electrosurgical 'pencil' shaft recess **2769** formed within electrosurgical 'pencil' shaft **2702.1.**

So as not to have the surgeon set down the TD or TDM, to pick up other instrumentation from the surgical tray to stem bleeding blood vessel(s), it may be beneficial during some surgical procedures to have spot coagulator coagulation capabilities within the same instrument.

In the depicted embodiment SC shaft **2762** is slidably coupled to the modular TD shaft **2702** by SC coupler **2765.** SC coupler **2765** is one example of a means for moveably coupling a SC with a surgical tool. SC coupler **2765** may be configured to allow SC shaft **2762** to slide relative to SC coupler **2765** and modular TD shaft **2702.**

In the depicted embodiment, means for selectively moving a SC (Spot Coagulator Moving Means = SCMM) **2781.1** (an example of such SCMM may include rails, grooves, tracks, ratchets, cables, arms, lines, etc.) is affixed to modular TD shaft **2702.** In the depicted embodiment, SC slidable toggle **2782** is affixed to SC shaft **2762** and moves with the distal (opposite of tip) end of the SC shaft between two rails **2781.1.** Moving SC slidable toggle **2782** toward the modular TD tip **2701** within the SCMM may allow SC tip **2761** to be advanced axially to an operational position and retracted axially to a storage position. SC tip **2761** may retract into SC sheath **2765.1** via SC opening **2765.4** when SC slidable toggle **2782** is moved rearward. In some contemplated embodiments SC coupler **2765** and/or SC sheath **2765.1** may be absent.

SC **2700** is electrically coupled with modular TD by SC electrical contact **2703.9** which may comprise conductive metal button. In contemplated embodiments SC electrical contact may comprise a conductive spring and/or a terminus of a wire and/or terminus of a conductive strip and the like. SC electrical contact **2703.9** may be coupled to SC electrical conduit **2768** which may be permanently encased inside a SCMM **2781.1** and may be affixed or releasably affixed to modular TD plug **2703** and/or shaft **2702.** In the depicted embodiment, SCMM **2781.1** comprises a polypropylene. In other embodiments, SCMM may comprise a nonconductive polymer and/or other nonconductive plastic and/or nonconductive material and/or malleable metallic pieces (which may provide 'flex' & 'memory' to the SCMM. In some embodiments, the SCMM may be styled to some degree look a bit like a fencing foil guard. In the depicted embodiment, SC shaft **2762** may comprise an electrically insulating and/or supportive coating and/or cover overlying a conductive material and/or metal axial core (shown in phantom) **2703.7.**

Although the depicted embodiment shows a manually deployed SC, other contemplated embodiments may allow deployment to be (including but not limited to): motorized and/or spring activated and/or screw driven and/or ratchet style and/or cog style and/or pneumatic and/or hydraulic, etc. Although, the embodiment depicted in Fig. 27 shows the SC shaft 2762 pointing about axially (relative to the modular TD axis) and/or passing about over the middle of the front of the modular TD tip **2701,** in other contemplated embodiments, SC shaft **2662** may point at an angle within about 30 degrees of the axis of the modular TD and/or may pass over any other points on the modular TD tip **2701** besides the middle front of the modular TD tip. In other contemplated embodiments, SC shaft **2762** may point at angles greater than 30 degrees of the axis of the modular TD. In some embodiments, SC shaft **2762** may be flexible and its forward axis redirected upon passing through SC coupler **2765;** in some embodiments SC coupler may pivot and/or rotate and/or have one or more non-axial passageways for SC shaft **2762** that may redirect the axis and/or pointing of the forward part of SC shaft **2762.** For example, some embodiments may comprise a plurality of passageways for SC shaft **2762** each pointing in a different direction. In such embodiments one or more such passageways may be axial and one or more may be non-axial. In other contemplated embodiments, SC coupler **2765** and/or SC sheath **2765.1** may have one or more pathways that may lead to SC shaft exiting at a similar range of angles from the axis of the modular TD.

In other contemplated embodiments, a variety of geometrically shaped lysing elements and/or protrusions may co-exist side by side. For example, in some embodiments, two adjacent protrusions may differ in shape and/or size, 2 adjacent lysing elements may differ in length and/or shape and/or TD tip **2701** may otherwise be nonsymmetrical in one or more views.

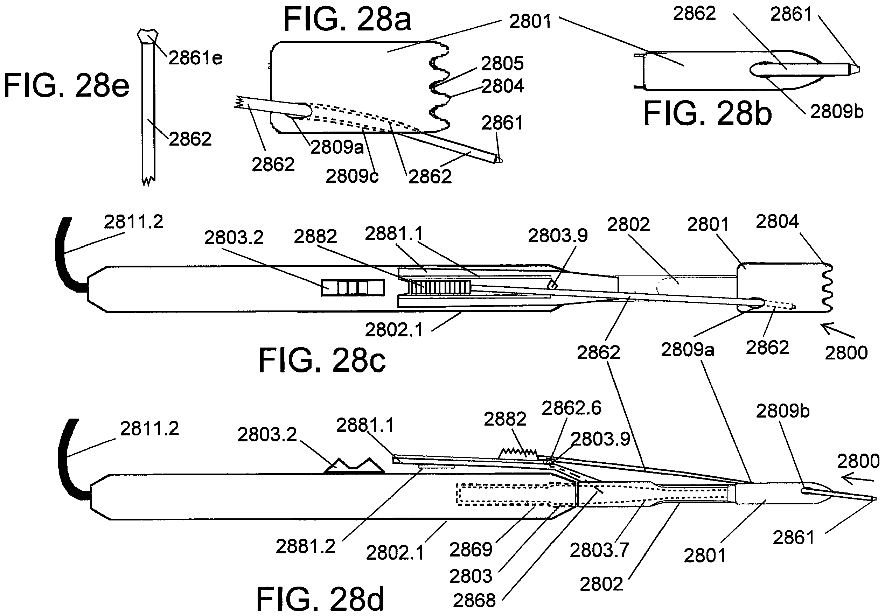

In the depicted embodiment of FIG 27, measurements about those of similar parts in FIGs. 26a,b embodiments are contemplated wherein sizes of about one-fifth to about five times these dimensions may have possible uses. It is also contemplated in some veterinary embodiments; tip sizes of about one-tenth to 20 times the aforementioned dimensions may also have possible uses. As is similar to FIGs. 26, SCMM rails may measure 5mm wide & 30mm long, however, in the embodiment depicted in FIG. 27, in which the SCMM **2781.1** will be similar to that shown in FIGs. 28, the SCMM may be part of a polymeric protrusion extending from the modular TD shaft that passes over the shaft of the electrosurgical 'pencil' **2702.1.** In some embodiments, the SCMM may be shaped slightly resembling the curviform guard of a fencing foil with a width of about 1cm and a length of about 45mm. In some contemplated embodiments an adhesive placed on the bottom of the SCMM and/or between the SCMM and the shaft of the electrosurgical 'pencil' may affix the two parts to some degree.

FIG 28a,b,c,d depict an embodiment in which the SC **2800** may be an integral part of the modular TD. FIGs. 28a,b,c,d depict an embodiment that differs from the embodiment depicted in FIGs. 26a,b in that the embodiment of FIGs. 28a,b,c,d comprises: two modular TD tip openings **2809a** & **2809b,** modular TD tip passageway **2809c** & Spot Coagulator Moving Means (SCMM) **2881.1** which is rigidly affixed to TD shaft **2802.** SC moving means **2881.1** may further comprise electric lead **2868** which may be further electrically coupled to modular TD shaft conductive axial core **2803.7.** SCMM **2881.1** is configured to encase electric lead **2868** which may extend to SC electrical contact **2803.9.** FIGs. 28 depicts an embodiment that also differs from the embodiment depicted in FIGs. 26a,b in that the embodiment of FIGs. 28 lacks: SC coupler **2665,** SC sheath **2665.1,** & means for rigidly affixing SCMM **2681** to modular TD shaft **2702.1** or modular TD handle. However, in the embodiment depicted in FIG. 28d, an adhesive pad **2881.2** is positioned below SCMM, which may be used to affix the SCMM **2881.1** to the electrosurgical 'pencil' shaft and/or handle.

Each of the other elements depicted in FIGs. 28 may be identical to the corresponding elements shown in FIGs. 26a,b and are referenced by like numerals (numbers higher by 200). For example, in the depicted embodiment, SC **2800** comprises: Spot Coagulator Moving Means (SCMM) **2881.1** which is rigidly affixed to TD shaft **2802,** SC shaft **2862,** and/or SC tip **2861.** SC moving means **2881.1** may further comprise electric lead **2868** which may be further electrically coupled to modular TD shaft conductive axial core **2803.7.** SCMM **2881.1** may be configured to encase electric lead **2868** which may extend to SC electrical contact **2803.9.** Some embodiments may be configured to extend in a shape that at least substantially matches a distal end of an existing surgical tool such as Bovie electrosurgical 'pencil'. Some embodiments may further be configured such that **2881.1** may be malleable and/or flexible so as to allow a surgeon to conform the SC to the electrosurgical tool.

In the depicted embodiment, the Modular TD comprises a Modular TD tip **2801,** a Modular TD shaft **2802,** a Modular TD plug **2803.** The modular TD **2800** depicted in this embodiment comprises axial protrusions **2804** and lysing elements **2805.** In other embodiments, the modular TD may comprise axial, non-axial and/or transitional protrusions as discussed elsewhere in this application. Modular TD **2800** is modular in that it is removable from an electrosurgical 'pencil' shaft **2802.1** and/or an electrosurgical 'pencil' handle. Rocker switch **2803.2** may control the electrosurgical energy such as a suitable waveform derived from an electrosurgical generator (not seen in this view) brought into the handle via conduit **2811.2.** In the depicted embodiment, modular TDM tip plug **2803** is configured to be received within a corresponding electrosurgical 'pencil' shaft recess **2869** formed within electrosurgical 'pencil' shaft **2802.1.**

So as not to have the surgeon set down the TD or TDM, to pick up other instrumentation from the surgical tray to stem bleeding blood vessel(s), it may be beneficial during some surgical procedures to have spot coagulator coagulation capabilities within the same instrument.

In the depicted embodiment SC shaft **2862** is slidably coupled to the modular TD tip **2801** by two modular TD tip openings **2809a** & **2809b** & modular TD tip passageway **2809c.** Modular TD tip openings **2809a** & **2809b** & modular TD tip passageway **2809c** may be configured to allow SC shaft **2862** to slide relative to modular TD tip **2801.**

In the depicted embodiment, Means for selectively moving a SC (Spot Coagulator Moving Means = SCMM) **2881.1** (an example of such SCMM may include rails, grooves, tracks, ratchets, cables, arms, lines, etc.) is affixed to modular TD shaft **2802.** In the depicted embodiment, SC slidable toggle **2882** is affixed to SC shaft **2862** and moves with the distal (opposite of tip) end of the SC shaft between two rails **2881.1.** Moving SC slidable toggle **2882** toward the modular TD tip **2801** within the SCMM may allow SC tip **2861** to be advanced axially to an operational position and retracted axially to a storage position. SC tip **2861** may retract into modular TD tip **2801** via modular TD tip opening **2809b** when SC slidable toggle **2882** is moved rearward.

SC **2800** is electrically coupled with modular TD by SC electrical contact **2803.9** which may comprise conductive metal button. In contemplated embodiments SC electrical contact may comprise a conductive spring and/or a terminus of a wire and/or terminus of a conductive strip and the like. SC electrical contact **2803.9** may be coupled to SC electrical conduit **2868** which may be permanently encased inside a SCMM **2881.1** and/or may be affixed or releasably affixed to modular TD plug **2803** and/or shaft **2802. In** the depicted embodiment, SCMM **2881.1** comprises a polypropylene. In other embodiments, SCMM may comprise a nonconductive polymer and/or other nonconductive plastic and/or nonconductive material and/or malleable metallic pieces (which may provide 'flex' & 'memory' to the SCMM. In some embodiments, the SCMM may be styled to some degree look a bit like a fencing foil guard. In the depicted embodiment, SC shaft **2862** may comprise an electrically insulating and/or supportive coating and/or cover overlying a conductive material and/or metal axial core (shown in phantom) **2803.7.**

Although the depicted embodiment shows a manually deployed SC, other contemplated embodiments may allow deployment to be (including but not limited to): motorized and/or spring activated and/or screw driven and/or ratchet style and/or cog style and/or pneumatic and/or hydraulic, etc. Although, the embodiment depicted in Figs. 28 shows the SC shaft **2862** pointing in a non-axial angle (relative to the modular TD axis), in other contemplated embodiments, SC shaft **2862** may point at an angle within about 45 degrees of the long axis of the modular TD shaft. In other contemplated embodiments, SC shaft **2862** may point at angles greater than 45 degrees of the long axis of the modular TD. In some embodiments, SC shaft **2862** may be flexible and may be able to be redirected upon passing through TD tip **2801.** In some embodiments, modular TD tip may comprise one or more passageways that may SC shaft **2862** that may redirect the axis and/or pointing of the forward part of SC shaft **2862.** For example, some embodiments may comprise a plurality of passageways for SC shaft **2862** each pointing in a different direction. In such embodiments one or more such passageways may be axial and one or more may be non-axial.

Modular TD tip openings **2809a** & **2809b,** are formed on modular TD tip **2801** such that passageway **2809c** extends therebetween.

The said passageway **2809c** may be watertight to prevent ionic fluids from reaching other electrical components within the TD tip. In the depicted embodiment, SC tip **2861** may only able to conduct electrosurgical energy delivered by the 'pencil' when the toggle **2882** has engaged the aforementioned contacts **2862.6** & **2803.9.** In further contemplated embodiments, SC tip **2861** may conduct electrosurgical energy when toggle or SC tip is a range of positions including but not limited to 'not deployed.' In contemplated embodiments, electrical contacts may comprise any known in the art.

In contemplated embodiments, SC shaft **2862** may emanate axially from modular TD tip **2801.** In some embodiments, a thicker modular TD tip may comprise modular TD tip openings & an axially directed modular TD tip passageway emanating above and/or below lysing elements and/or protrusions. In other contemplated embodiments, a thicker modular TD tip may comprise modular TD tip openings & a non-axially directed modular TD tip passageway. In other contemplated embodiments, SC tip **2861** may emanate within 15 degrees of axially from modular TD tip **2801.**

In contemplated embodiments, SC shaft **2862** may emanate axially from modular TD tip **2801** wherein frontal modular TD tip opening **2809b,** &/or passageway **2809c** may emanate from an axial relative recession & thus adjacent one or more axial protrusions. The SC tip may be positioned at and/or about the orifice of TD tip opening **2809b** so that SC tip **2861** may apply the selected electrosurgical waveform for cutting and/or 'coag' and/or blend settings (to perform as if it were a shaped lysing segment, for example concave, convex, beveled and/or straight as described elsewhere in this application) and/or if deployed (extended) for spot coagulation, SC tip **2861** may perform spot coagulation. In another contemplated embodiment, as depicted in FIG. 28e, the forward (distal) aspects of an SC tip **2861e** (mounted on a SC shaft **2862**) may be modified and/or fashioned to resemble and/or function as a lysing element. Thus, when such a lysing-element-like-SC tip **2861e** may be positioned at and/or about the orifice of TD tip opening **2809b** (wherein frontal modular TD tip opening **2809b,** &/or passageway **2809c** may emanate from the base/proximal location of a relative recession) the lysing-element-like-SC tip **2861e** may benefit from physical characteristics it may share with various lysing element shapes described in this application. Lysing element design has been described in detail elsewhere in this application. In another contemplated embodiment, as depict in Fig 28e, the lysing-element-like-SC tip **2861e** may in a storage position comprise one of the lysing elements positioned within a recession of TD tip **2801.** In such embodiments, lysing-element-like-SC tip **2861e** may be configured to extend out of its corresponding recession as SC **2862** extends forward to a Spot Coagulating operational position. When the lysing-element-like-SC tip **2861e** is in a storage position (resting between protrusions in a relative recession similar to its counterpart lysing element **2805**) the lysing-element-like-SC tip **2861e** may be 'operational' for hemostatic tissue dissection purposes as opposed to spot coagulation purposes.

In other contemplated embodiments, a variety of geometrically shaped lysing elements and/or protrusions may co-exist side by side. For example, in some embodiments, two adjacent protrusions may differ in shape and/or size, 2 adjacent lysing elements may differ in length and/or shape and/or TD tip **2801** may otherwise be nonsymmetrical in one or more views.

In the depicted embodiment of FIG 28, measurements about those of similar parts in FIGs. 28a,b, embodiments are contemplated wherein sizes of about one-fifth to about five times these dimensions may have possible uses. It is also contemplated in some veterinary embodiments; tip sizes of about one-tenth to 20 times the aforementioned dimensions may also have possible uses. As is similar to FIGs. 26 &/or 27, SCMM rails may measure 5mm wide & 30mm long, however, in the embodiment depicted in FIG. 28, in which the SCMM **2881.1** will be similar to that shown in FIGs. 27, the SCMM may be part of a polymeric protrusion extending from the modular TD shaft that passes over the shaft of the electrosurgical 'pencil' **2802.1.** In some embodiments, the SCMM may be shaped slightly resembling the curviform guard of a fencing foil with a width of about 1cm and a length of about 45mm. In some contemplated embodiments an adhesive placed on the bottom of the SCMM and/or between the SCMM and the shaft of the electrosurgical 'pencil' may affix the two parts to some degree.

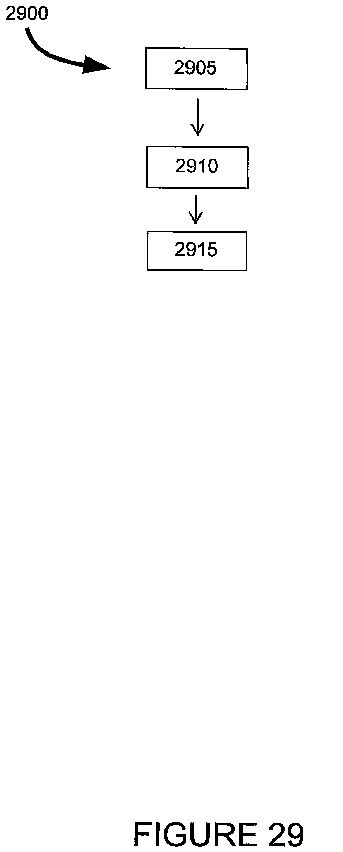

FIG. 29 (was 24) illustrates an implementation of a method **2900** according to this disclosure for making an electrosurgical incision using the lysing elements of a TD in tissue and/or tissue plane with the assistance of a TD. In some implementations, surgeon(s) may need to incise layers of tissues in order to access tissue and/or an organ to repair or treat it. In some implementations, the skin surrounding the anticipated entrance wound for the surgical area may be cleansed by, for example, with isopropyl alcohol (degreaser) followed by germicidal chlorhexidine scrub. Then, a local anesthetic may be applied (such as by injecting) 1% lidocaine + 1:100,000adrenaline to the skin.

Step **2905** may comprise the initial skin opening or body cavity opening steps of such a procedure. Step **2905** may comprise forming an incision. In some implementations, step **2905** may comprise making the incision using the same surgical tool used to perform every other step in method **2900.** In other implementations step **2905** may be performed in part with the use of another surgical instrument such as the scalpel. For example, step **2905** may be initially performed with, for example, a #15 Bard-Parker™ Scalpel to a length of about 1cm into the target tissue to be incised for example the epidermis and dermis. In some implementations, step **2905** may comprise making the initial skin incision using a standard a sharp surgical instrument such as for example, a scalpel and/or sharp scissors. Step **2910** may further comprise placing the tip of the TD tip in an orientation substantially perpendicular to the tissue plane to be cut with at least one protrusion lying on opposite sides of the plane of tissue to be incised and extending the initial incision using the TD tip. In some implementations, step **2910** may comprise using the TD tip in an unzipping fashion to completely separate the planar tissue into two separate surfaces. Step **2910** may further comprise energizing the lysing elements with electrosurgical energy. Step **2910** may further comprise energizing the lysing elements with electrosurgical cutting and/or coagulation and/or 'blended' waveforms. Step **2910** may further comprise pushing and/or moving forward the TD and its attendant lysing elements so as to cut the intended tissue layer and/or plane. Step **2915** may comprise positioning the tip of the TD in an orientation substantially perpendicular to a second tissue which may be adjacent to the first tissue plane referenced in step **2910** with at least one protrusion lying on opposite sides of the second tissue plane and using the lysing elements of the TD tip to cut the second tissue plane into separate surfaces. A variety of other steps may be performed before during or after the steps depicted in FIG. 29 as would be apparent to one of ordinary skill in the art. In some implementations, the tissues traversed may require closure by suturing and/or stapling and/or tissue glue and/or taping and/or binding. In some implementations, organs and/or organ systems that the TD may be useful to cut through include the surface skin and/or fascial layers and/or muscular layers and/or protecting layers (for example peritoneum) to access organs and organ systems that may include but need not be limited to muscle, and/or parotid, and/or salivary gland, and/or thyroid, and/or lung, and/or heart, and/or gastrointestinal, and/or liver, and/or pancreas, and/or spleen, and/or gallbladder, and/or kidney, and/or adrenal, and/or prostate, and/or ovary, and/or uterus, and/or bladder, and/or vascular, and/or lymph nodes and/or skeleton, and/or lung.

Any of the embodiments of TDM and/or TD discussed herein including, but not limited to, the embodiments discussed with Figs 1-30 etc. may be used in conjunction with one or more of the steps disclosed in connection with Figure 29.

In a more general implementation of a method according to this disclosure for dissection and modification of tissues, a first step may comprise creating an incision into a patient's skin.

A second step may comprise inserting a Tissue Dissecting and Modifying Wand into the incision and positioning the Tissue Dissecting and Modifying Wand within the body. The Tissue Dissecting and Modifying Wand may comprise a tip having a plurality of protrusions with lysing elements positioned between the protrusions. The Tissue Dissecting and Modifying Wand may also comprise an energy window positioned on top of the Tissue Dissecting and Modifying Wand that is configured to deliver energy to modify tissues.

A third step may comprise fanning out the Tissue Dissecting and Modifying Wand to define a target region within which to dissect and modify tissues. This step may comprise separating tissue using the lysing element(s) to define the target region. During this step, in some implementations, the patient's target tissue may be placed under tension by stretching/tightening the skin at the target region during the fanning/tissue separation.

A fourth step may comprise activating the energy window and moving the energy window around within the target region for hemostasis and/or to induce postoperative fibrosis. Alternatively, the energy window may be activated prior to the third step such that the step of fanning out the Tissue Dissecting and Modifying Wand to define the target region also comprises heating tissues to induce fibrosis and/or hemostasis within the target region.

In another embodiment of a method for separating and modifying tissue using a tissue dissecting and modifying wand, the method may comprise creating an incision into a patient's skin. A tissue dissecting and modifying wand may be inserted into the incision. The tissue dissecting and modifying wand may comprise: a tip comprising a plurality of protrusions; at least one lysing element positioned between at least two adjacent protrusions among the plurality of protrusions; and an energy window configured to deliver energy to tissue adjacent to the tissue dissecting and modifying wand during a procedure. The energy window may comprise an electromagnetic emission energy window, and wherein the energy window is positioned and configured to deliver electromagnetic energy from the tissue dissecting and modifying wand to tissue adjacent to the tissue dissecting and modifying wand during a procedure.

The tissue dissecting and modifying wand may further comprise a ITOMSensor positioned on the tissue dissecting and modifying wand and configured to provide speed data (for example, the difference in speed of adjacent tissue versus a tissue dissecting and modifying wand tip during a procedure. In such implementations, data may be received from the tissue dissecting and modifying wand generated from the ITOMSensor, wherein the data allows a user to determine or control the energy deposited per unit of tissue surface within a patient's body during treatment using energy from an electromagnetic delivery energy window. In some implementations, the energy deposited per unit of tissue surface area may be derived from distance travelled by the TDM relative to the tissue surface. In other implementations, the speed data may be used by a related processor and/or accompanying software to automatically modify the energy delivered by the TDM.

In another implementation of a method for separating and modifying tissue using a tissue dissecting and modifying wand, the tissue dissecting and modifying wand may comprise a tip comprising a first plurality of protrusions and a second plurality of protrusions, wherein the first plurality of protrusions is positioned to at least substantially extend in a first direction, and wherein the second plurality of protrusions is positioned to at least substantially extend in a second direction distinct from the first direction; at least one lysing element positioned between at least two adjacent protrusions in the first plurality of protrusions; at least one lysing element positioned between at least two adjacent protrusions in the second plurality of protrusions; and a ITOMSensor positioned on the tissue dissecting and modifying wand and configured to provide speed of the tissue dissecting and modifying wand during a procedure. In such implementations, the method may comprise a step of receiving data from the tissue dissecting and modifying wand generated from the ITOMSensor, wherein the data allows a user to determine speed (for example, the difference in speed of adjacent tissue versus a tissue dissecting and modifying wand tip during a procedure) . In this manner, the ITOMSensor may allow a user to determine &/or control the energy deposited per unit of tissue surface within a patient's body during treatment using energy from an electromagnetic delivery energy window.. In other implementations, such as implementations involving use of a TD lacking an energy window, the ITOMSensor may allow the user to determine and/or control the rate of energy delivery from the lysing elements.

An example of an embodiment of an apparatus according to this disclosure for tissue dissection and modification may comprise:
a handle;
a tip comprising a plurality of protrusions having one or more lysing elements positioned between the protrusions; and
an energy window positioned on an upper side of the apparatus, wherein the energy window may comprise an electromagnetic emission energy window, and wherein the energy window is positioned and configured to deliver electromagnetic energy from the tissue dissecting and modifying wand to tissue adjacent to the tissue dissecting and modifying wand during a procedure.

In some embodiments, as described above, the electromagnetic emission energy window may comprise fiberoptics &/or mirrors configured to deliver LASER energy from the energy window.

In alternative embodiments, as described above, the electromagnetic emission energy window may comprise radiofrequency/electrosurgical energy delivering elements.

An example of an embodiment of an apparatus according to this disclosure for tissue dissection and modification may comprise:
a handle;
a tip comprising a plurality of protrusions having one or more lysing elements positioned between the protrusions; and
an electromagnetic emission energy window positioned on an upper side of the apparatus, wherein the electromagnetic emission energy window may comprise fiberoptics &/or mirrors configured to deliver LASER energy from the energy window.

In alternative embodiments, as described above, the electromagnetic emission energy window may comprise radiofrequency/electrosurgical energy delivering elements.

FIGs. 30a-c depict an embodiment of a surgical device comprising a TDM **3000.** The TDM comprises a conductive element **3003.9** protruding from an electroconductive metallic shaft 3003. Conductive element **3003.9** is shaped like a fin, and may, as previously discussed, be configured to be selectively electrically coupled with another conductive element or elements in one or more operational positions. For example, in some embodiments, a spot coagulator may be movable relative to shaft **3003** between one or more storage positions and one or more operational positions. In the one or more operational positions, conductive element **3003.9** may be configured to contact a corresponding conductive element on the spot coagulator. Similarly, in the one or more storage positions, conductive element **3003.9** may be electrically isolated from the corresponding conductive element or elements such that the spot coagulator tip is not able to deliver coagulating electrical energy..

The TDM **3000** depicted in FIGS. 30a-30c further comprises a bovie electrosurgical pencil shaft **3002.1** and a spot coagulator comprising a spot coagulator shaft **3062** and a spot coagulator tip **3061.** The spot coagulator is coupled to the TDM **3000** by way of SC coupler **3065.1.** In the depicted embodiment, SC coupler **3065.1** comprises an integral extension of the surgical device tip **3001,** which, as previously discussed, may comprise a plurality of protrusions 3004 and at least one lysing element **3005** positioned between each adjacent protrusion 3004. In some embodiments, SC coupler **3065.1** may also comprise a sheath configured to receive tip **3061** in a storage position.

Shaft **3003** may comprise a plug at a proximal end configured to be positioned in a distal end of a recess of a surgical tool, such as the Bovie pencil shaft **3002.1** depicted in the figures.

The embodiment depicted in FIGS. 30a-30c further comprises SC opening **3065.4,** TD shaft conductive core **3003.7,** and SC coupler conductive element **3003.9.** TD shaft conductive core **3003.7** may form a lysing element plate **3005** at its distal end, which may be used to form a distal tip comprising the lysing elements of the TDM **3000** SC coupler conductive element **3003.9** comprises a protruding electrically-conductive element. More particularly, SC coupler conductive element **3003.9** comprises a corresponding contact that may be used to facilitate desired contact with a portion of the SC, such as an SC shaft projecting contact (hidden from view). SC tip **3061** may pass into and/or out of SC opening **3065.4.**

Each of the other elements depicted in FIGs. 30a-c may be similar to corresponding elements shown in FIGs. 13a-b and are referenced by like numerals (numbers higher by 1700). For example, FIGs. 30a-c depict an embodiment comprising SC shaft **3062**, SC tip **3061,** SC toggle **3081,** and/or SC sheath **3065.1.** In some embodiments, TD **3000** may comprise a modular TD. The modular TD **3000** depicted in this embodiment comprises a modular TD tip **3001,** modular TD shaft conductive core **3003.7,** modular TD plug **3003** and also comprises axial protrusions **3004** and axial lysing elements **3005** but lacks non-axial protrusions and lysing segments. The standard electrosurgical 'pencil' depicted, comprises shaft **3002.1,** and/or 'pencil' switch **3002.2.** SC tip **3061** may retract into SC sheath **3065.1** via SC opening **3065.4.** In some embodiments, SC opening **3065.4** may be formed within an SC gate. SC toggle **3081** may comprise a grip. Although the embodiment depicted in Fig. 30a shows the SC shaft **3062** pointing axially (relative to the modular TD axis) and/or passing about over the middle of the front of the modular TD tip **3001,** in other contemplated embodiments, SC shaft 3062 may point at an angle within about 30 degrees of the axis of the modular TD and/or may pass over any other points on the modular TD tip **3001** besides the middle front of the modular TD tip. In other contemplated embodiments, SC shaft **3062** may point at angles greater than 30 degrees of the axis of the modular TD.

In the depicted embodiment, the SC connecting contact **3003.9** may comprise a conductive piece emanating and/or projecting from the conductive core of modular TD shaft 3003 , for example a 'fin'; such pieces may be manufactured by stamping and/or welding or other methods known in the art. An example of a protruding electrically-conductive element comprising a fin is shown in FIG. 30b. SC connecting contact **3003.9** may be configured to accommodate axial movement of SC shaft **3062** between one or more operational positions and one or more storage positions. For example, some embodiments may comprise a plurality of adjacent conductive projections and/or rings or other non-insulated areas such that SC tip **3061** may be positioned at a plurality of preset distances from modular TD tip **3001.** In the depicted embodiment of FIG. 30a, SC connecting contact **3003.9** (hidden from view) is electrically coupled to the modular TD shaft **3062.** In contemplated embodiments, SC connecting contact **3003.9** may be electrically coupled to the modular TD tip **3001** and/or modular TD plug **3003.** In some embodiments, the electrically-conductive element may be flexible, such as spring-loaded or spring-biased, to further facilitate desired electrical contact.

In some embodiments, SC coupler **3065.1** may comprise at least one passageway to receive SC shaft 3062. In some embodiments, SC coupler **3065.1** may be an integral part of tip 3001, as shown in FIG. 30a and FIG. 30c. In embodiments comprising a shaft **3003** having a conductive core, the conductive core may comprise a lysing element plate **3005,** and the lysing element(s) of tip **3001** may be part of the lysing element plate **3005,** as depicted in FIGS. 30a-c.

In the depicted embodiment, the spot coagulator is configured to move axially with respect to an axis of the TDM **3000** between at least one operational position and at least one storage position. However, other embodiments are contemplated in which the spot coagulator movement need not be axial, either in whole or in part.

It will be understood by those having skill in the art that changes may be made to the details of the above-described embodiments without departing from scope of the invention according to the claims. For example, any suitable combination of various embodiments, or the features thereof, is contemplated.

Any methods disclosed herein comprise one or more steps or actions for performing the described method. The method steps and/or actions may be interchanged with one another. In other words, unless a specific order of steps or actions is required for proper operation of the embodiment, the order and/or use of specific steps and/or actions may be modified.

Throughout this specification, any reference to "one embodiment," "an embodiment," or "the embodiment" means that a particular feature, structure, or characteristic described in connection with that embodiment is included in at least one embodiment. Thus, the quoted phrases, or variations thereof, as recited throughout this specification are not necessarily all referring to the same embodiment.

Similarly, it should be appreciated that in the above description of embodiments, various features are sometimes grouped together in a single embodiment, figure, or description thereof for the purpose of streamlining the disclosure. This method of disclosure, however, is not to be interpreted as reflecting an intention that any claim require more features than those expressly recited in that claim. Rather, inventive aspects lie in a combination of fewer than all features of any single foregoing disclosed embodiment. It will be apparent to those having skill in the art that changes may be made to the details of the above-described embodiments without departing from the scope of the invention as claimed.

Furthermore, the described features, components, structures, steps, or characteristics may be combined in any suitable manner in one or more alternative embodiments and/or implementations. In other words, any of the features, components, structures, steps, or characteristics disclosed in any one disclosed embodiment may be combined with features, components, structures, steps, or characteristics of other disclosed embodiments.

## Claims

1. An apparatus (100) for tissue separation and modification, comprising:
a surgical device comprising:
a main tip (101) comprising a plurality of protrusions (151b);
at least one lysing element (153b) positioned between at least two adjacent protrusions (151b) in the plurality of protrusions (151b);
an elongate main shaft (102) coupled to the main tip (101), the main shaft (102) having a longitudinally extending axis;
wherein the at least two adjacent protrusions (151b) are oriented in a non-axial direction relative to the aforesaid axis of the main shaft (102), **characterised in that** the at least two adjacent protrusions (151b) are spaced from each other along the aforesaid axis of the main shaft (102);
and further **characterised in that** the plurality of protrusions (151b) comprise two sets of non-axial protrusions (151b) extending in directions that are substantially opposite from one another, and wherein the apparatus includes a respective lysing element (153b), between adjacent protrusions (151b) of each set.

2. The apparatus of claim 1, further including a spot coagulator (660) comprising:
a spot coagulator shaft (662); and
a spot coagulator tip (661) configured to deliver energy for coagulating a blood vessel during a surgical procedure with the apparatus for tissue separation and modification, wherein the spot coagulator tip (661) is movable with respect to the plurality of protrusions (151b); and, preferably, the spot coagulator tip (661) is configured to pivot about a base to selectively deliver energy to different regions of the main tip (101).

3. The apparatus of claim 2, further comprising a first conductive element and a second conductive element, wherein the first conductive element is configured to electrically couple to the second conductive element in an operational configuration to allow the spot coagulator tip (661) to deliver energy for coagulating a blood vessel, and wherein the first conductive element is electrically insulated from the second conductive element in a storage configuration to prevent the spot coagulator tip (661) from delivering energy; wherein the spot coagulator tip (661) is preferably axially movable with respect to the main shaft (102) such that the spot coagulator (660) can be advanced to the operational configuration and retracted to the storage configuration.

4. The apparatus of claim 3, wherein the spot coagulator (660) is configured to provide for a plurality of operational positions such that at least one conductive element of the spot coagulator (660) couples with at least one other conductive element such that the spot coagulator (660) can deliver energy for coagulating a blood vessel at any of the plurality of operational positions.

5. The apparatus of claim 4, further comprising a plurality of conductive elements, wherein the second conductive element is one of the plurality of conductive elements, and wherein each of the conductive elements corresponds to one of the plurality of operational positions.

6. The apparatus of claim 4, wherein at least one of the first and second conductive elements comprises an insulator and a plurality of conducting sections, wherein each of the conducting sections comprises an opening (662.6) in the insulator to allow the conducting sections to make contact with another conductive element, and wherein each of the conducting sections corresponds to one of the plurality of operational positions.

7. The apparatus of claim 2, wherein the main shaft (602) is configured to be received in a shaft recess (169) of an electrosurgical device; and
the spot coagulator (660) comprises:
means for selectively moving the spot coagulator (660) relative to the main tip (101), whereby the spot coagulator tip (661) can deliver electrical energy for coagulating the blood vessel during the surgical procedure.

8. The apparatus of claim 7, wherein the means for selectively moving the spot coagulator (1260) comprises a slidable toggle (1281) coupled with the spot coagulator shaft (1262), wherein the slidable toggle (1281) is configured to axially advance the spot coagulator tip (1261) between a retracted position and at least one operational position, preferably between a retracted position and a plurality of discrete operational positions.

9. The apparatus of claim 7, wherein the means for selectively moving the spot coagulator (660) further comprises a first conductive element, wherein the spot coagulator shaft (662) further comprises a second conductive element, wherein the first conductive element is configured to selectively electrically couple with the second conductive element in an operational position, and wherein the first conductive element is configured to selectively electrically decouple from the second conductive element in a storage position.

10. The apparatus of claim 9, further comprising a spot coagulator coupler configured to receive the spot coagulator (660) therethrough, the spot coagulator coupler being preferably an integral part of the main tip (101).

11. The apparatus of claim 2, wherein the surgical device further comprises:
a spot coagulator coupler (1265) comprising at least one passageway; and
a protruding conductive element;
wherein the spot coagulator (1260) is configured to engage the protruding conductive element to receive energy from the surgical device to allow the spot coagulator tip (1261) to deliver electrical energy, and wherein the spot coagulator (1260) extends through the at least one passageway.

12. The surgical apparatus of claim 11, wherein the main shaft (1302) comprises a conductive core (1303.7); and preferably the conductive core (1303.7) comprises a lysing element plate (3005), the at least one lysing element (153b) being part of the lysing element plate (3005).

13. The surgical apparatus of claim 11, further comprising a toggle (1281) coupled with the spot coagulator (1260), wherein the toggle (1281) is configured to move at least a portion of the spot coagulator (1260) with respect to the main tip (1201) between at least one operational position and at least one storage position; and preferably the toggle (1281) is configured to move the at least a portion of the spot coagulator (1260) axially with respect to an axis of the surgical apparatus between the at least one operational position and the at least one storage position.

14. The surgical apparatus of claim 11, wherein the spot coagulator coupler (1265) comprises a sheath, wherein the spot coagulator tip (1261) is configured to be received in the sheath (1265.1) in a storage position, wherein the spot coagulator tip (1261) is configured to extend out of the sheath in an operational position, and wherein the spot coagulator (1260) is configured such that the spot coagulator tip (1261) can receive electrical energy in the operational position and such that the spot coagulator tip (1261) is prevented from receiving electrical energy in the storage position.

## Patentansprüche

1. Vorrichtung (100) zum Sezieren und Modifizieren von Gewebe, umfassend: eine chirurgische Vorrichtung, umfassend:
eine Hauptspitze (101), umfassend eine Vielzahl von Vorsprüngen (151b);
mindestens ein Lyseelement (153b), das zwischen mindestens zwei benachbarten Vorsprüngen (151b) in der Vielzahl von Vorsprüngen (151b) positioniert ist;
ein länglicher Hauptschaft (102), der mit der Hauptspitze (101) gekoppelt ist, wobei der Hauptschaft (102) eine sich in Längsrichtung erstreckende Achse aufweist;
wobei die mindestens zwei benachbarten Vorsprünge (151b) in einer nicht-axialen Richtung relativ zur oben genannten Achse des Hauptschafts (102) ausgerichtet ist, **dadurch gekennzeichnet, dass** die mindestens zwei benachbarten Vorsprünge (151b) entlang der oben genannten Achse des Hauptschafts (102) voneinander beabstandet sind;
und ferner **dadurch gekennzeichnet, dass** die Vielzahl von Vorsprüngen (151b) zwei Sätze nicht-axialer Vorsprünge (151b) umfasst, die sich in Richtungen erstrecken, die einander im Wesentlichen entgegengesetzt sind, und wobei die Vorrichtung ein jeweiliges Lyseelement (153b) zwischen benachbarten Vorsprüngen (151b) jedes Satzes enthält.

2. Vorrichtung nach Anspruch 1, ferner enthaltend einen Punktkoagulator (660), umfassend: einen Punktkoagulatorschaft (662);
und eine Punktkoagulatorspitze (661), die konfiguriert ist, um Energie zum Koagulieren eines Blutgefäßes während eines chirurgischen Verfahrens mit der Vorrichtung zum Sezieren und Modifizieren von Gewebe zu liefern, wobei die Punktkoagulatorspitze (661) bezüglich der Vielzahl von Vorsprüngen (151b) beweglich ist; und wobei die Punktkoagulatorspitze (661) vorzugsweise konfiguriert ist, um eine Basis zu schwenken, um Energie selektiv an unterschiedliche Regionen der Hauptspitze (101) zu liefern.

3. Vorrichtung nach Anspruch 2, ferner umfassend ein erstes leitendes Element und ein zweites leitendes Element, wobei das erste leitende Element konfiguriert ist, um in einer operativen Konfiguration elektrisch mit dem zweiten leitenden Element zu koppeln, um der Punktkoagulatorspitze (661) zu ermöglichen, Energie zum Koagulieren eines Blutgefäßes zu liefern, und wobei das erste leitende Element in einer Lagerkonfiguration vom zweiten elektrischen Element elektrisch isoliert ist, um zu verhindern, dass die Punktkoagulatorspitze (661) Energie liefert; wobei die Punktkoagulatorspitze (661) vorzugsweise bezüglich des Hauptschafts (102) axial beweglich ist, so dass der Punktkoagulator (660) in die operative Konfiguration vorgeschoben und in die Lagerkonfiguration zurückgezogen werden kann.

4. Vorrichtung nach Anspruch 3, wobei der Punktkoagulator (600) konfiguriert ist, um eine Vielzahl operativer Positionen vorzusehen, so dass mindestens ein leitendes Element des Punktkoagulators (660) mit mindestens einem anderen leitenden Element koppelt, so dass der Punktkoagulator (660) Energie zum Koagulieren eines Blutgefäßes an jede der Vielzahl operativer Positionen liefern kann.

5. Vorrichtung nach Anspruch 4, ferner umfassend eine Vielzahl leitender Elemente, wobei das zweite leitende Element eines der Vielzahl leitender Elemente ist, und wobei jedes der leitenden Elemente einer der Vielzahl operativer Positionen entspricht.

6. Vorrichtung nach Anspruch 4, wobei mindestens eines des ersten und zweiten leitenden Elements einen Isolator und eine Vielzahl leitender Abschnitte umfasst, wobei jeder der leitenden Abschnitte eine Öffnung (662.6) im Isolator umfasst, um den leitenden Abschnitten zu gestatten, Kontakt mit einem anderen leitenden Element herzustellen, und wobei jeder der leitenden Abschnitte einer der Vielzahl operativer Positionen entspricht.

7. Vorrichtung nach Anspruch 2, wobei der Hauptschaft (602) konfiguriert ist, um in einer Schaftaussparung (169) eines elektrochirurgischen Geräts aufgenommen zu werden;
und der Punktkoagulator (660) umfasst:
Mittel zum selektiven Bewegen des Punktkoagulators (660) relativ zur Hauptspitze (101), wodurch die Punktkoagulatorspitze (661) elektrische Energie zum Koagulieren des Blutgefäßes während des chirurgischen Verfahrens liefern kann.

8. Vorrichtung nach Anspruch 7, wobei die Mittel zum selektiven Bewegen des Punktkoagulators (1260) einen verschiebbaren Schalter (1281) umfassen, der mit dem Punktkoagulatorschaft (1262) gekoppelt ist, wobei der verschiebbare Schalter (1281) konfiguriert ist, um die Punktkoagulatorspitze (1261) zwischen einer zurückgezogenen Position und mindestens einer operativen Position axial vorzuschieben, vorzugsweise zwischen einer zurückgezogenen Position und einer Vielzahl getrennter operativer Positionen.

9. Vorrichtung nach Anspruch 7, wobei die Mittel zum selektiven Bewegen des Punktkoagulators (660) ferner ein erstes leitendes Element umfassen, wobei der Punktkoagulatorschaft (662) ferner ein zweites leitendes Element umfasst, wobei das erste leitende Element konfiguriert ist, um in einer operativen Position selektiv mit dem zweiten leitenden Element elektrisch zu koppeln, und wobei das erste leitende Element konfiguriert ist, um in einer Lagerposition selektiv vom zweiten leitenden Element elektrisch zu entkoppeln.

10. Vorrichtung nach Anspruch 9, ferner umfassend einen Punktkoagulatorkoppler, der konfiguriert ist, den Punktkoagulator (660) dadurch aufzunehmen, wobei der Punktkoagulatorkoppler vorzugsweise ein integraler Teil der Hauptspitze (101) ist.

11. Vorrichtung nach Anspruch 2, wobei die chirurgische Vorrichtung ferner umfasst:
einen Punktkoagulatorkoppler (1265), umfassend mindestens einen Durchlass;
und ein vorstehendes leitendes Element;
wobei der Punktkoagulator (1260) konfiguriert ist, um das vorstehende leitende Element in Eingriff zu nehmen, um Energie von dem chirurgischen Gerät zu empfangen, um es der Punktkoagulatorspitze (1261) zu ermöglichen, elektrische Energie zu liefern, und wobei sich der Punktkoagulator (1260) durch den mindestens einen Durchlass erstreckt.

12. Chirurgische Vorrichtung nach Anspruch 11, wobei der Hauptschaft (1302) einen leitenden Kern (1303.7) umfasst; und vorzugsweise der leitende Kern (1303.7) eine Lyseelementplatte (3005) umfasst, wobei das mindestens eine Lyseelement (153b) Teil der Lyseelementplatte (3005) ist.

13. Chirurgische Vorrichtung nach Anspruch 11, ferner umfassend einen Schalter (1281), der mit dem Punktkoagulator (1260) verbunden ist, wobei der Schalter (1281) konfiguriert ist, um mindestens einen Teil des Punktkoagulators (1260) bezüglich der Hauptspitze (1201) zwischen mindestens einer operativen Position und der mindestens einen Lagerposition zu bewegen; und wobei vorzugsweise der Schalter (1281) konfiguriert ist, den mindestens einen Teil des Punktkoagulators (1260) axial bezüglich einer Achse der chirurgischen Vorrichtung zwischen der mindestens einen operativen Position und der mindestens einen Speicherposition zu bewegen.

14. Chirurgische Vorrichtung nach Anspruch 11, wobei der Punktkoagulatorkoppler (1265) eine Hülse umfasst, wobei die Punktkoagulatorspitze (1261) konfiguriert ist, um in einer Speicherposition in der Hülse (1265.1) aufgenommen zu werden, wobei die Punktkoagulatorspitze (1261) konfiguriert ist, um sich in einer operativen Position aus der Hülse zu erstrecken, und wobei der Punktkoagulator (1260) so konfiguriert ist, dass die Punktkoagulatorspitze (1261) in der operativen Position elektrische Energie aufnehmen kann, und so dass die Punktkoagulatorspitze (1261) daran gehindert wird, in der Lagerposition elektrische Energie zu empfangen.

## Revendications

1. Appareil (100) de séparation et de modification de tissu, comprenant :
un dispositif chirurgical comprenant :
une pointe principale (101) comprenant une pluralité de protubérances (151b) ;
au moins un élément de lyse (153b) positionné entre au moins deux protubérances adjacentes (151b) dans la pluralité de protubérances (151b) ;
une tige principale allongée (102) couplée à la pointe principale (101), la tige principale (102) présentant un axe s'étendant longitudinalement ;
lesdites au moins deux protubérances adjacentes (151b) étant orientées dans une direction non axiale par rapport à l'axe susmentionné de la tige principale (102), **caractérisé en ce que** lesdites au moins deux protubérances adjacentes (151b) sont espacées l'une de l'autre le long de l'axe susmentionné de la tige principale (102) ;
et **caractérisé en outre en ce que** la pluralité de protubérances (151b) comprend deux séries de protubérances non axiales (151b) s'étendant dans des directions qui sont sensiblement opposées l'une par rapport à l'autre, et ledit appareil comprenant un élément de lyse respectif (153b), entre des protubérances adjacentes (151b) de chaque série.

2. Appareil selon la revendication 1, comprenant en outre un coagulateur ponctuel (660) comprenant :
une tige de coagulateur ponctuel (662) ; et
une pointe de coagulateur ponctuel (661) conçue pour délivrer une énergie pour la coagulation d'un vaisseau sanguin pendant une intervention chirurgicale avec l'appareil pour la séparation et la modification de tissu, ladite pointe de coagulateur ponctuel (661) étant mobile par rapport à la pluralité de protubérances (151b) ; et, de préférence, la pointe de coagulateur ponctuel (661) étant conçue pour pivoter autour d'une base pour délivrer de manière sélective une énergie à différentes régions de la pointe principale (101).

3. Appareil selon la revendication 2, comprenant en outre un premier élément conducteur et un second élément conducteur, ledit premier élément conducteur étant conçu pour se coupler électriquement au second élément conducteur en configuration opérationnelle pour permettre à la pointe de coagulateur ponctuelle (661) de délivrer une énergie pour la coagulation d'un vaisseau sanguin, et ledit premier élément conducteur étant électriquement isolé du second élément conducteur en configuration de stockage pour empêcher la pointe de coagulateur ponctuel (661) de délivrer une énergie ; ladite pointe de coagulateur ponctuel (661) étant de préférence mobile axialement par rapport à la tige principale (102) de sorte que le coagulateur ponctuel (660) puisse être avancé jusqu'à la configuration opérationnelle et rétracté jusqu'à la configuration de stockage.

4. Appareil selon la revendication 3, ledit coagulateur ponctuel (660) étant configuré pour fournir une pluralité de positions opérationnelles de sorte qu'au moins un élément conducteur du coagulateur ponctuel (660) se couple à au moins un autre élément conducteur de sorte que le coagulateur ponctuel (660) puisse délivrer une énergie pour la coagulation d'un vaisseau sanguin dans l'une quelconque de la pluralité de positions opérationnelles.

5. Appareil selon la revendication 4, comprenant en outre une pluralité d'éléments conducteurs, ledit second élément conducteur étant l'un de la pluralité d'éléments conducteurs, et chacun des éléments conducteurs correspondant à l'une de la pluralité de positions opérationnelles.

6. Appareil selon la revendication 4, au moins l'un des premier et second éléments conducteurs comprenant un isolant et une pluralité de sections conductrices, chacune des sections conductrices comprenant une ouverture (662.6) dans l'isolant pour permettre aux sections conductrices d'établir un contact avec un autre élément conducteur, et chacune des sections conductrices correspondant à l'une de la pluralité de positions opérationnelles.

7. Appareil selon la revendication 2, ladite tige principale (602) étant conçue pour être reçue dans un logement de tige (169) d'un dispositif électrochirurgical ; et
le coagulateur ponctuel (660) comprenant :
un moyen pour déplacer de manière sélective le coagulateur ponctuel (660) par rapport à la pointe principale (101), grâce auquel la pointe de coagulateur ponctuel (661) peut délivrer une énergie électrique pour la coagulation du vaisseau sanguin pendant l'intervention chirurgicale.

8. Appareil selon la revendication 7, ledit moyen pour déplacer de manière sélective le coagulateur ponctuel (1260) comprenant une genouillère coulissante (1281) couplée à la tige de coagulateur ponctuel (1262), ladite genouillère coulissante (1281) étant conçue pour faire avancer axialement la pointe de coagulateur ponctuel (1261) entre une position rétractée et au moins une position opérationnelle, de préférence entre une position rétractée et une pluralité de positions opérationnelles distinctes.

9. Appareil selon la revendication 7, ledit moyen pour déplacer de manière sélective le coagulateur ponctuel (660) comprenant en outre un premier élément conducteur, ladite tige de coagulateur ponctuel (662) comprenant en outre un second élément conducteur, ledit premier élément conducteur étant conçu pour se coupler électriquement de manière sélective au second élément conducteur en position opérationnelle, et ledit premier élément conducteur étant conçu pour se découpler électriquement de manière sélective du second élément conducteur en position de stockage.

10. Appareil selon la revendication 9, comprenant en outre un coupleur de coagulateur ponctuel conçu pour recevoir le coagulateur ponctuel (660) à travers lui, le coupleur de coagulateur ponctuel faisant de préférence partie intégrante de la pointe principale (101).

11. Appareil selon la revendication 2, ledit dispositif chirurgical comprenant en outre :
un coupleur de coagulateur ponctuel (1265) comprenant au moins une voie de passage ; et
un élément conducteur saillant ;
ledit coagulateur ponctuel (1260) étant conçu pour venir au contact de l'élément conducteur saillant pour recevoir une énergie en provenance du dispositif chirurgical pour permettre à la pointe de coagulateur ponctuel (1261) de délivrer une énergie électrique, et ledit coagulateur ponctuel (1260) s'étendant à travers ladite au moins une voie de passage.

12. Appareil chirurgical selon la revendication 11, ladite tige principale (1302) comprenant un noyau conducteur (1303.7) ; et de préférence le noyau conducteur (1303.7) comprenant une plaque d'élément de lyse (3005), ledit au moins un élément de lyse (153b) faisant partie de la plaque d'élément de lyse (3005).

13. Appareil chirurgical selon la revendication 11, comprenant en outre une genouillère (1281) couplée au coagulateur ponctuel (1260), ladite genouillère (1281) étant conçue pour déplacer au moins une partie du coagulateur ponctuel (1260) par rapport à la pointe principale (1201) entre au moins une position opérationnelle et au moins une position de stockage ; et de préférence la genouillère (1281) étant conçue pour déplacer ladite au moins une partie du coagulateur ponctuel (1260) axialement par rapport à un axe de l'appareil chirurgical entre ladite au moins une position opérationnelle et ladite au moins une position de stockage.

14. Appareil chirurgical selon la revendication 11, ledit coupleur de coagulateur ponctuel (1265) comprenant un étui, ladite pointe de coagulateur ponctuel (1261) étant conçue pour être reçue dans l'étui (1265.1) en position de stockage, ladite pointe de coagulateur ponctuel (1261) étant conçue pour s'étendre hors de l'étui en position opérationnelle, et ledit coagulateur ponctuel (1260) étant conçu de sorte que la pointe de coagulateur ponctuel (1261) puisse recevoir une énergie électrique en position opérationnelle et de sorte que la pointe de coagulateur ponctuel (1261) soit empêcher de recevoir une énergie électrique en position de stockage.
